# EUROPÄISCHE PATENTSCHRIFT

(11) **EP 1 601 671 B1**
(45) Veröffentlichungstag und Bekanntmachung des Hinweises auf die Patenterteilung: **29.07.2009**
(21) Anmeldenummer: 04712484.7
(22) Anmeldetag: 19.02.2004
(51) Int. Cl.: C07D 413/12, C07D 419/12, C07D 263/32, A61P 3/10

(54) **CYCLOALKYLDERIVATE MIT BIOISOSTEREN CARBONSÄURE - GRUPPEN, VERFAHREN ZU IHRER HERSTELLUNG UND IHRE VERWENDUNG ALS ARZNEIMITTEL**
CYCLOALKYL DERIVATIVES COMPRISING BIOISOSTERIC CARBOXYLIC ACID GROUPS, METHOD FOR THE PRODUCTION THEREOF, AND USE THEREOF AS A MEDICAMENT
DERIVES DE CYCLOALKYLES COMPORTANT DES GROUPES ACIDES CARBOXYLIQUES BIOISOSTERES, LEUR PROCEDE DE PRODUCTION ET LEUR UTILISATION EN TANT QUE MEDICAMENTS

(30) Priorität: 27.02.2003 DE 10308354
(43) Veröffentlichungstag der Anmeldung: 07.12.2005
(73) Patentinhaber: Sanofi-Aventis Deutschland GmbH, 65929 Frankfurt am Main (DE)
(72) Erfinder: GRETZKE, Dirk, 60596 Frankfurt (DE); GLOMBIK, Heiner, 65719 Hofheim (DE); FALK, Eugen, 60529 Frankfurt (DE); GOERLITZER, Jochen, 60594 Frankfurt am Main (DE); KEIL, Stefanie, 65719 Hofheim (DE); SCHAEFER, Hans-Ludwig, 65239 Hochheim (DE); STAPPER, Christian, 55118 Mainz (DE); WENDLER, Wolfgang, 65618 Selters (DE)
(86) Internationale Anmeldenummer: PCT/EP2004/001585
(87) Internationale Veröffentlichungsnummer: WO 2004/076447

(56) Entgegenhaltungen:
- WO-A-00/64876
- WO-A-01/00603

## Beschreibung

Cycloalkylderivate mit bioisosteren Carbonsäure - Gruppen, Verfahren zu ihrer Herstellung und ihre Verwendung als Arzneimittel

Die Erfindung betrifft Cycloalkylderivate mit bioisosteren Carbonsäure - Gruppen sowie deren physiologisch verträgliche Salze und physiologisch funktionelle Derivate.

Es sind bereits strukturähnliche Verbindungen zur Behandlung von Hyperlipidämie und Diabetes im Stand der Technik beschrieben (WO 2000/64876).

Der Erfindung lag die Aufgabe zu Grunde, Verbindungen zur Verfügung zu stellen, die eine therapeutisch verwertbare Modulation des Lipid- und/oder Kohlehydratstoffwechsels erlauben und sich somit zur Prävention und/oder Behandlung von Krankheiten wie Typ 2 Diabetes und Atherosklerose sowie deren vielfältigen Folgeerkrankungen eignen.

Überraschenderweise wurde eine Serie von Verbindungen gefunden, die die Aktivität von PPAR Rezeptoren modulieren. Insbesondere eignen sich die Verbindungen zur Aktivierung von PPARalpha und PPARgamma, wobei das Ausmaß der relativen Aktivierung je nach Verbindungen unterschiedlich sein kann.

Die Erfindung betrifft daher Verbindungen der Formel I 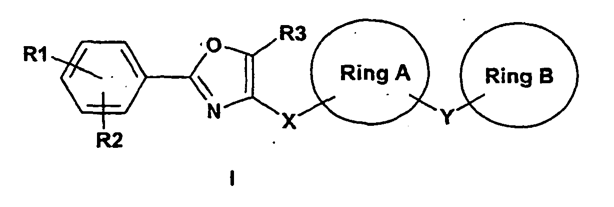 worin bedeuten
- Ring A: (C₃-C₈)-Cycloalkandiyl, (C₃-C₈)-Cycloalkendiyl, wobei in den Cycloalkandiyl-oder Cycloalkendiylringen ein oder mehrere Kohlenstoffatome durch Sauerstoffatome ersetzt sein können;
- R1, R2: unabhängig voneinander H, F, Br, CF₃, OCF₃, (C₁-C₆)-Alkyl, O-(C₁-C₆)- Alkyl, SCF₃, SF₅, OCF₂-CHF₂, O-Phenyl, OH, NO₂; oder
- R1 und R2: in Nachbarstellung zusammen mit dem Phenylring für ein kondensiertes, ganz, teilweise oder nicht gesättigtes bicyclisches (C₉-C₁₂)-Aryl- oder (C₉- C₁₁)-Heteroaryl-Ring-System;
- R3: H, CF₃, (C₁-C₆)-Alkyl, (C₁-C₆)-Alkoxy, (C₃-C₈)-Cycloalkyl, Phenyl;
- X: (C₁-C₆)-Alkandiyl, wobei in der Alkandiylgruppe ein oder mehrere Kohlenstoffatome durch Sauerstoffatome ersetzt sein können;
- Y: (C₁-C₆)-Alkandiyl oder (C₁-C₆)-Alkendiyl, wobei in der Alkandiyl- oder Alkendiyigruppe ein oder mehrere CH₂-Gruppen durch O, CO, S, SO oder SO₂ ersetzt sein können, und Alkandiyl oder Alkendiyl gegebenenfalls durch OH substituiert sein können;
- Ring B: Phenyl, das ein- oder zweifach substituiert sein kann mit NO₂, Cl, CN, (C₁- C₆)-Alkyl, (C₁-C₆)-Alkoxy oder Tetrazol; oder Pyrrolidin-2-on, das zusätzlich ein N- oder S-Atom im Ring enthält und durch Oxo oder Thioxo substituiert ist durch R4 am N-Atom substituiert sein kann;
- R4: (C₁-C₆)-Alkyl, Phenyl, Benzyl;
sowie deren physiologisch verträgliche Salze.

Bevorzugt sind Verbindungen der Formel I, worin bedeuten
- Ring A: (C₃-C₈)-Cycloalkandiyl, (C₃-C₈)-Cycloalkendiyl, wobei in den Cycloalkandiyl- oder Cycloalkendiylringen ein Kohlenstoffatom durch ein Sauerstoffatom ersetzt sein kann;
- R1, R2: unabhängig voneinander H, F, Br, CF₃, OCF₃, (C₁-C₆)-Alkyl, O-(C₁-C₆)- Alkyl, SCF₃, SF₅, OCF₂-CHF₂, O-Phenyl, OH, NO₂; oder
- R1 und R2: in Nachbarstellung zusammen mit dem Phenylring für ein kondensiertes, ganz, teilweise oder nicht gesättigtes bicyclisches (C₉-C₁₂)-Aryl- oder (C₉- C₁₁)-Heteroaryl-Ring-System;
- R3: H, CF₃, (C₁-C₆)-Alkyl, (C₃-C₈)-Cycloalkyl, Phenyl;
- X: (C₁-C₆)-Alkandiyl, wobei in der Alkandiylgruppe ein Kohlenstoffatom durch ein Sauerstoffatom ersetzt sein kann
- Y: (C₁-C₆)-Alkandiyl oder (C₁-C₆)-Alkendiyl, wobei in der Alkandiyl- oder Alkendiylgruppe ein oder zwei CH₂-Gruppen durch O, CO, S, SO oder SO₂ ersetzt sein können und Alkandiyl oder Alkendiyl gegebenenfalls durch OH substituiert sein können;
- Ring B: Phenyl, das ein- oder zweifach substituiert sein kann mit NO₂, Cl, CN, (C₁- C₆)-Alkyl , (C₁-C₆)-Alkoxy oder Tetrazol; oder Pyrrolidin-2-on, das zusätzlich ein N- oder S-Atom in 4-Position enthält und durch Oxo oder Thioxo in 5-Position substituiert ist durch R4 am N1-Atom substituiert sein kann;
- R4: (C₁-C₆)-Alkyl, Phenyl, Benzyl;
sowie deren physiologisch verträgliche Salze.

Besonders bevorzugt sind Verbindungen der Formel I, worin bedeuten
- Ring A: (C₃-C₈)-Cycloalkandiyl, worin ein Kohlenstoffatom durch ein Sauerstoffatom ersetzt ist;
- R1, R2: unabhängig voneinander H, F, Br, CF₃, OCF₃, (C₁-C₆)-Alkyl, O-(C₁-C₆)- Alkyl, SCF₃, SF₅, OCF₂-CHF₂, O-Phenyl, OH, NO₂; oder
- R1 und R2: in Nachbarstellung zusammen mit dem Phenylring für ein kondensiertes, nicht gesättigtes bicyclisches (C₉-C₁₂)-Aryl- oder (C₉-C₁₁)-Heteroaryl-Ring- System;
- R3: H, CF₃, (C₁-C₆)-Alkyl, (C₃-C₈)-Cycloalkyl, Phenyl;
- X: (C₁-C₆)-Alkandiyl, worin das C1-Kohlenstoffatom durch ein Sauerstoffatom ersetzt ist;
- Y: (C₁-C₆)-Alkandiyl oder (C₁-C₆)-Alkendiyl, wobei in der Alkandiyl- oder Alkendiylgruppe ein oder zwei CH₂-Gruppen durch O, CO oder SO₂ ersetzt sein können und Alkandiyl oder Alkendiyl gegebenenfalls durch OH substituiert sein können; .
- Ring B: Phenyl, das ein- oder zweifach substituiert sein kann mit NO₂, Cl, CN, (C₁- C₆)-Alkyl , (C₁-C₆)-Alkoxy oder Tetrazol; oder Pyrrolidin-2-on, das zusätzlich ein N- oder S-Atom in 4-Position enthält und durch Oxo oder Thioxo in 5-Position substituiert ist durch R4 am N1-Atom substituiert sein kann;
- R4: (C₁-C₆)-Alkyl, Phenyl, Benzyl;
sowie deren physiologisch verträgliche Salze.

Ganz besonders bevorzugt sind Verbindungen der Formel I, worin bedeuten
- Ring A: Cyclohexan-1,3-diyl;
- R1, R2: unabhängig voneinander H, F, Br, CF₃, OCF₃, (C₁-C₆)-Alkyl, O-(C₁-C₆)- Alkyl, SCF₃, SF₅, OCF₂-CHF₂, O-Phenyl, OH, NO₂; oder
- R1 und R2: in Nachbarstellung zusammen mit dem Phenylring für ein kondensiertes, nicht gesättigtes bicyclisches (C₉-C₁₀)-Aryl- oder (C₉-C₁₀)-Heteroaryl-Ring- System steht;
- R3: H, CF₃, (C₁-C₆)-Alkyl, (C₃-C₆)-Cycloalkyl, Phenyl;
- X: CH₂-O;
- Y: (C₁-C₄)-Alkandiyl, O-(C₁-C₄)-Alkandiyl, (C₁-C₄)-Alkendiyl, O-(C₁-C₄)- Alkendiyl, O-SO₂, O-CO, wobei Alkandiyl durch OH substituiert sein kann;
- Ring B: Phenyl, das ein- oder zweifach substituiert sein kann mit NO₂, Cl, CN, (C₁- C₆)-Alkyl, (C₁-C₆)-Alkoxy oder Tetrazol; oder Thiazolidin-1,4-dion, das durch R4 am N3-Atom substituiert sein kann;
- R4: (C₁-C₆)-Alkyl, Phenyl, Benzyl;
sowie deren physiologisch verträgliche Salze oder

Verbindungen der Formel I, worin bedeuten
- Ring A: Cyclohexan-1,3-diyl;
- R1, R2: unabhängig voneinander H, F, Br, CF₃, (C₁-C₆)-Alkyl, O(C₁-C₆)-Alkyl; oder
- R1 und R2: zusammen mit dem Phenylring für Naphthyl steht;
- R3: C₁-C₆)-Alkyl, (C₅-C₆)-Cycloalkyl, Phenyl;
- X: CH₂-O
- Y: (C₁-C₄)-Alkandiyl, O-(C₁-C₄)-Alkandiyl, (C₁-C₄)-Alkendiyl, O-(C₁-C₄)- Alkendiyl, O-SO₂, O-CO, wobei Alkandiyl durch OH substituiert sein kann;
- Ring B: Phenyl, das ein- oder zweifach substituiert sein kann mit NO₂, Cl, CN, (C₁- C₆)-Alkyl , (C₁-C₆)-Alkoxy oder Tetrazol oder
- Ring B: Thiazolidin-2,4-dion, das durch R4 am N3-Atom substituiert sein kann;
- R4: (C₁-C₆)-Alkyl, Phenyl, Benzyl;
sowie deren physiologisch verträgliche Salze.

Ganz besonders hervorzuheben sind auch die Verbindungen der Formel I, worin ein oder mehrere Reste die folgenden Bedeutungen haben:
- Ring A: Cyclohexan-1,3-diyl; oder
- R2: Wasserstoff; oder
- R2: Wasserstoff und R1 in meta oder para-Stellung verknüpft ist; oder
- X: -CH₂-O-; oder
- Y: -CH₂-CH₂-; oder solche mit
- Ring B: Thiazolidin-2,4-dion.

Die vorliegende Erfindung umfasst ebenfalls alle Kombinationen der "bevorzugten Ausführungsformen" der hierin beschriebenen Erfindung.

Die Alkyl-Reste in den Substituenten R1, R2, R3 und R4 können sowohl geradkettig wie verzweigt sein.

Unter Aryl wird ein aromatisches carbocyclisches mono- oder bicyclisches RingSystem verstanden, das 6 bis 10 Atome im Ring oder in den Ringen enthält.

Heteroaryl ist ein mono- oder bicyclisches aromatisches Ringsystem mit 4 bis 11 Ringgliedern, worin mindestens ein Atom im Ringsystem ist ein Heteroatom aus der Reihe N, O und S.

Die Verbindungen der Formel I enthalten mindestens zwei Assymmetriezentren und können darüber hinaus mehr enthalten. Daher können die Verbindungen der Formel I in Form ihrer Racemate, racemischen Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in.H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

### Verwendung

Diese Erfindung bezieht sich weiterhin auf die Verwendung von Verbindungen der Formel I und ihren pharmazeutischen Zusammensetzungen als PPAR-Rezeptor Liganden. Die erfindungsgemäßen PPAR-Rezeptor-Liganden eignen sich als Modulatoren der Aktivität der PPAR Rezeptoren. Peroxisomen-Proliferatoren-Aktivierte Rezeptoren (PPAR) sind durch Liganden aktivierbare Transkriptionsfaktoren, die zur Klasse der Kern-Hormon-Rezeptoren gehören. Es existieren drei PPAR Isoformen, PPARalpha, PPARgamma und PPARdelta, die von verschiedenen Genen kodiert werden (Peroxisome proliferator-activated receptor (PPAR): structure, mechanisms of activation and diverse functions: Motojima K, Cell Struct Funct. 1993 Oct; 18(5): 267-77).

Von PPARgamma existieren zwei Varianten, PPARgamma₁ und gamma₂, die das Ergebnis eines alternativen Einsatzes von Promotoren und einer differenziellen mRNA-Spleißung (Vidal-Puig et al. J. Clin. Invest., 97:2553-2561, 1996) sind. Die verschiedenen PPAR Rezeptoren besitzen eine unterschiedliche Gewebsverteilung und modulieren unterschiedliche physiologische Funktionen. Die PPAR-Rezeptoren spielen eine Schlüsselrolle bei unterschiedlichen Aspekten der Regulation einer Vielzahl von Genen, deren Genprodukte direkt oder indirekt am Lipid- und Kohlehydratstoffwechsel entscheidend beteiligt sind. So spielen z. B. PPARalpha Rezeptoren bei der Regulation des Fettsäurekatabolismus oder des Lipoproteinstoffwechsels in der Leber eine wichtige Rolle, während PPARgamma zum Beispiel bei der Regulation der Fettzelldifferenzierung entscheidend beteiligt ist. Darüberhinaus sind PPAR Rezeptoren aber auch an der Regulation vieler weiterer physiologischer Prozesse, auch solcher die nicht direkt mit dem Kohlehydrat- oder Lipidstoffwechsel in Verbindung stehen, beteiligt. Die Aktivität der unterschiedlichen PPAR Rezeptoren kann durch verschiedene Fettsäuren, Fettsäurederivate sowie synthetische Verbindungen in unterschiedlichem Ausmaß moduliert werden.

Entsprechende Reviews über Funktionen, physiologische Wirkung und Pathophysiologie siehe: Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63.

Die vorliegende Erfindung betrifft Verbindungen der Formel I, die sich zur Modulierung der Aktivität von PPAR Rezeptoren eignen, insbesondere der Aktivität von PPARalpha und PPARgamma. Je nach Profil der Modulation sind die Verbindungen der Formel I zur Behandlung, Kontrolle und Prohylaxe nachfolgend beschriebener Indikationen, sowie einer Reihe anderer, damit verbundener pharmazeutischer Anwendungen geeignet (siehe z.B. Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63; Jean-Charles Fruchart, Bart Staels and Patrick Duriez: PPARS, Metabolic Disease and Arteriosclerosis, Pharmacological Research, Vol. 44, No. 5, 345-52, 2001; Sander Kersten, Beatrice Desvergne & Walter Wahli: Roles of PPARs in health and disease, NATURE, VOL 405 ,25 MAY 2000, 421-4; Ines Pineda Torra, Giulia Chinetti, Caroline Duval, Jean-Charles Fruchart and Bart Staels: Peroxisome proliferator-activated receptors: from transcriptional control to clinical practice, Curr Opin Lipidol 12: 2001 245-254).

Besonders geeignet sind solche Verbindungen zur Behandlung und/oder Prävention von
1.
   - Störungen des Fettsäurestoffwechsels und Glucoseverwertungsstörungen
   - Störungen, bei denen Insulin Resistenz eine Rolle spielt
2. Diabetes mellitus, insbesondere Typ 2 Diabetes einschließlich der Verhinderung der damit verbundenen Folgeerkrankungen. Besondere Aspekte sind dabei die
   - Hyperglykämie,
   - Verbesserung der Insulinresistenz,
   - Verbesserung der Glucose-Toleranz,
   - Schutz der ß-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid,- hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
4. Verschiedene andere Zuständen, die mit dem Metabolischen Syndrom assoziiert sein können, sind wie:
   - Adipositas (Fettsucht), einschließlich abdominale Adipositas
   - Thrombosen, Stadien von Hypejkoagulabilität und Thromboseneigung (arteriell und venös)
   - Hoher Blutdruck
   - Herzinsuffizienz, wie z.B. (aber nicht beschränkt auf) bei Zustand nach Myokardinfarkt; hypertensive Herzerkrankung oder Kardiomyopathie
5. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Waren, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg bis50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindüngsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist: Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine ÖI-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von.0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formel I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente;
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparäten, worin mehrere Wirkstoffe in einer pharmazeutischen Zubereitung vorliegen, erfolgen.

### Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus® (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden. Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind,

Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel I in Kombination mit Insulin verabreicht.

Bei einer Ausführungsform der Erfindung werden die Verbindungen der Formel I in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188)

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform werden die Verbindungen der Formel I in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel I in Kombination mit einem PPARgamma Agonist, wie z.B. Rosiglitazon, Pioglitazon, verabreicht.

Bei einer Ausführungsform werden die Verbindungen der Formel I in Kombination mit Verbindungen mit inhibitorischer Winkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel I in Kombination mit einem Gallensäureresorptionsinhibitor verabreicht (siehe z.B. US 6,245,744, US 6,221,897, US 6,277,831, EP 0683 773, EP 0683 774).

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6,342,512) verabreicht.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPARalpha Agonist verabreicht.

Bei einer Ausführungsform der Erfindung werden die Verbindungen der Formel I in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel I in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonist verabreicht.

### Antiobesita

Bei einer Ausführungsform der Erfindung werden die Verbindungen der Formel I in Kombination mit einem Lipase Inhibitor, wie z.B. Ortistat, verabreicht.

Bei einer Ausführungsform ist der weitere Wirkstoff Fenfluramin oder Dexfenfluramin. Bei noch einer Ausführungsform ist der weitere Wirkstoff Sibutramin.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäüre {4-[(4-amino-quinazolin-2-ylamino)-methyl]-cyclohexylmethyl}- amid; hydrochloride (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2'-(2,3-dimethyl-1H-indol-6-yloxy)-ethylamino]-ethanol; hydrochloride (WO 01/83451)), MSH (Melanocyl-stimulierendes. Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Beneoxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin.

Bei einer Ausführungsform werden die Verbindungen der Formel I in Kombination mit Medikamenten mit Wirkungen auf das Herz-Kreislauf und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der Verbindungen wurde wie folgt getestet:

### Bestimmung von EC50-Werten von PPAR-Agonisten im zellulären PPARalpha-Test

### Prinzip

Für die Analyse der Wirkstärke von Substanzen, die an humanes PPARalpha binden und es in agonistischer Weise aktivieren, wird eine stabil transfizierte HEK-Zellinie (HEK= human embryo kidney) benutzt, die hier als PPARalpha-Reporterzellinie bezeichnet wird. Sie enthält zwei genetische Elemente, ein Luziferase-Reporterelement (pdeltaM-GAL4-Luc-Zeo) und ein PPARalpha-Fusionsprotein (GR-GAL4-humanPPARalpha-LBD), welches die Expression des Luziferase-Reporterelementes in Abhängigkeit eines PPARalpha-Liganden vermittelt. Das stabil und konstitutiv exprimierte Fusionsprotein GR-GAL4-humanPPARalpha-LBD bindet im Zellkern der PPARalpha-Reporterzellinie über den GAL4-Proteinanteil an die GAL4-DNA-Bindungsmotife 5'-oberhalb des Luziferase-Reporterelementes, das im Genom der Zellinie integriert ist. Ohne Zugabe eines PPARalpha-Liganden ist die Expression des Luziferase-Reportergens nur gering, sofern im Test fettsäuredepletiertes fötales Kälberserum (cs-FKS) verwendet wird. PPARalpha-Liganden binden und aktivieren das PPARalpha-Fusionsprotein und bewirken darüber die Expression des Luciferasereportergens. Die gebildete Luziferase lässt sich über ein entsprechendes Substrat mittels Chemilumineszenz nachweisen.

### Konstruktion der Zelllinie

Die PPARalpha-Reporterzellinie wurde in 2 Stufen hergestellt: Zuerst wurde das Luziferase-Reporterelement konstruiert und stabil in HEK-Zellen transfiziert. Dazu wurden fünf Bindungsstellen des Hefe-Transkriptionsfaktors GAL4 (jeweils 5'-CGGAGTACTGTCCTCCGAG-3') 5'-oberhalb eines 68 bp langen minimalen MMTV-Promotors (Genbank-Accession # V01175) einkloniert. Der minimale MMTV-Promotorabschnitt enthält eine CCAAT-Box und ein TATA-Element, um eine effiziente Transkription durch die RNA-Polymerase II zu ermöglichen. Die Klonierung und Sequenzierung des GAL4-MMTV-Konstruktes erfolgte analog wie bei Sambrook J. et. al. beschrieben (Molecular cloning, Cold Spring Harbor Laboratory Press, 1989). Danach wurde 3'-unterhalb des GAL4-MMTV-Elementes das gesamte Luziferasegen von Photinus pyralis (Genbank-Accession # M15077) einkloniert. Nach der Sequenzierung wurde das aus fünf GAL4-Bindungsstellen, MMTV-Promotor und Luziferasegen bestehende Luziferase-Reporterelement in ein Zeocin-Resistenz vermittelndes Plasmid umkloniert, um zu dem Plasmid pdeltaM-GAL4-Luc-Zeo zu gelangen. Dieser Vektor wurde nach den Angaben in Ausubel, F.M. et al. (Current protocols in molecular biology, Vol. 1-3, John Wiley & Sons, Inc., 1995) in HEK-Zellen transfiziert. Danach wurde unter Verwendung von zeocinhaltigem Medium (0,5 mg/ml) ein geeigneter stabiler Zellklon selektioniert, der eine möglichst niedrige Basalexpression des Luziferasegens zeigte. In einem zweiten Schritt wurde das PPARalpha-Fusionsprotein (GR-GAL4-humanPPARalpha-LBD in den beschriebenen stabilen Zellklon eingebracht. Dazu wurde zunächst die für die N-terminalen 76 Aminosäuren des Glukocorticoid-Rezeptors (Genbank-Accession # P04150) kodierende cDNA mit dem für die Aminosäuren 1-147 des Hefetranskriptionstaktors GAL4 (Genbank-Accession # P04386) kodierenden cDNA-Abschnitt verknüpft. Am 3'-Ende dieses GR-GAL4-Konstruktes wurde die cDNA der Ligandenbindungsdomäne des humanen PPARalpha-Rezeptors einkloniert (Aminosäuren 5167-Y468; Genbank-Accession # S74349). Das so hergestellte Fusionskonstrukt (GR-GAL4-humanPPARalpha-LBD) wurde in das Plasmid pcDNA3 (Firma Invitrogen) umkloniert, um darin eine konstitutive Expression durch den Cytomegalovirus-Promotor zu ermöglichen. Dieses Plasmid wurde mit einer Restriktionsendonuklease linearisiert und stabil in den bereits beschriebenen, das Luziferase-Reporterelement enthaltenden Zellklon transfiziert. Durch Selektion mit Zeocin (0,5 mg/ml) und G418 (0,5 mg/ml) wurde die fertige PPARalpha-Reporterzellinie isoliert, die ein Luziferase-Reporterelement enthält und konstitutiv das PPARalpha-Fusionsprotein (GR-GAL4-humanPPARalpha-LBD) exprimiert.

### Durchführung des Tests

Die Aktivität von PPARalpha-Agonisten wird in einem 3-Tagestest bestimmt, der nachfolgend beschrieben ist:

### Tag 1

Die PPARalpha-Reporterzellinie wird bis zu einer 80 %-igen Konfluenz in DMEM-Medium (# 41965-039, Invitrogen) kultiviert, das mit folgenden Zusätzen versetzt ist: 10% cs-FKS (fötales Kälberserum; #SH-30068.03, Hyclone), 0,5 mg/ml Zeocin (#R250-01, Invitrogen), 0,5 mg/ml G418 (#10131-027, Invitrogen), 1% Penicillin-Streptomycin-Lösung (#15140-122, Invitrogen) und 2 mM L-Glutamin (#25030-024, Invitrogen). Die Kultivierung erfolgt in Standard-Zellkulturflaschen (# 353112, Becton Dickinson) in einem Zellkulturbrutschrank bei 37°C in Anwesenheit von 5% CO₂. Die zu 80% konfluenten Zellen werden einmal mit 15 ml PBS gewaschen (#14190-094, Invitrogen), mit 3 ml Trypsinlösung (#25300-054, Invitrogen) für 2 min bei 37°C behandelt, in 5 ml des beschriebenen DMEM-Mediums aufgenommen und in einem Zellzählgerät gezählt. Nach der Verdünnung auf 500.000 Zellen/ml werden jeweils 35.000 Zellen pro weil einer 96 well-Mikrotiterplatte mit klarem Plastikboden (#3610, Corning Costar) ausgesät. Die Platten werden für 24 h in einem Zellkulturbrutschrank bei 37°C und 5% CO₂ inkubiert.

### Tag 2

Zu testende PPARalpha-Agonisten werden in einer Konzentration von 10 mM in DMSO gelöst. Diese Stocklösung wird in DMEM-Medium (#41965-039, Invitrogen) verdünnt, das mit 5 % cs-FKS (#SH-30068.03, Hyclone), 2 mM L-Glutamin (#25030-024, Invitrogen) und den bereits beschriebenen Antibiotika (Zeocin, G418, Penicillin und Streptomycin) versetzt ist.

Testsubstanzen werden in 11 verschiedenen Konzentrationen im Bereich von 10 µM bis 100 pM getestet. Potentere Verbindungen werden in Konzentrationsbereichen von 1 µM bis 10 µM oder zwischen 100 nM und 1 pM geprüft.

Das Medium der an Tag 1 ausgesäten PPARalpha-Reporterzelllinie wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Die DMSO-Konzentration in dem Test beträgt unter 0.1 % v/v, um zelltoxische Effekte des Lösungsmittels zu vermeiden. Jede Platte wurde mit einem Standard PPARalpha-Agonisten belegt, der ebenfalls in 11 verschiedenen Konzentrationen verdünnt wird, um die Funktionsfähigkeit des Tests in jeder Einzelplatte nachzuweisen. Die Testplatten werden für 24 h in einem Brutschrank bei 37°C und 5 % CO₂ inkubiert.

### Tag 3

Die mit den Testsubstanzen behandelten PPARalpha-Reporterzellen werden aus dem Brutschrank entnommen und das Medium abgesaugt. Zur Lyse der Zellen werden 50 µl Bright Glo Reagens (Firma Promega) pro well einer 96-well Mikrotiterplatte zupipettiert. Nach einer 10 minütigen Inkubation im Dunkeln bei Raumtemperatur werden die Mikrotiterplatten im Lumineszenzmeßgerät (Trilux der Firma Wallac) gemessen. Die Messzeit pro well einer Mikrotiterplatte beträgt 1 sec.

### Auswertung

Die Rohdaten des Lumineszenzmeßgerätes werden in ein Microsoft Excel-File transferiert. Dosis-Wirkungskurven und EC50-Werte von PPAR-Agonisten werden mit dem Program XL.Fit nach Vorgabe des Herstellers (Firma IDBS) berechnet.

Die PPARalpha-EC50-Werte für die Verbindungen der Beispiele 1 bis 68 liegen in diesem Assay im Bereich von 3nM bis >10 µM.

Die Ergebnisse für die Aktivität einiger erfindungsgemäßer Verbindungen der Formel I sind in der folgenden Tabelle I angegeben:

Die Ergebnisse für die Aktivität der efindungsgemäßen Verbindungen der Formel I sind in der folgenden Tabelle I angegeben:

**Tabelle I**

| **Beispiel Nr.** | **EC50 PPARalpha [nM]** |
|---|---|
| VIII | 91 |
| XX | 1931 |
| XXI | 1251 |
| XXIV | 227 |
| XXVI | 709 |
| XXVII | 726 |
| XXXIV | 114 |
| XXXV | 187. |

Aus der Tabelle I ist ersichtlich, dass die erfindungsgemäßen Verbindungen der Formel I den PPARalpha-Rezeptor aktivieren und damit zum Beispiel analog zu klinisch verwendeten Fibraten im Organismus eine Triglyceridsenkung bewirken (siehe z.B. J.-Ch. Fruchard et al.,: PPARS, Metabolic Disease and Atherosclerosis, Pharmacological Research, Vol. 44, No. 5, 345-52, 2001; S. Kersten et al.: Roles of PPARs in health and disease, NATURE, VOL 405 ,25 MAY 2000, 421-4;1. Pineda et al.: Peroxisome proliferator-activated receptors: from transcriptional control to clinical practice,Curr Opin Lipidol 12: 2001, 245-254).

### Bestimmung von EC50-Werten von PPAR-Agonisten im zellulären PPARgamma - Test

### Prinzip

Zur Bestimmung der zellulären PPARgamma Aktivität von PPAR-Agonisten wird ein transientes Transfektionssystem eingesetzt. Es basiert auf der Verwendung eines Luziferase-Reporterplasmides (pGL3basic-5xGAL4-TK) und eines PPARgamma-Expressionsplasmides (pcDNA3-GAL4-humanPPARgammaLBD). Beide Plasmide werden vorübergehend (= transient) in humane embryonale Nierenzellen (HEK-Zellen) transfiziert. In diesen Zellen wird dann das Fusionsprotein GAL4-humanPPARgammaLBD exprimiert, das an die GAL4-Bindungsstellen des Reporterplasmides bindet. In Anwesenheit eines PPARgamma-aktiven Liganden wird durch das aktivierte Fusionsprotein GAL4-humanPPARgammaLBD die Expression des Luziferase-Reportergens induziert, was sich nach Zugabe eines Luziferasesubtrates in Form eines Chemilumineszenzsignals nachweisen lässt. Im Unterschied zur stabil transfizierten PPARalpha-Reporterzellinie werden beim zellulären PPARgamma-Test die beiden Komponenten (Luziferase-Reporterplasmid und PPARgamma-Expressionsplasmid) transient in HEK-Zellen transfiziert, weil die stabile und permanente Expression des PPARgamma-Fusionsproteins zelltoxisch ist.

### Konstruktion der Plasmide

Das Luziferase-Reporterplasmid pGL3basic-5xGAL4-TK basiert auf dem Vektor pGL3basic der Firma Promega. Zur Herstellung des Reporterplasmides wurden fünf Bindungsstellen des Hefe-Transkriptionsfaktors GAL4 (jede Bindungsstelle mit der Sequenz 5'-CTCGGAGGACAGTACTCCG-3'), 5'-oberhalb zusammen mit einem 160 bp langen Thymidinkinase-Promotorabschnitt (Genbank-Accession # AF027128) in pGL3basic einkloniert. 3'-unterhalb des Thymidinkinasepromotors liegt das gesamte Luziferasegen von Photinus pyralis (Genbank-Accession # M15077), welches bereits Bestandteil des verwendeten Plasmides pGL3basic ist. Die Klonierung und Sequenzierung des Reporterplasmides pGL3basic-5xGAL4-TK erfolgte analog wie bei Sambrook J. et. al. beschrieben (Molecular cloning, Cold Spring Harbor Laboratory Press, 1989).

Zur Herstellung des PPARgamma-Expressionsplasmides pcDNA3-GAL4-humanPPARgammaLBD wurde in das Plasmid pcDNA3 (Firma Invitrogen) 3'-unterhalb des Cytomegalovirus-Promotors zunächst die für die Aminosäuren 1-147 des Hefetranskriptionsfaktors GAL4 (Genbank-Accession # P04386) kodierende DNA einktoniert. 3'-unterhalb der GAL4-DNA-Bindungsdomäne wurde anschließend die cDNA der Ligandenbindungsdomäne (LBD) des humanen PPARgamma-Rezeptors kloniert (Aminosäuren 1152-Y475; Accession # g1480099). Klonierung und Sequenzierung des PPARgamma-Expressionsplasmides pcDNA3-GAL4-humanPPARgammaLBD erfolgten wiederum analog wie bei Sambrook J. et. al. beschrieben (Molecular cloning, Cold Spring Harbor Laboratory Press, 1989). Neben dem Luziferase-Reporterplasmid pGL3basic-5xGAL4-TK und dem PPARgamma-Expressionsplasmid pcDNA3-GAL4-humanPPARgammaLBD werden für den zellulären PPARgamma-Test noch das Referenzplasmid pRL-CMV (Firma Promega), sowie das Plasmid pBluescript-SK(+) der Firma Stratagene verwendet. Alle vier Plasmide wurden vor der Transfektion in HEK-Zellen mit einem Plasmidpräparationskit der Firma Qiagen präpariert, der eine möglichst endotoxinfreie Plasmidqualität gewährleistet.

### Durchführung des Tests

Die Aktivität von PPARgamma-Agonisten wird in einem 4-Tagestest bestimmt, der nachfolgend beschrieben ist. Vor der Transfektion werden HEK-Zellen in DMEM-Medium (# 41965-039, Invitrogen) kultiviert, das mit folgenden Zusätzen versetzt ist: 10% FKS (#16000-044, Invitrogen), 1% Penicillin-Streptomycin-Lösung (#15140-122, Invitrogen) und 2 mM L-Glutamin (#25030-024, Invitrogen).

### Tag 1

Zunächst wird Lösung A hergestellt, ein Transfektionsgemisch, das neben DMEM-Medium alle vier bereits beschriebenen Plasmide enthält. Für einen Test werden pro 96-well Mikrotiterplatte folgende Mengen zum Ansetzen von 3 ml Lösung A verwendet: 2622 µl antibiotika- und serumfreies DMEM-Medium (#41965-039, Invitrogen), 100 µl Referenzplasmid pRL-CMV (1 ng/µl), 100 µl Luziferase-Reporterplasmid pGL3basic-5xGAL4-TK (10 ng/µl), 100 µl PPARgamma-Expressionsplasmid pcDNA3-GAL4-humanPPARgammaLBD (100 ng/µl) und 78 µl Plasmid pBluescript-SK(+) (500 ng/µl). Danach werden pro 96-well Mikrotiterplatte 2 ml Lösung B durch Mischen von 1,9 ml DMEM-Medium (# 41965-039, Invitrogen) mit 100 µl PolyFect-Transfektionsreagens (Firma Qiagen) hergestellt. Anschließend werden 3 ml Lösung A mit 2 ml Lösung B zu 5. ml Lösung C versetzt, durch mehrfaches Pipettieren gründlich gemischt und für 10 min bei Raumtemperatur inkubiert.

80% konfluente HEK-Zellen aus einer 175 cm² großen Zellkulturflasche werden einmal mit 15 ml PBS gewaschen (#14190-094, Invitrogen) und mit 3 ml Trypsinlösung (#25300-054, Invitrogen) für 2 min bei 37°C behandelt. Danach werden die Zellen in 15 ml DMEM-Medium (# 41965-039, Invitrogen) aufgenommen, welches mit 10% FKS (# 16000-044, Invitrogen), 1% Penicillin-Streptomycin-Lösung (#15140-122, Invitrogen) und 2 mM L-Glutamin (#25030-024, Invitrogen) versetzt ist. Nach dem Zählen der Zellsuspension im Zellzählgerät wird die Suspension auf 250.000 Zellen/ml verdünnt. Für 1 Mikrotiterplatte werden 15 ml dieser Zellsuspension mit 5 ml der Lösung C vermengt Pro well einer 96 well-Mikrotiterplatte mit klarem Plastikboden (#3610, Corning Costar) werden 200 µl der Suspension ausgesät. Die Platten werden für 24 h in einem Zellkulturbrutschrank bei 37°C und 5 % CO₂ inkubiert.

### Tag 2

Zu testende PPAR-Agonisten werden in einer Konzentration von 10 mM in DMSO gelöst. Diese Stocklösung wird in DMEM-Medium (# 41965-039, Invitrogen) verdünnt, welches mit 2 % Ultroser (#12039-012, Biosepra), 1 % Penicillin-Streptomycin-Lösung (#15140-122, Invitrogen) und 2 mM L-Glutamin (#25030-024, Invitrogen) versetzt ist. Testsubstanzen werden in insgesamt 11 verschiedenen Konzentrationen im Bereich von 10 µM bis 100 pM getestet. Potentere Verbindungen werden in Konzentrationsbereichen von 1 µM bis 10 pM geprüft.

Das Medium der an Tag 1 transfizierten und ausgesäten HEK-Zellen wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Jede Platte wird mit einem Standard PPARgamma-Agonisten belegt, der ebenfalls in 11 verschiedenen Konzentrationen verdünnt wird, um die Funktionsfähigkeit des Tests in jeder Einzetplatte nachzuweisen. Die Testplatten werden für 48 h in einem Brutschrank bei 37 °C und 5 % CO₂ inkubiert.

### Tag 4

Nach dem Absaugen des Mediums werden gemäß den Angaben des Herstellers pro well je 50 µl Dual-Glo^{™} Reagens (Dual-Glo^{™} Luciferase Assay System; Firma. Promega) zugegeben, um die Zellen zu lysieren und das Substrat für die in den Zellen gebildete Firefly-Luziferase (Photinus pyralis) zur Verfügung zu stellen. Nach einer 10 minütigen Inkubation im Dunkeln bei Raumtemperatur wird die Firefly-Luziferase-vermittelte Chemilumineszenz im Messgerät gemessen (1 sec. Meßzeit/well; Trilux der Firma Wallac). Danach wird pro well je 50 µl des Dual-Glo^{™} Stop & Glo Reagens (Dual-Glo^{™} Luciferase Assay System; Firma Promega) zugegeben, um die Aktivität der Firefly-Luziferase abzustoppen und das Substrat für die vom Referenzplasmid pRL-CMV aus exprimierten Renilla-Luciferase zur Verfügung zu stellen. Nach einer weiteren 10 minütigen Inkubation im Dunkeln bei Raumtemperatur wird erneut für 1 sec/well die durch die Renilla-Luziferase vermittelte Chemilumineszenz im Messgerät gemessen.

### Auswertung

Die Rohdaten des Lumineszenzmeßgerätes werden in ein Microsoft Excel-File transferiert. Für jeden Meßpunkt, der sich von einem well der Mikrotiterplatte ableitet, wird der Quotient Firefly/Renilla-Luciferaseaktivität bestimmt. Aus den Quotienten werden die Dosis-Wirkungskurven und EC50-Werte von PPAR-Agonisten mit dem Program XL.Fit nach Vorgabe des Herstellers (Firma IDBS) berechnet.

Mit den in dieser Anmeldung beschriebenen PPAR-Agonisten wurden PPARgamma-EC50-Werte im Bereich von 15nM bis >10 µM gemessen.

Die nachfolgend aufgeführten Beispiele dienen zur Erläuterung der Erfindung, ohne diese jedoch einzuschränken

**Tabelle II:**

| | | | | | | | |
|---|---|---|---|---|---|---|---|
| 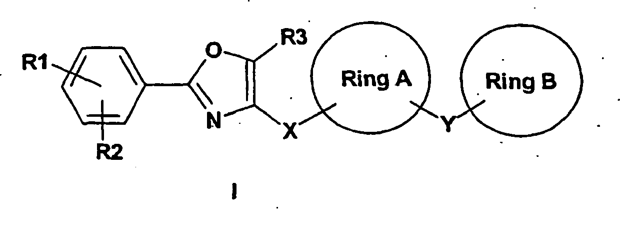 | | | | | | | |

| | **Ring A** | **Ring B** | **R1** | **R2** | **R3** | **X** | **Y** |
|---|---|---|---|---|---|---|---|
| I | 1,3-Cy | 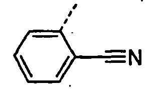 | 4-F | H | Me | CH2-O | -O-CH2- |
| II | 1,3-Cy | 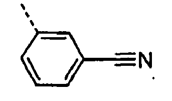 | 4-F | H | Me | CH2-O | -O-CH2- |
| III | 1,3-Cy | 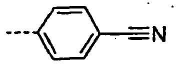 | 4-F | H | Me | CH2-O | -O-CH2-- |
| IV | 1,3-Cy | 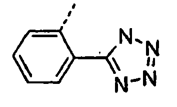 | 4-F | H | Me | CH2-O | -O-CH2- |
| V | 1,3-Cy | 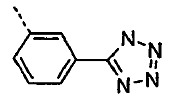 | 4-F | H | Me | CH2-O | -O-CH2- |
| VII | 1,3-Cy | 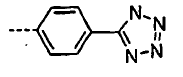 | 4-F | H | Me | CH2-O | -O-CH2- |
| VII | 1,3-Cy | 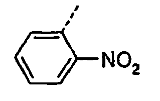 | 4-F | H | Me | CH2-O | -O-CH2- |
| VIII | 1,3-Cy | 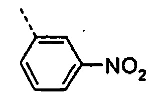 | 4-F | H | Me | CH2-O | -O-CH2- |
| IX | 1,3-Cy | 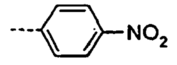 | 4-F | H | Me | CH2-O | -O-CH2- |
| X | 1,3-Cy | 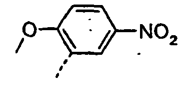 | 4-F | H | Me | CH2-O | -O-CH2- |
| XI | 1,3-Cy | 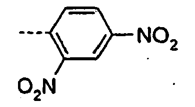 | 4-F | H | Me | CH2-O | -O-C(=O)- |
| XII | 1,3-Cy | 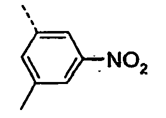 | 4-F | H | Me | CH2-O | -O-C(=O)- |
| XIII | 1,3-Cy | 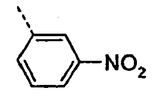 | 4-F | H | Me | CH2-O | -O-C(=O)- |
| XIV | 1,3-Cy | 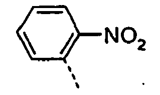 | 4-F | H | Me | CH2-O | -O-C(=O)- |
| XV | 1,3-Cy | 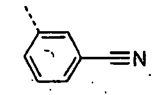 | 4-F | H | Me | CH2-O | -O-C(=O)- |
| XVI | 1,3-Cy | 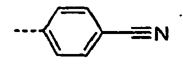 | 4-F | H | Me | CH2-O | -O-C(=O)- |
| XVII | 1,3-Cy | 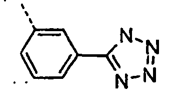 | 4-F | H | Me | CH2-O | -O-C(=O)- |
| XVIII | 1,3-Cy | 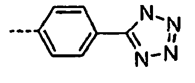 | 4-F | H | Me | CH2-O | -O-C(=O)- |
| XIX | 1,3-Cy | 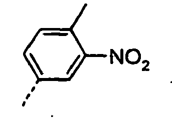 | 4-F | H | Me | CH2-O | -O-S(=O)2- |
| XX | 1,3-Cy | 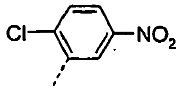 | 4-F | H | Me | CH2-O | -O-S(=O)2- |
| XXI | 1,3-Cy | 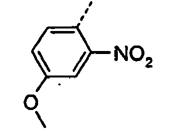 | 4-F | H | Me | CH2-O | -O-S(=O)₂- |
| XXII | 1,3-Cy | 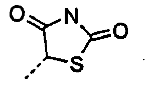 | 4-F | H | Me | CH2-O | -O-CH2-CH= |
| XXIII | 1,3-Cy | 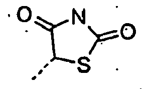 | 3-Me | H | Me | CH2-O | -O-CH2-CH= |
| XXIV | 1,3-Cy | 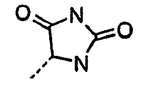 | 4-F | H | Me | CH2-O | -O-CH2-CH= |
| XXV, | 1,3-Cy | 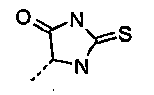 | 4-F | H | Me | CH2-O | -O-CH2-CH= |
| XXVI | 1,3-Cy | 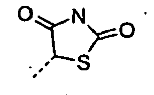 | 4-F | H | Me | CH2-O | -O-CH2-CH- |
| XXVII | 1,3-Cy | 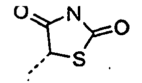 | 3-Me | H | Me | CH2-O | -O-CH2-CH-- |
| XXVIII | 1,3-Cy | 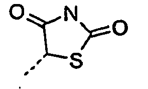 | 3-Me | H | Me | CH2-O | -CH2-CH(OH)- |
| XXIX | 1,3-Cy | 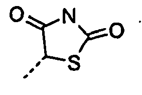 | 3-OMe | H | Me | CH2-O | -CH2-CH(OH)- |
| XXX | 1,3-Cy | 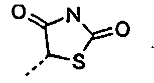 | 4-Me | H | Me | CH2-O | -CH2-CH(OH)- |
| XXXI | 1,3-Cy | 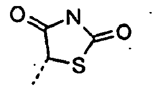 | 3-Me | H | Me | CH2-O | -CH2-CH2- |
| XXXII | 1,3-Cy | 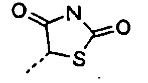 | 3-OMe | H | Me | CH2-O | -CH2-CH2- |
| XXXIII | 1,3-Cy | 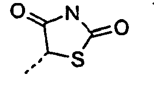 | 4-Me | H | Me | CH2-O | -CH2-CH2- |
| XXXIV | 1,3-Cy | 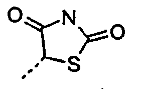 | 4-Me | H | Me | CH2-O | -CH= |
| XXXV | 1,3-Cy | 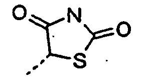 | 4-Me | H | Me | CH2-O | -CH2- |
| XXXVI | 1,3-Cy | 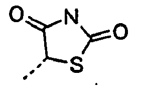 | 3-OMe | H | Cy | CH2-O | -CH2-CH2- |
| XXXVII | 1,3-Cy | 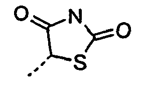 | 4-Me | H | Cy | CH2-O | -CH2-CH2- |
| XXXVII | 1,3-Cy | 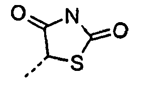 | 3-CF₃ | 5-CF₃ | Et | CH2-O | -CH2-CH2- |
| XXXIX | 1,3-Cy | 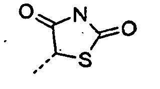 | 2-Me | 6-Me | Et | CH2-O | -CH2-CH2- |
| XL | 1,3-Cy | 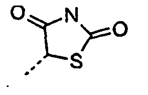 | 2-CF₃ | H | Me | CH2-O | -CH2-CH2- |
| XLI | 1,3-Cy | 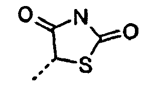 | 3-OMe | H | Et | CH2-O | -CH2-CH2- |
| XLII | 1,3-Cy | 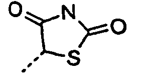 | 2-CF₃ | H | Et | CH2-O | -CH2-CH2- |
| XLIII | 1,3-Cy | 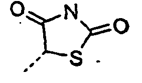 | 4-Me | H | Et | CH2-O | -CH2-CH2- |
| XLIV | 1,3-Cy | 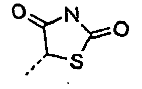 | 4-'Pr | H | Et | CH2-O | -CH2-CH2- |
| XLV | 1,3-Cy | 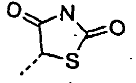 | 4-Me | H | ⁱPr | CH2-O | --CH2-CH2- |
| XLVI | 1,3-Cy | 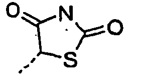 | 3-CF₃ | H | Me | CH2-O | -CH2-CH2-- |
| XLVIIa¹ | 1,3-Cy | 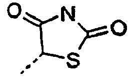 | 3-Me | H | Me | CH2-O | -CH2-CH2- |
| XLVIIb ² | -1,3-Cy | 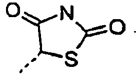 | 3-Me | H | Me | CH2-O | -CH2-CH2- |
| XLVIIa² | 1,3-Cy | 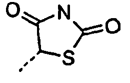 | 4-'Pr | H | Et | CH2-O | -CH2-CH2- |
| XLVIIIb¹ | 1,3-Cy | 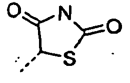 | 4-'Pr | H | Et | CH2-O | --CH2-CH2- |
| XLIXa² | 1,3-Cy | 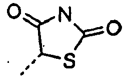 | 4-Me | H | Me | CH2-O | -CH2-CH2- |
| XLIXb¹ | 1,3-Cy | 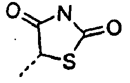 | 4-Me | H | Me | CH2-O | -CH2-CH2- |
| L² | 1,3-Cy | 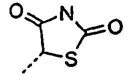 | 3-Me | 4-Me | Et | CH2-O | -CH2-CH2- |
| LI² | 1,3-Cy | 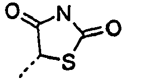 | 4-CF₃ | H | Et | CH2-O | -CH2-CH2- |
| LII ² | 1,3-Cy | 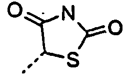 | 2-Naphthyl | | Et | CH2-O | -CH2-CH2- |
| LIII ² | 1,3-Cy | 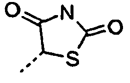 | 3-CF₃ | H | Et | CH2-O | -CH2-CH2- |
| LIV ² | 1,3-Cy | 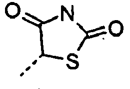 | 4-^{t}Bu | H | Et | CH2-O | -CH2-CH2- |
| LV² | 1,3-Cy | 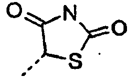 | 3-Me | 4-Me | ⁱPr | CH2-O | -CH2-CH2- |
| LVI² | 1,3-Cy | 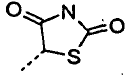 | 4-ⁱBu | H | Et | CH2-O | -CH2-CH2- |
| LVII² | 1,3-Cy | 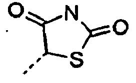 | 3-CF₃ | H | ⁱPr | CH2-O | -CH2-CH2- |
| LVIII² | 1,3-Cy | 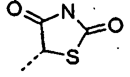 | 4-^{t}Bu | H | ⁱPr | CH2-O | -CH2-CH2- |
| LIX² | 1,3-Cy | 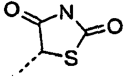 | 4-ⁱBu | H | ⁱPr | CH2-O | -CH2-CH2-- |
| LX² | 1,3-Cy | 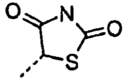 | 4-CF₃ | H | ⁱPr | CH2-O | --CH2-CH2- |
| LXI² | 1,3-Cy | 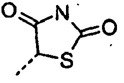 | 2-Naphthyl | | ⁱPr | CH2-O | -CH2-CH2- |
| LXI | 1,3-Cy | 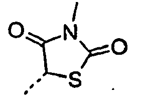 | 3-OMe | H | Me | CH2-O | --CH2-CH2-- |
| LXIII | 1,3-Cy | 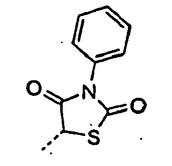 | 3-OMe | H | Me | CH2-O | --CH2-CH2- |
| LXIV | 1,3-Cy | 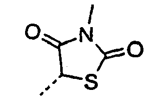 | 4-Me | H | Me | CH2-O | --CH2-CH2- |
| LXV | 1,3-Cy | 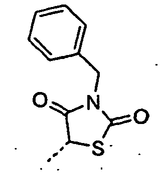 | 4-Me | H | Me | CH2-O | -CH2-CH2- |
| LXVI | 1,3-Cy | 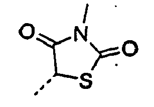 | 3-OMe | H | ¹Pr | CH2-O | -CH2-CH2- |
| LXVII | 1,3-Cy | 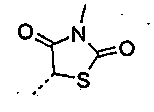 | 3-OMe | H | Ph | CH2-O | -CH2-CH2- |
| LXVIII | 1,3-Cy | 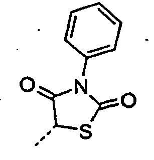 | 3-OMe | H | Ph | CH2-O | --CH2-CH2-- |
| | | | | | | | |

| | | | | | | | |
|---|---|---|---|---|---|---|---|
| 1,3 Cy ist definiert als: cis-1,3 Cyclohexandiyl mit der Stereochemie nach Cahn-Ingold-Prelog, wie sie in den Beispielen angegeben ist. Die Verknüpfung von Ring B zu Y sowie Verknüpfungen von Y zu Ring A und Ring B werden durch eine gestrichelte Linie (--) dargestellt. ¹ (1R, 3R)-Enantiomer ² (1S, 3S)-Enantiomer | | | | | | | |

Beschrieben ist weiterhin ein Verfahren zur Herstellung der Verbindungen der allgemeinen Formel I entsprechend den folgenden Reaktionsschemata A bis H:

### Syntheseschema A: Darstellung der enantiomerenreinen oder racemischen allgemeinen Verbindung A9

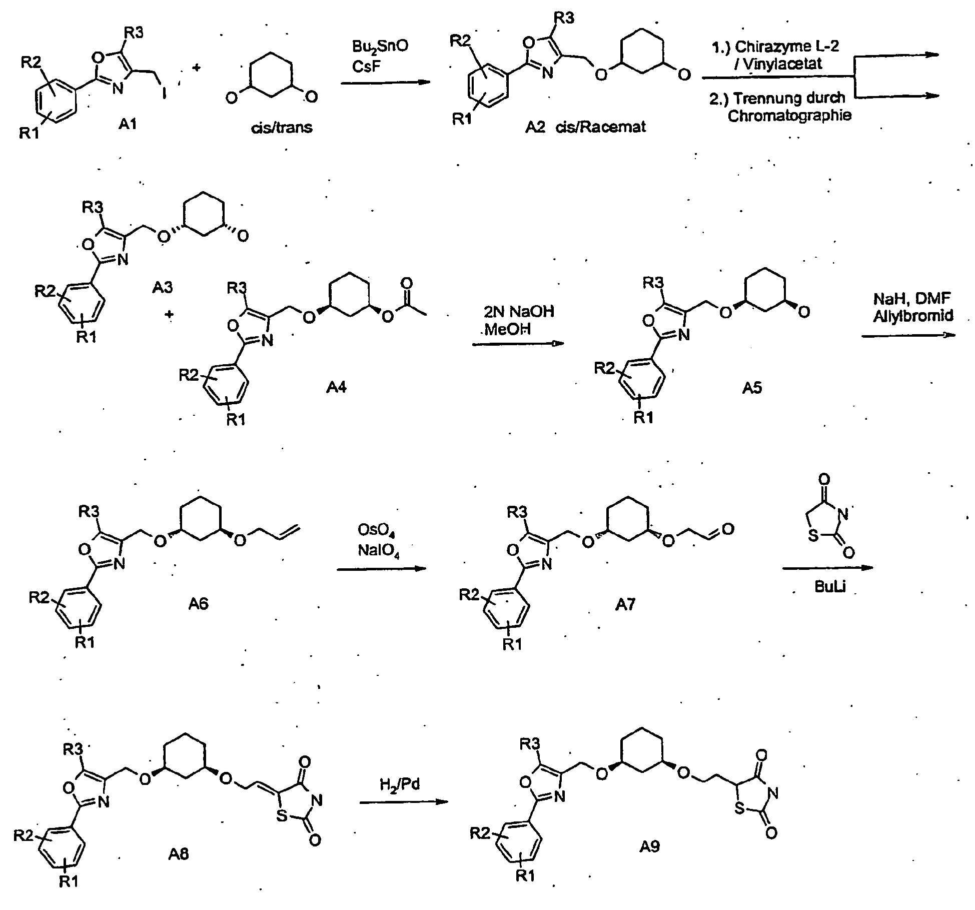

Es wird Cyclohexandiol zunächst mit Dibutylzinnoxid in Toluol mehrere Stunden am Wasserabscheider erhitzt und dann unter Zusatz von Dimethylformamid, Cäsiumfluorid und einem Oxazol der allgemeinen Formel **A1,** worin R1, R2 und R3 die oben beschriebenen Bedeutungen haben, durch mehrstündiges Rühren bei Raumtemperatur zu einer Verbindung der allgemeinen Formel **A2** umgesetzt, worin R1, R2, R3 die oben beschriebenen Bedeutungen haben.

Die Verbindung der allgemeinen Formel **A2** wird unter Verwendung von Chirazym L2 und Vinylacetat umgesetzt. Dabei entstehen die Verbindungen **A3** und **A4,** von denen **A4,** nach Trennung, mit Alkalihydroxiden zu einer Verbindung der allgemeinen Struktur **A5** umgesetzt wird, worin R1, R2, R3 die oben beschriebenen Bedeutungen haben.

Die Verbindung der allgemeinen Formel **A5** oder **A2** wird zu einer enantiomerenreinen oder racemischen Verbindung der allgemeinen Struktur **A6** umgesetzt, worin R1, R2, R3 die oben beschriebenen Bedeutungen haben. Zur Knüpfung der Etherbindung wird **A5** oder A2 beispielsweise in einem aprotischen Lösungsmittel wie Dimethylformamid unter Verwendung von starken Basen, z. B. Natriumhydrid deprotoniert und mit ungesättigten Bromiden, z. B. Allylbromid umgesetzt.

Die enantiomerenreine oder racemische Verbindung der allgemeinen Formel **A6** wird unter Verwendung von Osmiumtetroxid und Natriumperiodat zu der enantiomerenreinen oder racemischen Verbindung der allgemeinen Struktur **A7** umgesetzt, worin R1, R2, R3 die oben beschriebenen Bedeutungen haben.

Die enantiomerenreine oder racemische Verbindung der allgemeinen Formel **A7** wird zu enantiomerenreinen oder racemischen Verbindungen der allgemeinen Struktur **A8** umgesetzt, worin R1, R2, R3 die oben beschriebenen Bedeutungen haben. Dabei wird Thiazolidindion zuerst in einem inerten Lösungsmittel mit einer starken Base, z.B. n-Butyllithium, deprotoniert und anschließend mit der Komponente **A7** bei -70 °C umgesetzt, wobei nach saurer Aufarbeitung mit z.B. 6N Salzsäure, die Verbindung **A8** entsteht.

Die enantiomerenreine oder racemische Verbindung **A8** wird durch Hydrierung, beispielsweise durch Palladium auf Kohle als Katalysator in Lösungsmitteln wie Methanol oder Essigsäureethylester, zu einer enantiomerenreinen oder racemischen Verbindung der allgemeinen Formel **A9** umgesetzt, worin R1, R2, R3 die oben beschriebenen Bedeutungen haben.

### Syntheseschema B: Darstellung der allgemeinen Verbindung B8

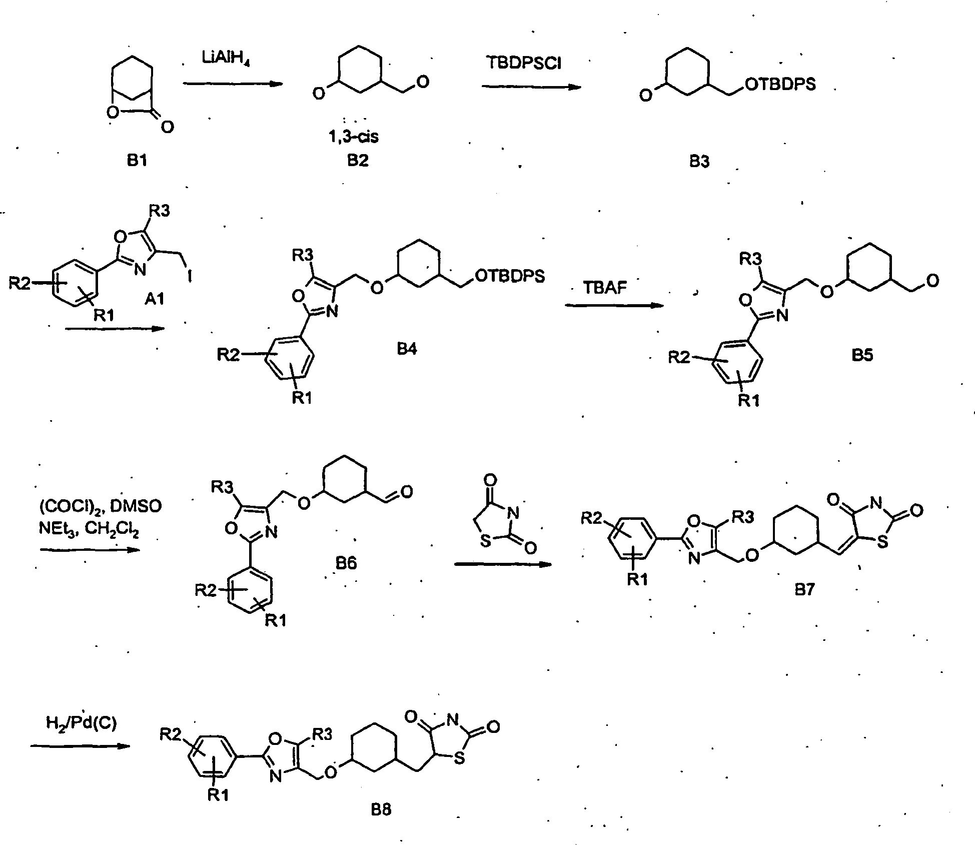

Die Verbindung der allgemeinen Formel **B4,** worin R1, R2 und R3 die oben genannten Bedeutungen haben, wird aus dem Lacton **B1** durch Lithiumalanat - Reduktion zum Diol **B2,** selektive Silylierung an der primären Alkoholfunktion zu Verbindung **B3,** Deprotonierung unter Verwendung von starken Basen, z. B. Natriumhydrid in einem aprotischen Lösungsmittel wie Dimethylformamid und Alkylierung mit Phenyloxazoyliodiden der allgemeinen Formel **A1,** worin R1, R2 und R3 die oben genannten Bedeutungen haben, gewonnen.

Die Verbindung der allgemeinen Formel **B4,** worin R1, R2 und R3 die oben genannten Bedeutungen wird zu einer Verbindung der allgemeinen Struktur **B5** umgesetzt, worin R1, R2, R3, die oben beschriebenen Bedeutungen haben, beispielsweise durch Entfernen der Silylschutzgruppe mit Fluorid, z.B. Tetrabutylammoniumfluorid.

Die Verbindung der allgemeinen Formel B5 wird unter Verwendung von Osmiumtetroxid und Natriumperiodat zu der Verbindung der allgemeinen Struktur **B6** umgesetzt, worin R1, R2, R3 die oben beschriebenen Bedeutungen haben.

Die Verbindung der allgemeinen Formel **B6** wird zu einer Verbindung der allgemeinen Struktur **B7** umgesetzt, worin R1, R2, R3 die oben beschriebenen Bedeutungen haben. Dabei wird Thiazolidindion zuerst in einem inerten Lösungsmittel mit einer starken Base, wie z. B. n-Butyllithium, deprotoniert und anschließend mit der Komponente B6 bei -70 °C umgesetzt, wobei nach saurer Aufarbeitung mit z. B. 6N Salzsäure, die Verbindung **B7** entsteht.

Die Verbindung **B7** wird durch Hydrierung, beispielsweise bei 3 bar Wasserstoffdruck durch Palladium auf Kohle als Katalysator in Lösungsmitteln wie Methanol oder Essigsäureethylester, zu einer Verbindung der allgemeinen Formel **B8** umgesetzt, worin R1, R2, R3 die oben beschriebenen Bedeutungen haben.

### Syntheseschema C: Darstellung der allgemeinen Verbindung C6

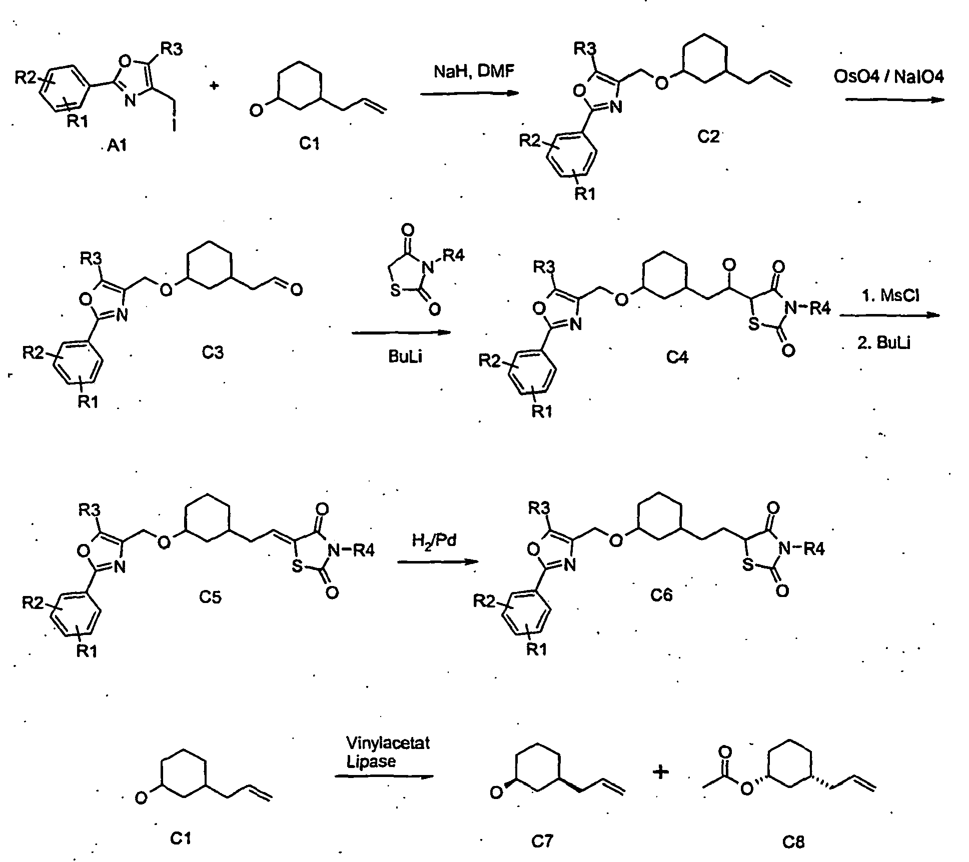

Es werden Verbindungen der allgemeinen Formel **A1,** worin R1, R2, R3 die oben beschriebenen Bedeutungen haben, mit cis-3-Allyl-cyclohexanol **C1** (oder wahlweise mit (1 S, 3S)-3-Allylcyclohexanoi **C7**) in aprotischen Lösungsmitteln wie Dimethylformamid gelöst und mit starken Basen wie z. B. Natriumhydrid zu Verbindungen der allgemeinen Formel **C2,** worin R1, R2, R3 die oben beschriebenen Bedeutungen haben, umgesetzt.

Die Verbindung der allgemeinen Formel **C2** wird unter Verwendung von Osmiumtetroxid und Natriumperiodat zu der Verbindung der allgemeinen Struktur **C3** umgesetzt, worin R1, R2, R3 die oben beschriebenen Bedeutungen haben.

Die Verbindung der allgemeinen Formel **C3** wird zu einer Verbindung der allgemeinen Struktur **C4** umgesetzt, worin R1, R2, R3 und R4 die oben beschriebenen Bedeutungen haben. Dabei wird Thiazolidindion zuerst in einem inerten Lösungsmittel mit einer starken Base, wie z. B. n-Butyllithium, deprotoniert und anschließend mit der Komponente **C3** bei -70°C umgesetzt, wobei nach Aufarbeitung mit z. B. 1N Salzsäure, die eine Verbindung der allgemeinen Formel **C4** entsteht.

Die Verbindung **C4** wird zu einer Verbindung der allgemeinen Formel **C5** umgesetzt, worin R1, R2, R3 und R4 die oben beschriebenen Bedeutungen haben.

Der Alkohol **C4** wird beispielsweise mit Mesylchlorid und Triethylamin in polaren Lösungsmitteln wie Dichlormethan versetzt und das Rohprodukt bei -70 °C mit n-Butyllithium zu **C5** umgesetzt.

Die Verbindung der allgemeinen Formel **C5** wird durch Hydrierung, beispielsweise bei 5 bar Wasserstoffdruck mit Palladium auf Kohle als Katalysator in Essigsäureethylester, zu einer Verbindung der allgemeinen Formel **C6** umgesetzt, worin R1, R2, R3 und R4 die oben beschriebenen Bedeutungen haben.

Enantiomerenreines (1S, 3S)-3-Allylcyclohexanol **C7** kann man aus racemischem **C1** durch Behandlung mit Lipase in Vinylacetat erhalten. Das ebenfalls entstehende (1R, 3R)-3-Allylcyclohexanylacetat **C8** kann chromatographisch abgetrennt werden.

### Syntheseschema D: Darstellung der allgemeinen Verbindung D2

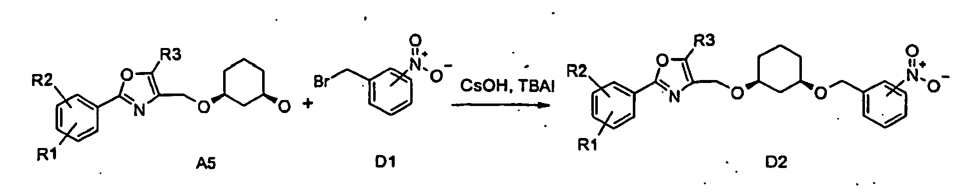

Die Verbindung der allgemeinen Formel **A5** oder **A2** wird unter Verwendung von Basen, z. B. Cäsiumhydroxid in einem Gemisch aus Wasser und Acetonitril, unter Verwendung eines Phasen - Transfer - Katalysators , beispielsweise Tetrabutylammoniumiodid, mit Nitrobenzylbromiden der allgemeinen Formel **D1** zu enantiomerenreinen oder racemischen Verbindungen der allgemeinen Struktur **D2** umgesetzt, worin R1, R2, R3 die oben beschriebenen Bedeutungen haben.

### Syntheseschema E: Darstellung der enantiomerenreinen oder racemischen allgemeinen Verbindung E3

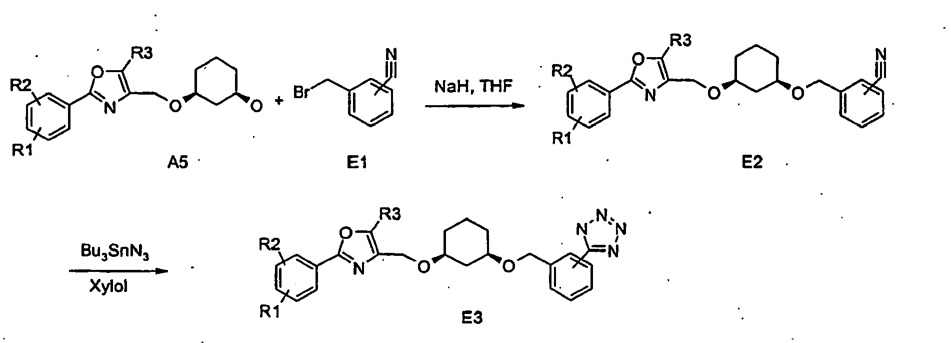

Die Verbindung der allgemeinen Formel **A5** oder **A2** wird unter Verwendung von starken Basen, z. B. Natriumhydrid in einem aprotischen Lösungsmittel deprotoniert und mit Cyanobenzylbromiden der allgemeinen Formel **E1** zu enantiomerenreinen oder racemischen Verbindungen der allgemeinen Struktur **E2** umgesetzt, worin R1, R2, R3 die oben beschriebenen Bedeutungen haben.

Die Verbindung der allgemeinen Formel **E2** wird unter Verwendung eines Metallazides, z. B. Tributylzinnazid, zu enantiomerenreinen oder racemischen Verbindungen der allgemeinen Struktur **E3** umgesetzt, worin R1, R2, R3, die oben beschriebenen Bedeutungen haben.

### Syntheseschema F: Darstellung der enantiomerenreinen oder racemischen allgemeinen Verbindung F2

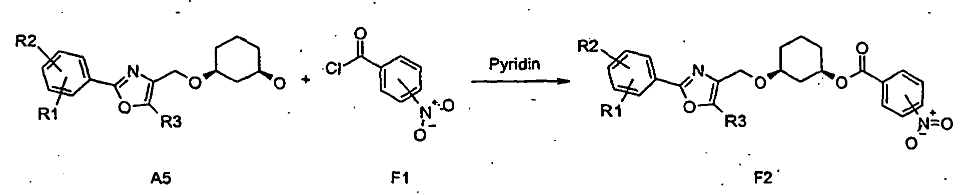

Die Verbindung der allgemeinen Formel **A5** oder **A2** wird unter Verwendung von Basen, z. B. Pyridin, mit Nitrobenzoylchloriden der allgemeinen Formel **F1** bei ca. 50 °C umgesetzt zu enantiomerenreinen oder racemischen Verbindungen der allgemeinen Formel **F2,** worin R1, R2, R3 die oben beschriebenen Bedeutungen haben.

### Syntheseschema G: Darstellung der enantiomerenreinen oder racemischen allgemeinen Verbindung G3

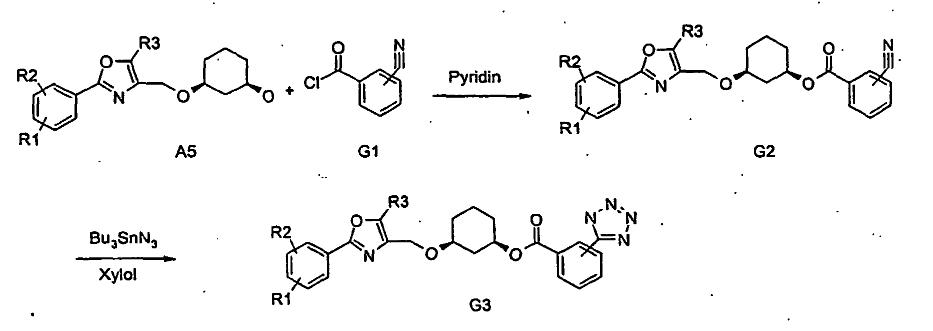

Die Verbindung der allgemeinen Formel **A5** oder **A2** wird unter Verwendung von Basen, z. B. Pyridin, mit Cyanobenzoylchloriden der allgemeinen Formel **G1** bei ca. 50 °C umgesetzt zu enantiomerenreinen oder racemischen Verbindungen der allgemeinen Formel G2, worin R1, R2, R3 die oben beschriebenen bedeutungen haben.

Die Verbindung der allgemeinen Formel **G2** wird unter Verwendung eines Metallazides, z. B. Tributylzinnazid, bei ca. 160°C zu enantiomerenreinen oder racemischen Verbindungen der allgemeinen Struktur **G3** umgesetzt, worin R1, R2, R3, die oben beschriebenen Bedeutungen haben.

### Syntheseschema H: Darstellung der enantiomerenreinen oder racemischen allgemeinen Verbindung H2

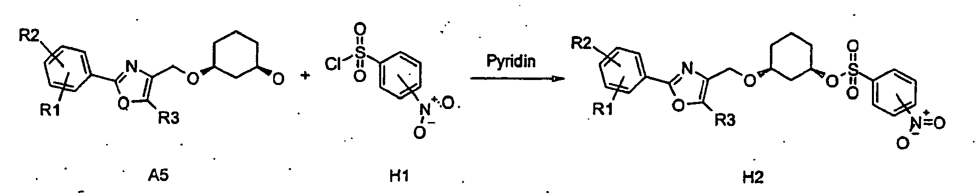

Die Verbindung der allgemeinen Formel **A5** oder **A2** wird unter Verwendung von Basen, z. B. Pyridin, mit Nitrobenzolsulfonsäurechloriden der allgemeinen Formel **G1** bei Raumtemperatur umgesetzt zu enantiomerenreinen oder racemischen Verbindungen der allgemeinen Formel **G2,** worin R1, R2, R3 die oben beschriebenen Bedeutungen haben.

Die verwendeten Abkürzungen stehen für:
- Ac: Acetyl
- ⁱBu: Isobutyl
- ^{t}Bu: tert-Butyl
- BuLi: n-Butyllithium
- Cy: Cyclohexyl
- DC: Dünnschichtchromatographie
- DCI: direkte chemische Ionisation (bei MS)
- DCM: Dichlormethan
- DMAP: 4-N,N-Dimethylaminopyridin
- DMF: N,N-Dimethylformamid
- DMSO: Dimethylsulfoxid
- EE: Essigsäureethylester
- EDC: N'-(3-Dimethylaminopropyl)-N-ethylcarbodiimid x HCl
- EI: Elektronenstoß-Ionisation (bei MS)
- eq: Äquivalent
- ESI: Elektronenspray-Ionisation (bei MS)
- Et: Ethyl
- ges.: gesättigt
- h: Stunde
- HATU: O-(7-Azabenzotriazol-1-yl)-N,N,N',N'-tetramethyluronium- Hexafluorophosphat
- HOBt: 1-Hydroxy-1H-benzotriazol x H₂O
- HPLC: Hochdruck-, Hochleistungsflüssigchromatographie
- LC-MS: Flüssigchromatographie-gekoppelte Massenspektroskopie
- Me: Methyl
- MS: Massenspektroskopie
- MsCl: Methansulfonylchlorid
- NMR: Kernresonanzspektroskopie
- Pd/C: Palladium auf Kohle
- ⁱPr: Isopropyl
- ⁿPr: n-Propyl
- R_{f}: Retentionszeit (bei DC)
- RT: Raumtemperatur
- TBAF: Tetrabutylammoniumfluorid
- TBAI: Tetrabutylammoniumiodid
- TBDPSCI: tert.-Butyl-di-Phenyl-silyl-chlorid
- THF: Tetrahydrofuran

Andere Verbindungen der Formel I können nach bekannten Verfahren oder entsprechend den oben beschriebenen Reaktionsschemata erhalten werden.

### Beispiel I

### 2-(4-Fluoro-phenyl)-5-methyl-4-[cis-3-(2-cyano-benzyloxy)-cyclohexyloxymethyl]-oxazol

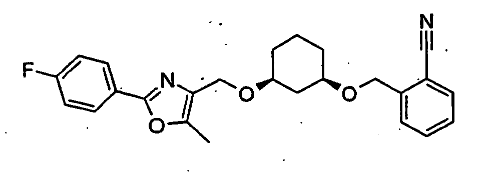

### rac-3-(cis-5-Methyl-2-m-tolyl-oxazol-4-ylmethoxy)-cyclohexanol

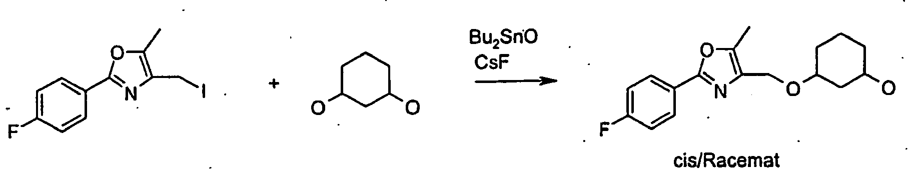

21.7 g 1,3-Cyclohexandiol werden mit 30.3 g Dibutylzinnoxid in450 ml Toluol gelöst und unter Rückfluß am Wasserabscheider zum Sieden erhitzt. Das Reaktionsvolumen wird während der Reaktionsdauer auf die Hälfte reduziert. Nach 3 Stunden wird das Reaktionsgemisch auf Raumtemperatur gekühlt und mit 300 ml Dimethylformamid, 29 g 2-(4-Fluoro-phenyl)-4-iodomethyl-5-methyl-oxazol 1 und 23.5 g Cäsiumfluorid versetzt. Man rührt 18 Stunden bei Raumtemperatur nach. Das Reaktionsgemisch wird durch Zugabe von Ethylacetat verdünnt und mit gesättigter Natriumchlorid - Lösung gewaschen. Die organische Phase wird über Magnesiumsulfat getrocknet, das Lösungsmittel im Vakuum entfernt und der Rückstand durch Flash-Chromatographie an Kieselgel (n-Heptan/Ethylacetat = 10:1 → 1:4) gereinigt. Man erhält 58 g rac-3-[2-(4-Fluoro-phenyl)-5-methyl-oxazol-4-ylmethoxy]-cyclohexanol als gelblichen Feststoff, der aus n-Heptan/Ethylacetat umkristallisiert wird. C₁₇H₂₀FNO₃ (305.35), MS (ESI): 306 (M + H⁺).

### (1R,3S)-3-[2-(4-Fluoro-phenyl)-5-methyl-oxazol-4-ylmethoxy]-cyclohexanol

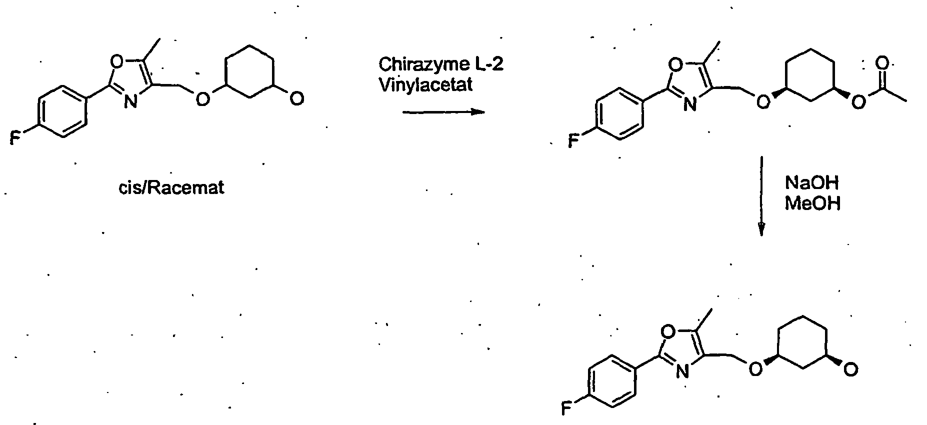

25 g rac-cis-3-[2-(4-Fluoro-phenyl)-5-methyl-oxazol-4-ylmethoxy]-cyclohexanol werden in 320 ml Vinylacetat gelöst und mit 1,3 g Chirazyme L-2 Lyo (Boehringer Mannheim) versetzt. Nach dreistündigem Rühren bei Raumtemperatur (LC-MS Kontrolle auf 40-45% Umsatz) wird das Enzym abfiltriert, mit Ethylacetat nachgewaschen und das Lösungsmittel im Vakuum entfernt. Der Rückstand wird durch Flash-Chromatographie an Kieselgel (n-Heptan/Ethylacetat = 3:1) gereinigt. Man erhält 8 g Essigsäure-(1R,3S)-3-[2-(4-fluoro-phenyl)-5-methyl-oxazol-4-ylmethoxy]-cyclohexylester als farbloses Öl. C₁₉H₂₂FNO₄ (347.39), MS (ESI): 348 (M + H⁺). Man nimmt das Acetat in 170 ml Methanol auf und rührt nach Zugabe von 27 ml 2N Natriumhydroxid-Lauge für eine Stunde bei Raumtemperatur. Der größte Teil des Lösungsmittels wird im Vakuum entfernt. Nach Zugabe von je 150 ml Wasser und Ethylacetat, wird die organische Phase mit Natriumchlorid - Lösung gewaschen. Die organische Phase wird über Magnesiumsulfat getrocknet, das Lösungsmittel im Vakuum entfernt. Man erhält 6,7 g 3-(1R,3S)-3-[2-(4-Fluoro-phenyl)-5-methyl-oxazol-4-ylmethoxy]-cyclohexanol als gelblichen Feststoff. C₁₇H₂₀FNO₃ (305.35), MS (ESI): 306 (M + H⁺).

### 2-(4-Fluoro-phenyl)-5-methyl-4-[cis-3-(2-cyano-benzyloxy)-cyclohexyloxymethyl]-oxazol

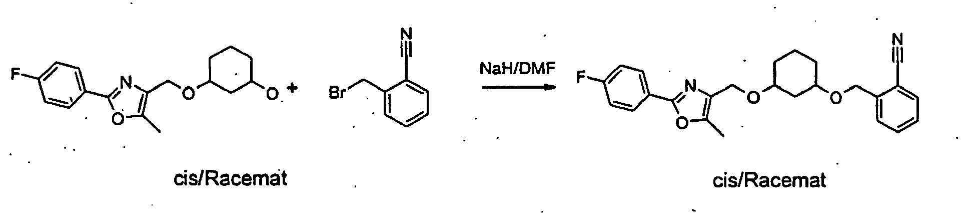

In einem ausgeheizten 25 ml-Zweihalskolben werden 0.15 g des Alkohols 3-[2-(4-Fluoro-phenyl)-5-methyl-oxazol-4-ylmethoxy]-cyclohexanol in 5 ml Dimethylformamid (trocken) gelöst und mit 0,05 g Natriumhydrid versetzt. Es wird 15 Minuten gerührt ,anschließend 0,19 g 2-(Brommethyl)-benzonitril zugegeben und 24 Stunden bei Raumtemperatur gerührt. Die Reaktion wird durch Zugabe von 2 ml 1 N Salzsäure abgebrochen und mit Ethylacetat (2x10 ml) extrahiert. Die vereinigten organischen Phasen werden mit gesättigter Natriumchlorid - Lösung gewaschen, über Magnesiumsulfat getrocknet und das Lösungsmittel anschließend im Vakuum entfernt. Durch Reinigung über präparative HPLC erhält man 0,07 g des gewünschten Produktes 2-(4-Fluoro-phenyl)-5-methyl-4-[cis-3-(2-cyano-benzyloxy)-cyclohexyloxymethyl]-oxazol als farbloses Öl.
C₂₅H₂₅FN₂O₃ (420.48), MS(ESI): 421 (M + H⁺)

### Beispiel II

### 2-(4-Fluoro-phenyl)-5-methyl-4-[cis-3-(3-cyano-benzyloxy)-cyclohexyloxymethyl]-oxazol

Analog zu Beispiel I wird aus dem Alkohol 3-[2-(4-Fluoro-phenyl)-5-methyl-oxazol-4-ylmethoxy]-cyclohexanol und 3-(Brommethyl)-benzonitril die Verbindung 2-(4-Fluoro-phenyl)-5-methyl-4-[cis-3-(3-cyano-benzyloxy)-cyclohexyloxymethyl]-oxazol erhalten: 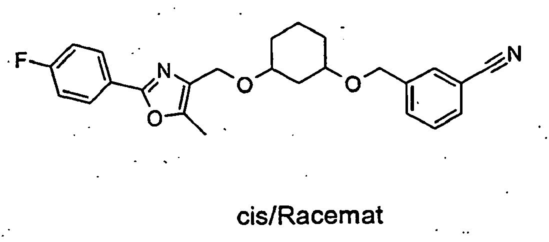 C₂₅H₂₅FN₂O₃ (420.48), MS(ESI): 421 (M + H⁺)

### Beispiel III

### 2-(4-Fluoro-phenyl)-5-methy-4-[cis-3-(4-cyano-benzyloxy)-cyclohexyloxymethyl]-oxazol

Analog zu Beispiel I wird aus dem Alkohol 3-[2-(4-Fluoro-phenyl)-5-methyl-oxazol-4-ylmethoxy]-cyclohexanol und 4-(Brommethyl)-benzonitril die Verbindung 2-(4-Fluoro-phenyl)-5-methyl-4-[cis-3-(4-cyano-benzyloxy)-cyclohexyloxymethyl]-oxazol erhalten: 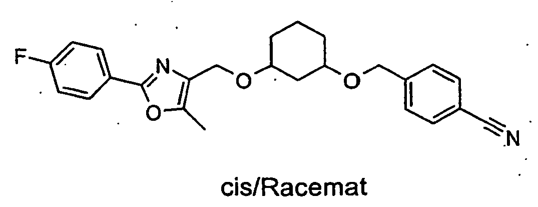 C₂₅H₂₅FN₂O₃ (420.48), MS(ESI): 421 (M + H⁺)

Die so synthetisierten Verbindungen (Beispiel I - III) können in die entsprechenden Tetrazole umgewandelt werden:

### Beispiel IV

### 5-(2-{cis-3-[2-(4-Fluoro-phenyl)-5-methyl-oxazol-4-ylmethoxy]-cyclohexyloxymethyl)-phenyl)-1-H-tetrazol

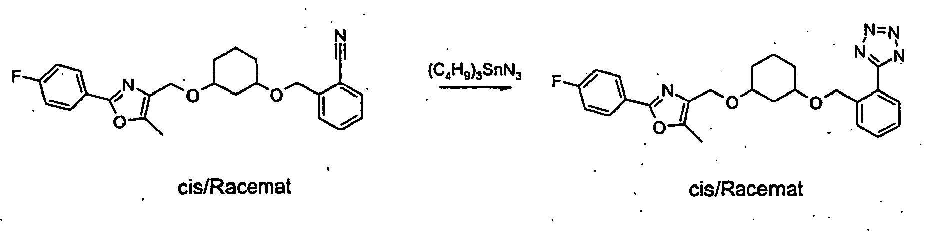

0,03 g des Nitrils 2-(4-Fluoro-phenyl)-5-methyl-4-[cis-3-(2-cyano-benzyloxy)-cyclohexyloxymethyl]-oxazol werden in 5 ml Xylol gelöst, mit 50 µl Tributylzinnäzid versetzt und 24 Stunden bei 160°C refluxiert. Die Reaktion wird durch Zugabe von 1 ml Trifluoressigsäure (in 1 ml Methanol) abgebrochen, mit 3 ml Wasser versetzt und mit Ethylacetat (2 x 10 ml) extrahiert. Die vereinigten organischen Phasen werden mit gesättigter Natriumclorid - Lösung gewaschen, über Magnesiumsulfat getrocknet und das Lösungsmittel anschließend im Vakuum entfernt. Durch Reinigung über präparative HPLC erhält man 0,02 g des gewünschten 5-(2-{cis-3-[2-(4-Fluoro-phenyl)-5-methyl-oxazol-4-ylmethoxy]-cyclohexyloxymethyl}-phenyl)-1-H-tetrazols als amorphen Feststoff. C₂₅H₂₆FN₅O₃ (463.51), MS(ESI): 464 (M + H⁺)

### Beispiel V

### 5-(3-{cis-3-[2-(4-Fluoro-phenyl)-5-methyl-oxazol-4-ylmethoxy]-cyclohexyloxymethyl}-phenyl)-1H-tetrazol

Analog zu Beispiel IV wurde aus 2-(4-Fluoro-phenyl)-5-methyl-4-[cis-3-(3-cyano-benzyloxy)-cyclohexyloxymethyl]-oxazol aus Beispiel II durch Umsetzung mit Tributylzinnhydrid 5-(3-{cis-3-[2-(4-Fluoro-phenyl)-5-methyl-oxazol-4-ylmethoxy]-cyclohexyloxymethyl}-phenyl)-1H-tetrazol erhalten: 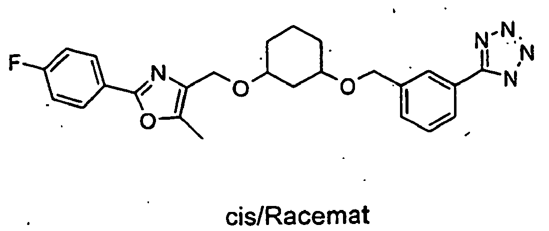 C₂₅H₂₆FN₅O₃ (463.51), MS(ESI): 464 (M+H⁺)

### Beispiel VI

### 5-(4-{cis-3-[2-(4-Fluoro-phenyl)-5-methyl-oxazol-4-ylmethoxy]-cyclohexyloxymethyl}-phenyl)-1H-tetrazol

Analog zu Beispiel IV wurde aus 2-(4-Fluoro-phenyl)-5-methyl-4-[cis-3-(4-cyano-benzyloxy)-cyclohexyloxymethyl]-oxazol aus Beispiel III durch Umsetzung mit Tributylzinnhydrid 5-(4-{cis-3-[2-(4-Fluoro-phenyl)-5-methyl-oxazol-4-ylmethoxy]-cyclohexyloxymethyl}-phenyl)-1H-tetrazol erhalten: 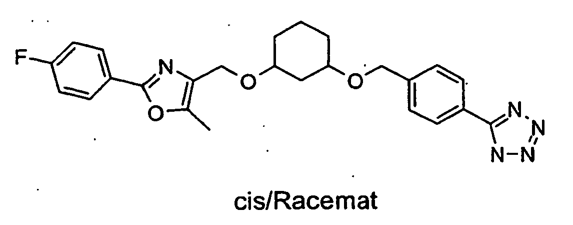 C₂₅H₂₆FN₅O₃ (463.51), MS(ESI): 464 (M + H⁺)

### Beispiel VII

### 2-(4-Fluoro-phenyl)-5-methyl-4-[cis-3-(2-nitro-benzyloxy)-cyclohexyloxymethyl]-oxazol

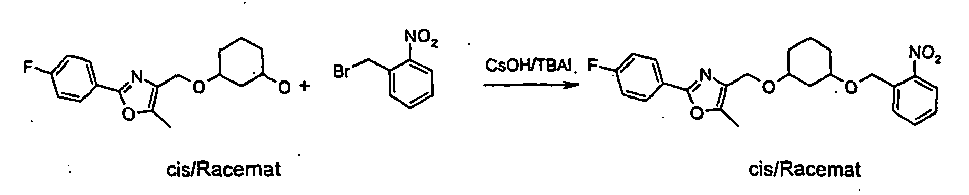

0,1 g 3-[2-(4-Fluoro-phenyl)-5-methyl-oxazol-4-ylmethoxyl-cyclohexanol werden in 3 ml Acetonitril gelöst und mit 0,21 g 2-Nitrobenzylbromid sowie 0,36 g Tetrabutylammoniumiodid versetzt. 0,57 ml Cäsiumhydröxid - Lösung (50%ige Lösung in Wasser) wird zugetropft und das Zwei-Phasen-Gemisch 12 Stunden kräftig bei Raumtemperatur gerührt. Reaktionskontrolle (LCMS) zeigt die Bildung des gewünschten Produktes neben unumgesetztem Alkohol 3-[2-(4-Fluoro-phenyl)-5-methyl-oxazol-4-ylmethoxy]-cyclohexanol. Durch Zugabe von weiteren 0,2 g 2-Nitrobenzylbromid (2 eq) bei Raumtemperatur und weiteren 12 Stunden Rühren bei Raumtemperatur wird die Reaktion durch Zugabe von 2 ml 1 N Salzsäure abgebrochen und mit Ethylacetat (2 x 10 ml) extrahiert. Die vereinigten organischen Phasen werden mit gesättigter Natriumchlorid - Lösung gewaschen und über Magnesiumsulfat getrocknet. Nach HPLC - Reinigung erhält man 0,05 g der Verbindung 2-(4-Fluoro-phenyl)-5-methyl-4-[cis-3-(2-nitro-benzyloxy)-cyclohexyloxymethyl]-oxazol als klares farbloses Öl.
C₂₄H₂₅FN₂O₅ (440,47), MS (ESI): 441 (M + H⁺)

### Beispiel VIII

### 2-(4-Fluoro-phenyl)-5-methyl-4-[cis-3-(3-nitro-benzyloxy)-cyclohexyloxymethyl]-oxazol

Analog zu Beispiel VII wurde aus 3-[2-(4-Fluoro-phenyl)-5-methyl-oxazol-4-ylmethoxy]-cyclohexanol und 3-Nitrobenzylbromid die folgende Verbindung 2-(4-Fluoro-phenyl)-5-methyl-4-[cis-3-(3-nitro-benzyloxy)-cyclohexyloxymethyl]-oxazol erhalten: 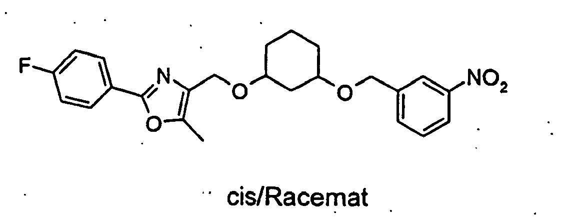 C₂₄H₂₅FN₂O₅ (440.47), MS (ESI): 441

### Beispiel IX

### 2-(4-Fluoro-phenyl)-5-methyl-4-[cis-3-(4-nitro-benzyloxy)-cyclohexyloxymethyl]-oxazol

Analog zu Beispiel VII wurde aus 3-[2-(4-Fluoro-phenyl)-5-methyl-oxazol-4-ylmethoxy]-cyclohexanol und 4-Nitrobenzylbromid die folgende Verbindung 2-(4-Fluoro-phenyl)-5-methyl-4-[cis-3-(4-nitro-benzyloxy)-cyclohexyloxymethyl]-oxazol erhalten: 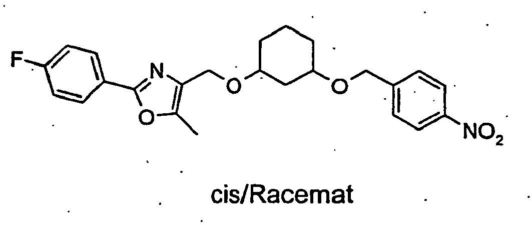 C₂₄H₂₅FN₂O₅ (440.47), MS (ESI): 441

### Beispiel X

### 2-(4-Fluoro-phenyl)-4-[cis-3-(2-methoxy-5-nitro-benzyloxy)-cyclohexyloxymethyl]-5-methyl-oxazol

Analog zu Beispiel VII wurde aus cis- 3-[2-(4-Fluoro-phenyl)-5-methyl-oxazol-4-ylmethoxy]-cyclohexanol und 2-Methoxy-5-nitro-benzylbromid die folgende Verbindung 2-(4-Fluoro-phenyl)-4-[cis-3-(2-methoxy-5-nitro-benzyloxy)-cyclohexyloxymethyl]-5-methyl-oxazol erhalten: 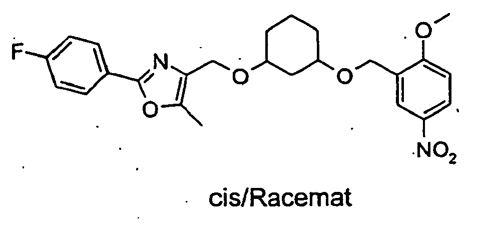 C₂₅H₂₇FN₂O₆ (470.50) MS (ESI): 471 (M + H⁺)

### Beispiel XI

### 3,5-Dinitro-benzoesäure-cis-3-[2-(4-fluoro-phenyl)-5-methyl-oxazol-4-ylmethoxy]-cyclohexylester

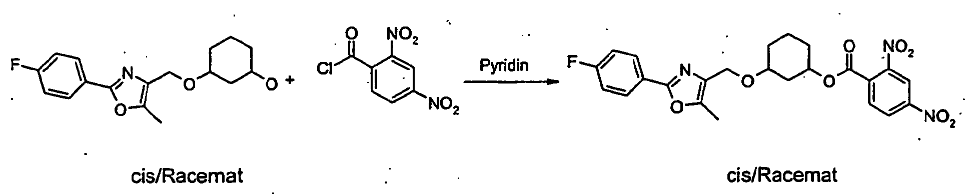

0,5 g 3-[2-(4-Fluoro-phenyl)-5-methyl-oxazol-4-ylmethoxy]-cyclohexanol werden in 3 ml Pyridin gelöst und bei 0 °C mit 0,6 g 3,5-Dinitrobenzoylchlorid versetzt. Das Reaktionsgemisch wird anschließend 10 Minuten auf 50 °C erhitzt. Reaktionskontrolle (LCMS) zeigt die Bildung des gewünschten Produktes neben unumgesetztem Alkohol cis-3-[2-(4-Fluoro-phenyl)-5-methyl-oxazol-4-ylmethoxy]-cyclohexanol. Nach weiteren 10 Minuten Rühren bei Raumtemperatur wird die Reaktion durch Zugabe von 5 ml konzentrierter Salzsäure abgebrochen und das Rohprodukt abgesaugt, mit Essigsäureethylester aufgenommen, mit gesättigter Natriumhydrogencarbonat- und Natriumchlorid - Lösung gewaschen und über Magnesiumsulfat getrocknet. Nach HPLC - Reinigung erhält man 0,15 g der Verbindung 3,5-Dinitro-benzoesäure-cis-3-[2-(4-fluoro-phenyl)-5-methyl-oxazol-4-ylmethoxy]-cyclohexylester als gelben Feststoff.
C₂₄H₂₂FN₃O₈ (499,46), MS (ESI): 500 (M + H⁺)

### Beispiel XII

### 3-Methyl-5-nitro-benzoesäure-cis-3-[2-(4-fluoro-phenyl)-5-methyl-oxazol-4-ylmethoxy]-cyclohexylester

Analog zu **Beispiel XI** wurde aus cis-3-[2-(4-Fluoro-phenyl)-5-methyl-oxazol-4-ylmethoxy]-cyclohexanol und 3-Methyl-5-nitro-benzoylchlorid die folgende Verbindung 3-Methyl-5-nitro-benzoesäure-cis-3-[2-(4-fluoro-phenyl)-5-methyl-oxazol-4-ylmethoxy]-cyclohexylester erhalten: 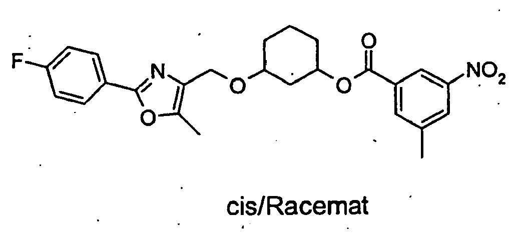 C₂₅H₂₅FN₂O₆ (468.48), MS (ESI): 469 (M + H⁺)

### Beispiel XIII

### 3-Nitro-benzoesäure-cis-3-[2-(4-fluoro-phenyl)-5-methyl-oxazol-4-ylmethoxy]-cyclohexylester

Analog zu Beispiel XI wurde aus cis-3-[2-(4-Fluoro-phenyl)-5-methyl-oxazol-4-ylmethoxy]-cyclohexanol und 3-Nitro-benzoylchlorid die folgende Verbindung 3-Nitrobenzoesäure-cis-3-[2-(4-fluoro-phenyl)-5-methyl-oxazol-4-ylmethoxy]-cyclohexylester erhalten: 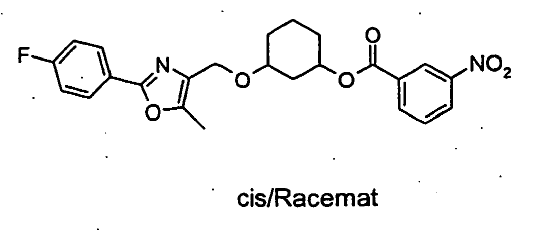 C₂₄H₂₃FN₂O₆, (454.46), MS (ESI): 455 (M + H⁺)

### Beispiel XIV

### 2-Nitro-benzoesäure-cis-3-[2-(4-fluoro-phenyl)-5-methyl-oxazol-4-ylmethoxy]-cyclohexylester

Analog zu Beispiel XI wurde aus cis-3-[2-(4-Fluoro-phenyl)-5-methyl-oxazol-4-ylmethoxy]-cyclohexanol und 2-Nitro-benzoylchlorid die folgende Verbindung 2-Nitrobenzoesäure-cis-3-[2-(4-fluoro-phenyl)-5-methyl-oxazol-4-ylmethoxy]-cyclohexylester erhalten: 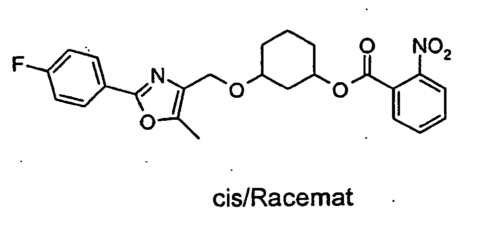 C₂₄H₂₃FN₂O₆ (454.46) MS (ESI): 455 (M + H⁺)

### Beispiel XV

### 3-Cyano-benzoesäure-cis-3-[2-(4-fluoro-phenyl)-5-methyl-oxazol-4-ylmethoxy]-cyclohexylester

Analog zu Beispiel XI wurde aus cis-3-[2-(4-Fluoro-phenyl)-5-methyl-oxazol-4-ylmethoxy]-cyclohexanol und 3-Cyano-benzoylchlorid die folgende Verbindung 3-Cyano-benzoesäure-cis-3-[2-(4-fluoro-phenyl)-5-methyl-oxazol-4-ylmethoxy]-cyclohexylester erhalten: 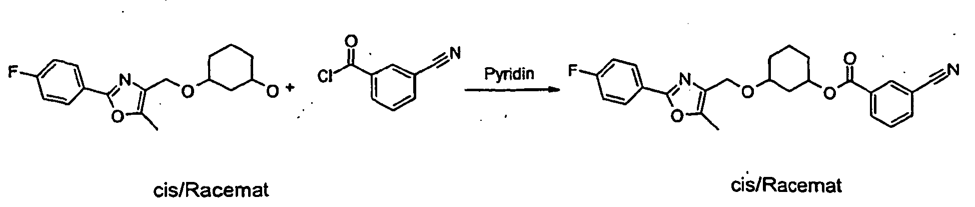 0,1 g 3-[2-(4-Fluoro-phenyl)-5-methyl-oxazol-4-ylmethoxy]-cyclohexanol werden in 3 ml Pyridin gelöst und bei 0°C mit 0,1 g 3-Cyano-benzoylchlorid versetzt. Das Reaktionsgemisch wird anschließend 10 Minuten auf 50 °C erhitzt. Reaktionskontrolle (LCMS) zeigt die Bildung des gewünschten Produktes neben unumgesetztem Alkohol cis-3-[2-(4-Fluoro-phenyl)-5-methyl-oxazol-4-ylmethoxy]-cyclohexanol. Nach Zugabe von weiteren 0,9 g 3-Cyanobenzoylchlorid und 30 Minuten Rühren bei Raumtemperatur wird die Reaktion durch Zugabe von 5 ml konzentrierter Salzsäure abgebrochen, mit Essigsäureethylester extrahiert, die organische Phase mit gesättigter Natriumhydrogencarbonat- und Natriumchlorid - Lösung gewaschen und über Magnesiumsulfat getrocknet. Nach HPLC - Reinigung erhält man 0,1 g der Verbindung 3-Cyano-benzoesäure-cis-3-[2-(4-fluoro-phenyl)-5-methyl-oxazol-4-ylmethoxy]-cyclohexylester als braunen Feststoff. C₂₅H₂₃FN₂O₄ (434,47), MS (ESI): 435 (M + H⁺)

### Beispiel XVI

### 4-Cyano-benzoesäure-cis-3-[2-(4-fluoro-phenyl)-5-methyl-oxazol-4-ylmethoxy]-cyclohexylester

Analog zu Beispiel XI wurde aus cis-3-[2-(4-Fluoro-phenyl)-5-methyl-oxazol-4-ylmethoxy]-cyclohexanol und 4-Cyano-benzoylchlorid die folgende Verbindung 4-Cyano-benzoesäure-cis-3-[2-(4-fluoro-phenyl)-5-methyl-oxazol-4-ylmethoxy]-cyclohexylester erhalten: 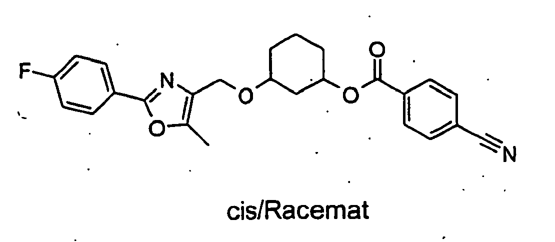 C₂₅H₂₃FN₂O₄ (434.47), MS(ESI): 435 (M + H⁺)

Die so synthetisierten Verbindungen (Beispiele XV - XVI) können durch Reaktion mit Tributylzinnazid in die entsprechenden Tetrazole umgewandelt werden

### Beispiel XVII

### 3-(1H-Tetrazol-5-yl)-benzoesäure-cis-3-[2-(4-fluoro-phenyl)-5-methyl-oxazol-4-ylmethoxy]-cyclohexyl-ester

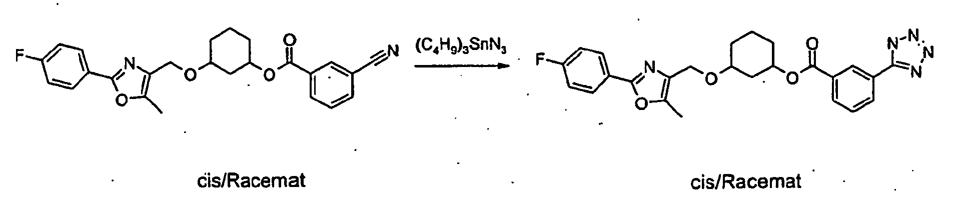

0,06 g des 3-Cyano-benzoesäure-cis-3-[2-(4-fluoro-phenyl)-5-methyl-oxazol-4-ylmethoxy]-cyclohexylester werden in 5 ml Xylol gelöst, mit 150 µm Tributylzinnazid versetzt und 24 Stunden bei 160 °C refluxiert. Die Reaktion wird durch Zugabe von 1 ml Trifluoressigsäure (in 1 ml Methanol) abgebrochen, mit 3 ml Wasser versetzt und mit Ethylacetat (2 x 10 ml) extrahiert. Die vereinigten organischen Phasen werden mit gesättigter Natriumclorid - Lösung gewaschen, über Magnesiumsulfat getrocknet und das Lösungsmittel anschließend im Vakuum entfernt. Durch Reinigung über präparative HPLC erhält man 0,04 g des gewünschten 3-(1 H-Tetrazol-5-yl)-benzoesäure-cis-3-[2-(4-fluoro-phenyl)-5-methyl-oxazol-4-ylmethoxy]-cyclohexyl-esters als amorphen Feststoff.
C₂₅H₂₄FN₅O₄ (477.49), MS(ESI): 478 (M + H⁺)

### Beispiel XVIII

### 4-Cyano-benzoesäure-cis-3-[2-(4-fluoro-phenyl)-5-methyl-oxazol-4-ylmethoxy]-cyclohexylester

Analog zu Beispiel XVII wurde aus 4-Cyano-benzoesäure-cis-3-[2-(4-fluoro-phenyl)-5-methyl-oxazol-4-ylmethoxy]-cyclohexylester und Tributylzinnazrid 4-Cyano-benzoesäure-cis-3-[2-(4-fluoro-phenyl)-5-methyl-oxazol-4-ylmethoxy]-cyclohexylester erhalten: 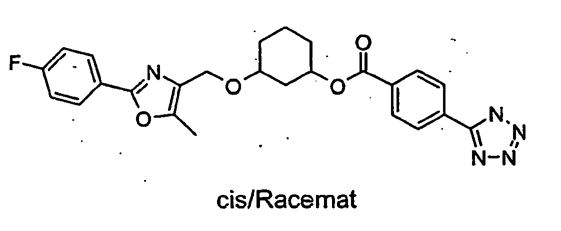 C₂₅H₂₄FN₅O₄ (477.49), MS(ESI): 478 (M + H⁺) .

### Beispiel XIX

### 4-Methyl-3-nitro-benzolsulfonsäure-cis-3-[2-(4-fluoro-phenyl)-5-methyl-oxazol-4-ylmethoxy]-cyclohexyl-ester

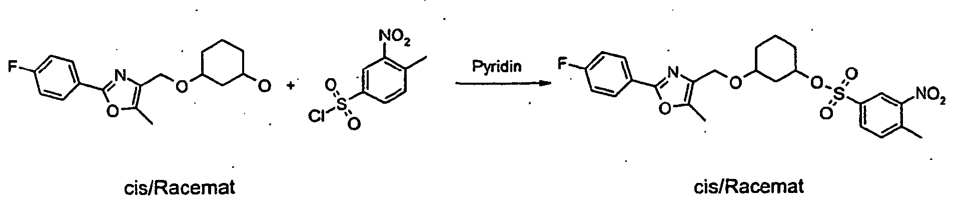

0,1 g cis-3-[2-(4-Fluoro-phenyl)-5-methyl-oxazol-4-ylmethoxy]-cyclohexanol und 0,15 g 4-Methyl-3-nitrobenzol-sulfonsäurechlorid werden in 10 ml trockenem Chloroform gelöst und bei 0 °C mit 2 ml Pyridin versetzt. Das Reaktionsgemisch wird anschließend 12 Stunden bei Raumtemperatur gerührt. Reaktionskontrolle (LCMS) zeigt die Bildung des gewünschten Produktes neben unumgesetztem Alkohol cis-3-[2-(4-Fluoro-phenyl)-5-methyl-oxazol-4-ylmethoxy]-cyclohexanol. Nach weiteren 10 Minuten Rühren bei Raumtemperatur wird die Reaktion durch Zugabe von 2 ml konzentrierter Salzsäure abgebrochen, mit Dichlormethan extrahiert und mit gesättigter Natriumhydrogencarbonat- sowie Natriumchlorid - Lösung gewaschen und über Magnesiumsulfat getrocknet. Nach HPLC - Reinigung erhält man 0,14 g der Verbindung 4-Methyl-3-nitro-benzenesulfonsäure-cis-3-[2-(4-fluoro-phenyl)-5-methyl-oxazol-4-ylmethoxy]-cyclohexyl-ester als zähes Öl.
C₂₄H₂₅FN₂O₇S (504,53), MS (ESI): 505 (M + H⁺)

### Beispiel XX

### 2-Chloro-5-nitro-benzolsulfonsäure-cis-3-[2-(4-fluoro-phenyl)-5-methyl-oxazol-4-ylmethoxy]-cyclohexyl-ester

Analog zu Beispiel XIX wurde aus cis-3-[2-(4-Fluoro-phenyl)-5-methyl-oxazol-4-ylmethoxy]-cyclohexanol und 2-Chloro-5-nitro-benzolsulfonsäure die Verbindung 2-Chloro-5-nitro-benzolsulfonsäure-cis-3-[2-(4-fluoro-phenyl)-5-methyl-oxazol-4-ylmethoxy]-cyclohexylester erhalten: 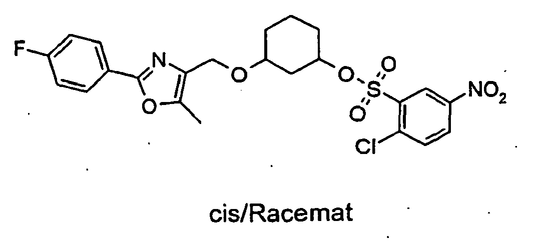 C₂₃H₂₂FClN₂O₇S(524.95), MS (ESI): 525 (M + H⁺)

### Beispiel XXI

### 4-Methoxy-2-nitro-benzolsulfonsäure-cis-3-[2-(4-fluoro-phenyl)-5-methyl-oxazol-4-ylmethoxy]-cyclohexyl-ester

Analog zu Beispiel XIX wurde aus cis-3-[2-(4-Fluoro-phenyl)-5-methyl-oxazol-4-ylmethoxy]-cyclohexanol und 4-Methoxy-2-nitro-benzolsulfonsäure 4-Methoxy-2-nitrobenzolsulfonsäure-cis-3-[2-(4-fluoro-phenyl)-5-methyl-oxazol-4-ylmethoxy]-cyclohexylester erhalten: 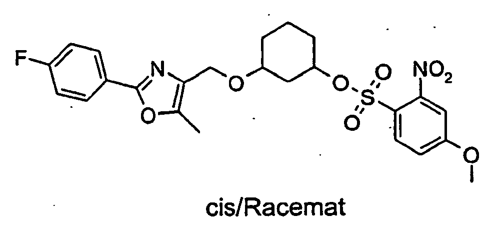 C₂₄H₂₅FN₂O₈S (520.53), MS (ESI): 521 (M + H⁺)

### Beispiel XXII

### 5-(2-{cis-3-[2-(4-Fluoro-phenyl)-5-methyl-oxazol-4-ylmethoxy]-cyclohexyloxy}-ethyliden)-thiazolidin-2,4-dion

### 4-(cis-3-Allyloxy-cyclohexyloxymethyl)-2-(4-fluoro-phenyl)-5-methyl-oxazol

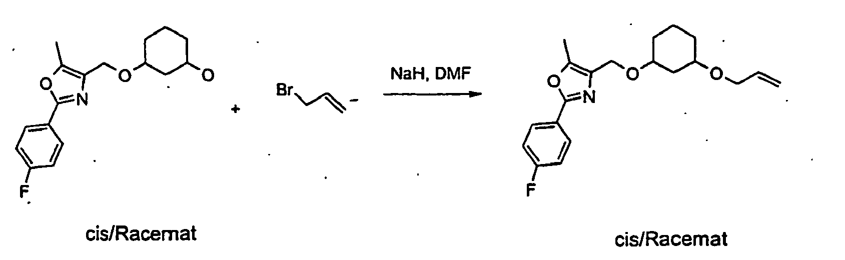

2 g cis-3-[2-(4-Fluoro-phenyl)-5-methyl-oxazol-4-ylmethoxy]-cyclohexanol werden in 15 ml Dimethylformamid gelöst und mit 0.3 g Natriumhydrid versetzt. Nach 30 Minuten werden 2.4 g Allylbromid zugetropft. Man rührt 5 Stunden bei Raumtemperatur nach. Dann wird 15 ml 1N Salzsäure zum Reaktionsgemisch gegeben und dreimal mit 15 ml Ethylacetat gewaschen. Die organische Phase wird über Magnesiumsulfat getrocknet und anschließend das Lösungsmittel im Vakuum entfernt. Der Rückstand wird mittels RP-HPLC gereinigt. Man erhält 2.4 g 4-(cis-3-Allyloxy-cyclohexyloxymethyl)-2-(4-fluoro-phenyl)-5-methyl-oxazol als gelbliches Öl. C₂₀H₂₄FNO₃ (345,42) MS(ESI): 346 (M+H⁺)

### [cis-3-[2-(4-Fluoro-phenyl)-5-methyl-oxazol-4-ylmethoxy]-cyclohexyl]-acetaldehyd

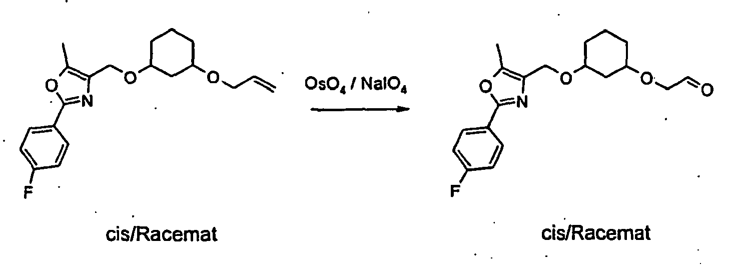

2.0 g 4-(cis-3-Allyloxy-cyclohexyloxymethyl)-2-(4-fluoro-phenyl)-5-methyl-oxazol werden in 50 ml Diethylether gelöst und mit 3.8 g Natriumperiodat, gelöst in 50 ml Wasser versetzt. Man gibt bei 0 °C 1 ml einer Osmiumtetroxid-Lösung (2.5 Gewichts% in tert-Butanol) hinzu und rührt kräftig bei Raumtemperatur nach. Nach 8 Stunden werden 100 ml Methyl-tert-butylether zugegeben und mit einer gesättigten Natriumthiosulfat-Lösung gewaschen. Die organische Phase wird über Magnesiumsulfat getrocknet und das Lösungsmittel im Vakuum entfernt. Der Rückstand wird an Kieselgel (n-Heptan:Ethylacetat = 1:1 → 1:5) gereinigt. Man erhält 1.4 g [cis-3-[2-(4-Fluoro-phenyl)-5-methyl-oxazol-4-ylmethoxy]-cyclohexyl]-acetaldehyds als farbloses Öl. C₂₀H₂₅NO₄ (343.42), MS(ESI): 344 (M+H⁺), R_{f}(n-Heptan:Ethylacetat = 1:1) = 0.25.

### 5-(2-{cis-3-[2-(4-Fluoro-phenyl)-5-methyl-oxazol-4-ylmethoxy]-cyclohexyloxy}-ethyliden)-thiazolidin-2,4-dion

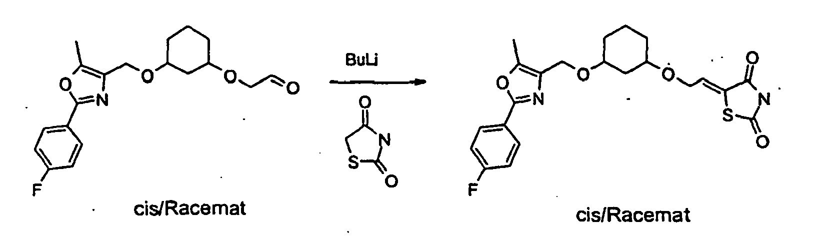

66 mg Thiazolidindion werden in 10 ml Tetrahydrofuran gelöst und bei -78 °C mit 0,11 ml einer 2.7 M Lösung von n-Butyllithium in n-Hexan versetzt. Man rührt 30 Minuten bei -78°C nach und fügt dann 150 mg [cis-3-[2-(4-Fluoro-phenyl)-5-methyl-oxazol-4-ylmethoxy]-cyclohexyl]-acetaldehyd, gelöst in 5 ml Tetrahydrofuran, hinzu. Nach 30 Minuten Rühren bei -78°C lässt man auf Raumtemperatur erwärmen. Es werden 5 ml 1 N Salzsäure zugefügt und dreimal mit je 20 ml Ethylacetat extrahiert. Die vereinigten organischen Phasen werden über Magnesiumsulfat getrocknet und das Lösungsmittel anschließend im Vakuum entfernt. Der Rückstand wird mittels RP-HPLC gereinigt. Man erhält 184 mg 5-(2-{cis-3-[2-(4-Fluoro-phenyl)-5-methyl-oxazol-4-ylmethoxy]-cyclohexyloxy}-ethyliden)- thiazolidin-2,4-dion als weißen Feststoff. C₂₂H₂₃FN₂O₅S (446.01), MS(ESI): 447 (M+H⁺).

### Beispiel XXIII

### 5-[2-[3-(5-Methyl-2-tolyl-oxazol-4-ylmethoxy)-cyclohexyloxy]-ethyliden]-thiazolidin-2,4-dion

Analog zu Beispiel XXII wurde aus [cis-3-(5-Methyl-2-m-tolyl-oxazol-4-ylmethoxy)-cyclohexyl]-acetaldehyd und Thiazolidindion die Verbindung 5-[2-[cis-3-(5-Methyl-2-tolyl-oxazol-4-ylmethoxy)-cyclohexyloxy]-ethyliden]-thiazolidin-2,4-dion erhalten: 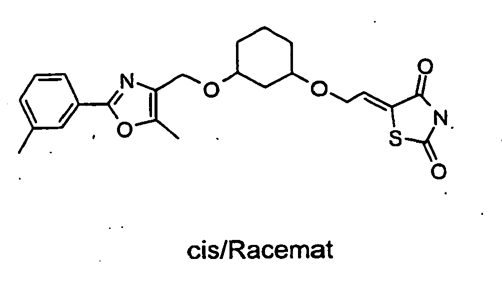 C₂₃H₂₆N₂O₅S (442,53), MS(ESI): 443 (M+H⁺)

### Beispiel XXIV

### 5-[2-{cis-3-[2-(4-Fluoro-phenyl)-5-methyl-oxazol-4-ylmethoxy]-cyclohexyloxy}-ethyliden]-imidazolidin-2,4-dion

Analog zu Beispiel XXII wurde aus [3-[2-(4-Fluoro-phenyl)-5-methyl-oxazol-4-ylmethoxy]-cyclohexyl]-acetaldehyd und Hydantoin die Verbindung 5-[2-{cis-3-[2-(4-Fluoro-phenyl)-5-methyl-oxazol-4-ylmethoxy]-cyclohexyloxy}-ethyliden]-imidazolidin-2,4-dion erhalten: 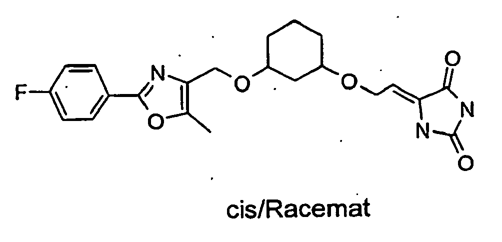 C₂₂H₂₄FN₃O₅ (429,45), MS(ESI): 430 (M+H⁺)

### Beispiel XXV

### 5-[2-{cis-3-[2-(4-Fluoro-phenyl)-5-methyl-oxazol-4-ylmethoxy]-cyclohexyloxy}-ethyliden]-2-thioxo-imidazolidin-4-on

Analog zu Beispiel XXII wurde aus [cis-3-[2-(4-Fluoro-phenyl)-5-methyl-oxazol-4-ylmethoxy]-cyclohexyl]-acetaldehyd und 2-Thioxo-imidazolidin-4-on -5-[2-{cis-3-[2-(4-Fluoro-phenyl)-5-methyl-oxazol-4-ylmethoxy]-cyclohexyloxy}-ethyliden]-2-thioxo-imidazolidin-4-on erhalten: 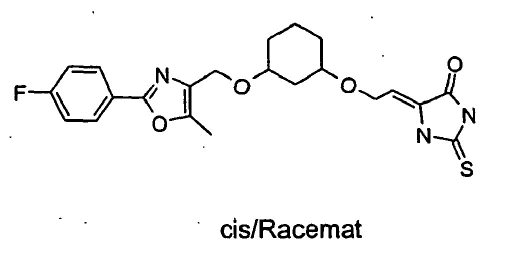 C₂₂H₂₄FN₃O₄S (445,59), MS(ESI): 446 (M+H⁺)

### Beispiel XXVI

### 5-(2-{cis-3-[2-(4-Fluoro-phenyl)-5-methyl-oxazol-4-ylmethoxy]-cyclohexyloxy}-ethyl)-thiazolidin-2,4-dion

Durch Hydrierung der in Beispiel Beispiel XXII genannten Verbindung 5-(2-{cis-3-[2-(4-Fluoro-phenyl)-5-methyl-oxazol-4-ylmethoxy]-cyclohexyloxy}-ethyliden)-thiazolidin-2,4-dion mit Wasserstoff wird die Verbindung 5-(2-{cis-3-[2-(4-Fluoro-phenyl)-5-methyl-oxazol-4-ylmethoxy]-cyclohexyloxy}-ethyl)-thiazolidin-2,4-dion erhalten: 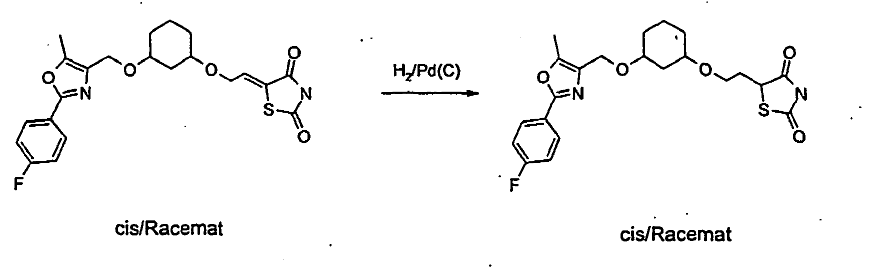 180 mg des ungesättigten 5-(2-{cis-3-[2-(4-Fluoro-phenyl)-5-methyl-oxazol-4-ylmethoxy]-cyclohexyloxy}-ethyliden)-thiazolidin-2,4-dions wird in 10 ml Ethylacetat gelöst und mit 20 mg Palladium auf Kohle versetzt. Anschließend wird bei 2 bar Wasserstoffdruck 2 Stunden bei Raumtemperatur gerührt. Es wird vom Katalysator abfiltriert, das Lösungsmittel im Vakuum entfernt und der Rückstand über RP-HPLC gereinigt. Man erhält 140 mg der Verbindung 5-(2-{cis-3-[2-(4-Fluoro-phenyl)-5-methyl-oxazol-4-ylmethoxy]-cyclohexyloxy}-ethyl)-thiazolidin-2,4-dion als gelblichen Feststoff. C₂₂H₂₅FN₂O₅S (448.01), MS(ESI): 449 (M+H⁺).

### Beispiel XXVII

### 5-{2-[(cis-3-(5-Methyl-2-tolyl-oxazol-4-ylmethoxy)-cyclohexyloxy]-ethyl}-thiazolidin-2,4-dion

Wie in Beispiel XXVI wird durch Hydrierung der in Beispiel Beispiel XXIII genannten Verbindung 5-[2-[cis-3-(5-Methyl-2-tolyl-oxazol-4-ylmethoxy)-cyclohexyloxy]-ethyliden]-thiazolidin-2,4-dion mit Wasserstoff die Verbindung 5-{2-[(cis-3-(5-Methyl-2-tolyl-oxazol-4-ylmethoxy)-cyclohexyloxy]-ethyl}-thiazolidin-2,4-dion erhalten: 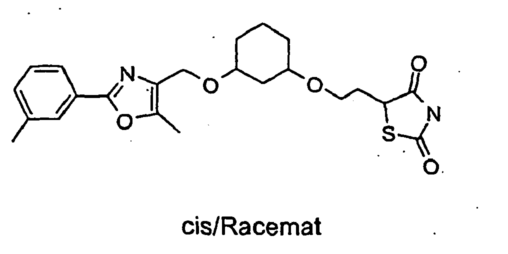 C₂₃H₂₈N₂O₅S (444,55), MS(ESI): 445 (M+H⁺)

### Beispiel XXVIII

### 5-{2-[cis-3-(5-Methyl-2-m-tolyl-oxazol-4-ylmethoxy)-cyclohexyl]-ethyl}-thiazolidin-2,4-dion

### 4-(cis-3-Allyl-cyclohexyloxymethyl)-2-(3-methyl-phenyl)-5-methyl-oxazol

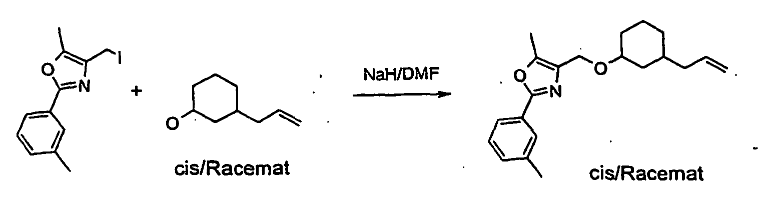

2 g cis-3-Allyl-cyclohexanol werden in 30 ml Dimethylformamid gelöst und mit 750 mg Natriumhydrid (60%ige Suspension in Paraffinöl) versetzt. Nach 30 Minuten werden 6.7 g 4-Iodomethyl-5-methyl-2-(3-methyl-phenyl)-oxazol gelöst in 20 ml Dimethylformamid zugetropft. Man rührt 1 Stunde bei Raumtemperatur nach. Dann werden 200 ml Methy-tert-buthylether zum Reaktionsgemisch gegeben und dreimal mit Wasser gewaschen. Die organische Phase wird über Magnesiumsulfat getrocknet und anschließend das Lösungsmittel im Vakuum entfernt. Der Rückstand wird mittels RP-HPLC gereinigt. Man erhält 1.56 g 4-(cis-3-Allyl-cyclohexyloxymethyl)-2-(3-methylphenyl)-5-methyl-oxazol als Öl. C₂₁H₂₇NO₂ (325.45), MS(ESI): 326 (M+H⁺), R_{f}(n-Heptan:Ethylacetat = 2:1) =0.28.

### {cis-3-[2-(3-Methyl-phenyl)-5-methyl-oxazol-4-ylmethoxy]-cyclohexy}-acetaldehyd

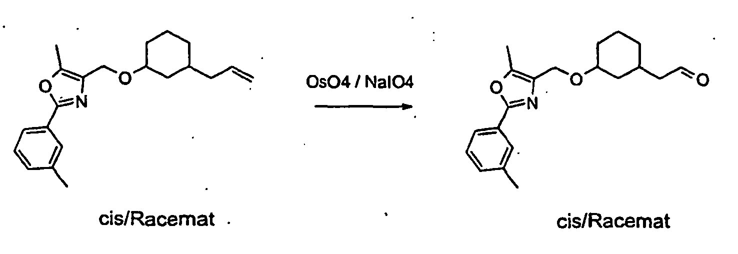

940 mg 4-(cis-3-Allyl-cyclohexyloxymethyl)-2-(3-methyl-phenyl)-5-methyl-oxazol werden in 50 ml Diethylether gelöst und mit 1.86 g Natriumperiodat, gelöst in 50 ml Wasser versetzt. Man gibt bei 0 °C 3 ml einer Osmiumtetroxid-Lösung (2.5 Gewichts% in tert-Butanol) hinzu und rührt kräftig bei Raumtemperatur nach. Nach 8 Stunden werden 100 ml Methyl-tert-butyl-ether zugegeben und mit einer gesättigten Natriumthiosulfat-Lösung gewaschen. Die organische Phase wird über Magnesiumsulfat getrocknet und anschließend das Lösungsmittel im Vakuum entfernt. Der Rückstand wird an Kieselgel mit dem Laufmittel n-Heptan:Ethylacetat = 4:1 gereinigt. Man erhält 270 mg {cis-3-[2-(3-Methyl-phenyl)-5-methyl-oxazol-4-ylmethoxy]-cyclohexyl}-acetaldehyd als gelbbraunes Öl. C₂₀H₂₅NO₃ (327.43), MS(ESI): 328 (M+H⁺), R_{f}(n-Heptan:Ethylacetat 2:1) = 0.07.

### 5-{1-Hydroxy-2-[cis-3-(5-methyl-2-m-tolyl-oxazol-4-ylmethoxy)-cyclohexyl]-ethyl}-thiazolidine-2,4-dion

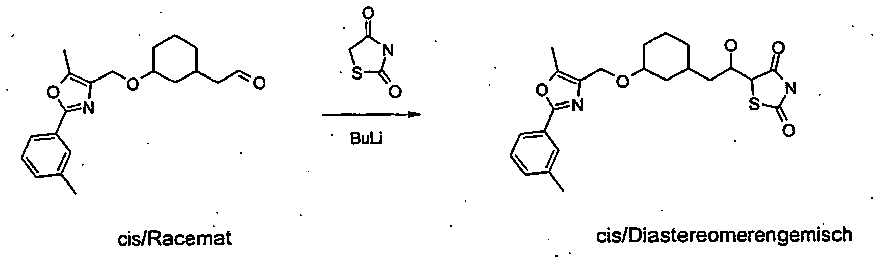

214 mg Thiazolidindion werden in 20 ml Tetrahydrofuran gelöst und bei -78°C mit 1.4 ml einer 2.7 M Lösung von n-Butyllithium in n-Hexan versetzt. Man rührt 30 Minuten bei -78°C nach und fügt dann 500 mg {cis-3-[2-(3-Methyl-phenyl)-5-methyl-oxazol-4-ylmethoxy]-cyclohexyl}-acetaldehyd, gelöst in 10 ml Tetrahydrofuran hinzu. Nach 30 minütigem Rühren bei -78°C lässt man auf Raumtemperatur erwärmen. Es werden 20 ml 1N Salzsäure zugefügt und dreimal mit je 50 ml Ethylacetat extrahiert. Die vereinigten organischen Phasen werden über Magnesiumsulfat getrocknet und anschließend das Lösungsmittel im Vakuum entfernt. Der Rückstand wird mittels RP-HPLC gereinigt. Man erhält 420 mg 5-{1-Hydroxy-2-[cis-3-(5-methyl-2-m-tolyl-oxazol-4-ylmethoxy)-cyclohexyl]-ethyl}- thiazolidine-2,4-dion als weißen Feststoff. C₂₃H₂₈N₂O₅S (444.45), MS(ESI): 445 (M+H⁺).

### Beispiel XXIX

### 5-(1-Hydroxy-2-{cis-3-[2-(3-methoxy-phenyl)-5-methyl-oxazol-4-ylmethoxy]-cyclohexyl}-ethyl)- thiazolidin-2,4-dion

Analog zu Beispiel XXVIII erhält man aus {cis-3-[2-(3-Methyl-phenyl)-5-methyl-oxazol-4-ylmethoxy]-cyclohexyl}-acetaldehyd und Thiazolididion die Verbindung 5-(1-Hydroxy-2-{cis-3-[2-(3-methoxy-phenyl)-5-methyl-oxazol-4-ylmethoxy]-cyclohexyl}-ethyl)-thiazolidin-2,4-dion. 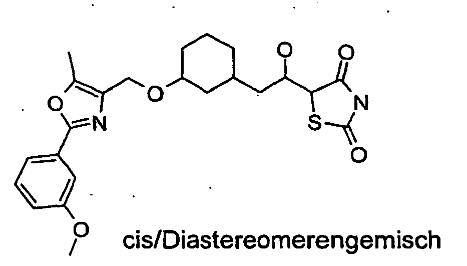 C₂₃H₂₈N₂O₆S (460.55), MS(ESI): 461 (M+H⁺).

### Beispiel XXX

### 5-{1-Hydroxy-2-[cis-3-(5-methyl-2-p-tolyl-oxazol-4-ylmethoxy)-cyclohexyl]-ethyl}-thiazolidin-2,4-dion

Analog zu Beispiel XXVIII wurde aus cis-[3-(5-Methyl-2-p-tolyl-oxazol-4-ylmethoxy)-cyclohexyl]-acetaldehyde und Thiazolididion die Verbindung 5-{1-Hydroxy-2-[cis-3-(5-methyl-2-p-tolyl-oxazol-4-ylmethoxy)-cyclohexyl]-ethyl}-thiazolidin-2,4-dion erhalten. 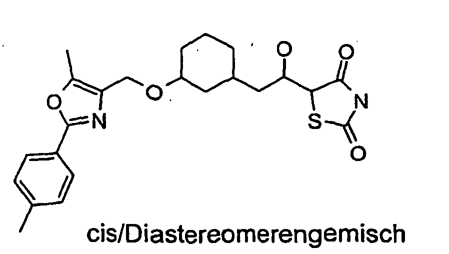 C₂₃H₂₈N₂O₅S (444.55), MS(ESI): 445 (M+H⁺).

### Beispiel XXXI

### 5-{2-[cis-3-(5-Methyl-2-m-tolyl-oxazol-4-ylmethoxy)-cyclohexyl]-ethyl}-thiazolidin-2,4-dion

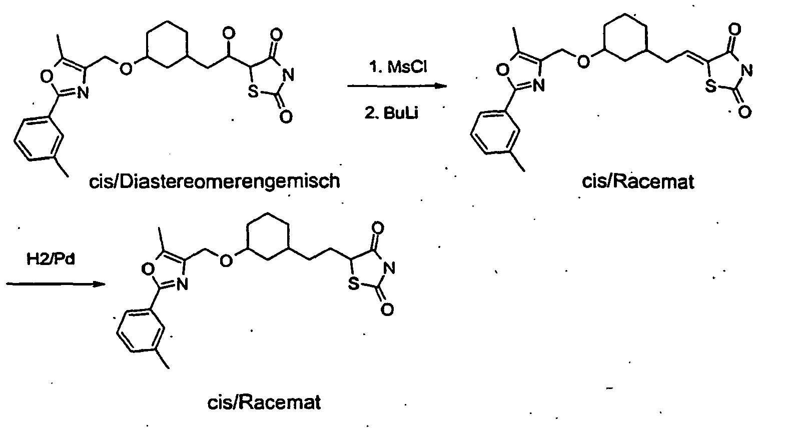

### 5-{2-[cis-3-(5-Methyl-2-m-tolyl-oxazol-4-ylmethoxy)-cyclohexyl]-ethylidene}-thiazolidin-2,4-dion

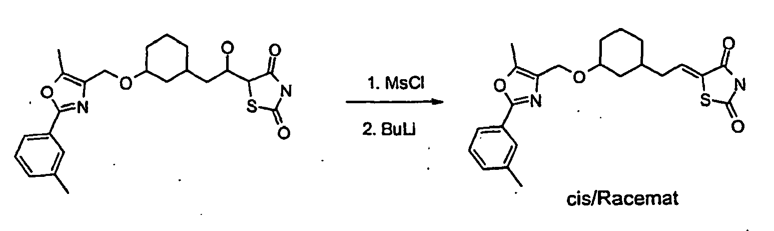

344 mg 5-{1-Hydroxy-2-[cis-3-(5-methyl-2-m-tolyl-oxazol-4-ylmethoxy)-cyclohexyl]-ethyl}- thiazolidin-2,4-dion werden in 20 ml Dichlormethan gelöst und mit 0.13 ml Triethylamin und und 0.12 ml Mesylchlorid versetzt. Nach zweistündigem Rühren bei Raumtemperatur werden nochmal 0.13 ml Triethylamin und und 0.12 ml Mesylchlorid nachgegeben. Es wird 12 Stunden bei Raumtemperatur gerührt. Es werden 100 ml Dichlormethan zugegeben und das Gemisch mit gesättigter Natriumhydrogencarbonatlösung gewaschen, über Magnesiumsulfat getrocknet und anschließend das Lösungsmittel im Vakuum entfernt. Der resultierende Rückstandwird in 10 ml Tetrahydrofuran gelöst und bei -78 °C mit 0.22 ml einer 2.7 M Lösung von n-Butyllithium in n-Hexan versetzt. Man rührt bei 0°C 30 Minuten nach, dann wird 20 ml 1N Salzsäure zugefügt und dreimal mit je 50 ml Ethylacetat extrahiert. Die vereinigten organischen Phasen werden über Magnesiumsulfat getrocknet und anschließend das Lösungsmittel im Vakuum entfernt. Der Rückstand wird mittels RP-HPLC gereinigt. Man erhält 81 mg 5-{2-[Cis-3-(5-Methyl-2-m-tolyl-oxazol-4-ylmethoxy)-cyclohexyl]-ethylidene}-thiazolidin-2,4-dion als weißen Feststoff. C₂₃H₂₆N₂O₄S (426.54), MS(ESI): 427 (M+H⁺).

### 5-{2-[cis-3-(5-Methyl-2-m-tolyl-oxazol-4-ylmethoxy)-cyclohexyl]-ethyl}-thiazolidin-2,4-dion

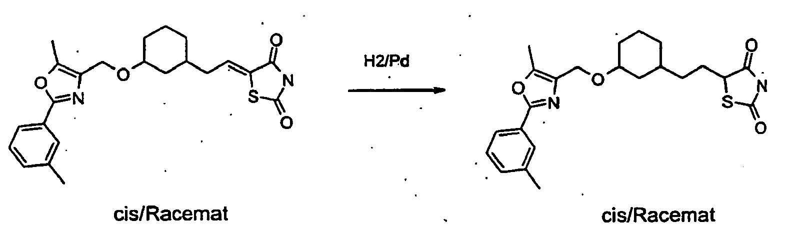

81 mg 5-{2-[cis-3-(5-Methyl-2-m-tolyl-oxazol-4-ylmethoxy)-cyclohexyl]-ethylidene}-thiazolidin-2,4-dion werden in 10 ml Ethylacetat gelöst und mit 10 mg Paladium (10% auf Aktivkohle) versetzt. Es wird 9 Stunden unter einer Wasserstoffatmosphäre (5 bar) gerührt. Anschließend wird der Katalysator über Celite abfiltriert und das Filtrat im Vakuum eingeengt. Der Rückstand wird mittels RP-HPLC gereinigt. Man erhält 60 mg 5-{2-[cis-3-(5-Methyl-2-m-tolyl-oxazol-4-ylmethoxy)-cyclohexyl]-ethyl}-thiazolidin-2,4-dion als Lyophilisat. C₂₃H₂₈N₂O₄S (428.55), MS(ESI): 429 (M+H⁺).

### Beispiel XXXII

### 5-(2-{cis-3-[2-(3-Methoxy-phenyl)-5-methyl-oxazol-4-ylmethoxy]-cyclohexyl}-ethyl)-thiazolidin-2,4-dion

Analog zu Beispiel XXXI wurde aus 5-(1-Hydroxy-2-{cis-3-[2-(3-methoxy-pheny)-5-methyl-oxazol-4-ylmethoxy]-cyclohexyl}-ethyl)-thiazolidin-2,4-dion die Verbindung 5-(2-{cis-3-[2-(3-Methoxy-phenyl)-5-methyl-oxazol-4-ylmethoxy]-cyclohexyl}-ethyl)-thiazolidin-2,4-dion erhalten. 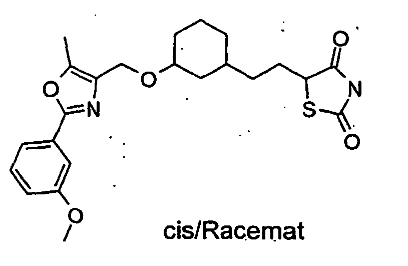 C₂₃H₂₈N₂O₅S (444.55), MS(ESI): 445 (M+H⁺).

### Beispiel XXXIII

### 5-{2-[cis-3-(5-Methyl-2-p-tolyl-oxazol-4-ylmethoxy)-cyclohexyl]-ethyl}-thiazolidin-2,4-dion

Analog zu Beispiel XXXI wurde aus 5-{1-Hydroxy-2-[cis-3-(5-methyl-2-p-tolyl-oxazol-4-ylmethoxy)-cyclohexyl]-ethyl}- thiazolidin-2,4-dion die Verbindung 5-{2-[cis-3-(5-Methyl-2-p-tolyl-oxazol-4-ylmethoxy)-cyclohexyl]-ethyl}thiazolidin-2,4-dion erhalten. 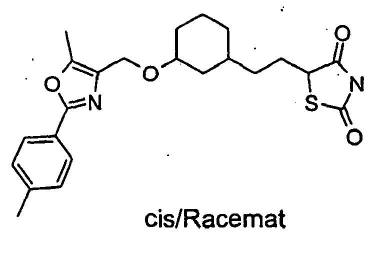 C₂₃H₂₈N₂O₄S (428.55), MS(ESI): 429 (M+H⁺).

### Beispiel XXXIV

### 5-[1-[cis-3-(5-Methyl-2-tolyl-oxazol-4-ylmethoxy)-cyclohexyl]-methyliden]-thiazolidin-2,4-dion

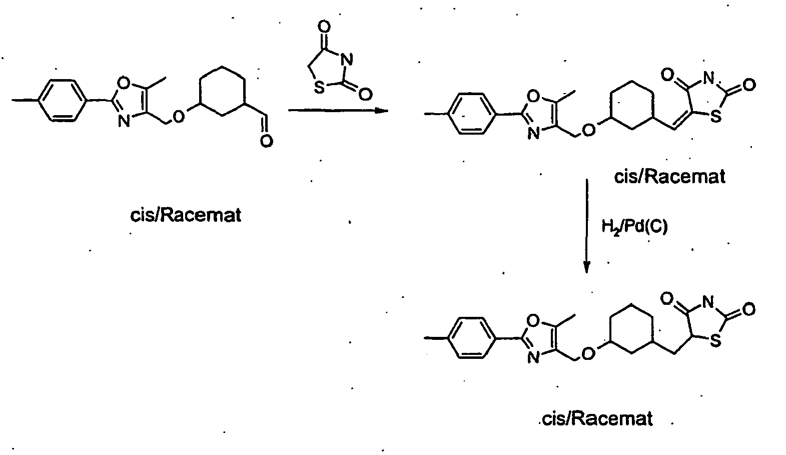

cis-3-Hydroxymethyl-cyclohexanol

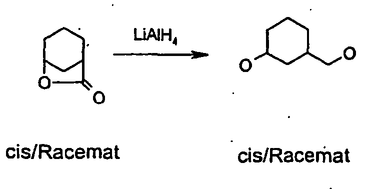

10 g 6-Oxa-bicyclo[3.2.1]octan-7-on werden in 300 ml Tetrahydrofuran gelöst und unter Eiskühlung mit 160 ml einer 1M Lösung von Lithiumaluminiumhydrid in Tetrahydrofuran versetzt. Nach 30 Minuten Rühren bei Raumtemperatur wird gesättigte Ammoniumchloridlösung zugesetzt und durch Zugabe einer 5%igen Zitronensäurelösung ein neutraler pH-Wert eingestellt. Das Tetrahydrofuran wird im Vakuum entfernt und der Rückstand dreimal mit je 150 ml Ethylacetat extrahiert. Die vereinigten organischen Phasen werden über Magnesiumsulfat getrocknet und anschließend das Lösungsmittel im Vakuum entfernt. Man erhält 10.5 g cis-3-Hydroxymethyl-cyclohexanol als farbloses Öl. C₇H₁₄O₂ (130.13), Rf(Ethylacetat) = 0.14.

### cis-3-(tert-Butyl-diphenyl-silanyloxymethyl)-cyclohexanol

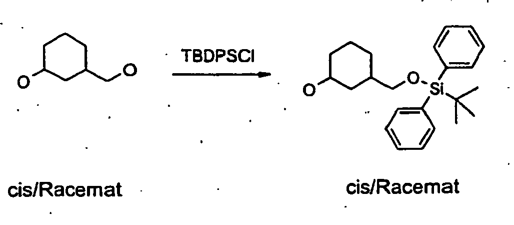

10.5 g cis-3-Hydroxymethyl-cyclohexanol werden in 300 ml Dimethylformamid gelöst und mit 23 ml tert-Butyl-diphenyl-silanylchlorid, 8.0 g Imidazol und 200 mg Dimethylaminopyridin versetzt. Es wird 12 Stunden bei Raumtemperatur gerührt. Das Dimethylformamid wird im Vakuum entfernt, der Rückstand in 300 ml Ethylacetat gelöst und fünfmal mit je 100 ml Wasser gewaschen. Die organische Phase wird über Magnesiumsulfat getrocknet und anschließend das Lösungsmittel im Vakuum entfernt. Man erhält 27.0 g cis-3-(tert-Butyl-diphenyl-silanyloxymethyl)-cyclohexanol als Öl. C₂₃H₃₂O₂Si (368.6), Rf(n-Heptan:Ethylacetat = 1:1) = 0.42.

### cis-3-(tert-Butyl-diphenyl-silanyloxymethyl)-cyclohexyloxymethyl]-5-methyl-2-p-tolyl-oxazol

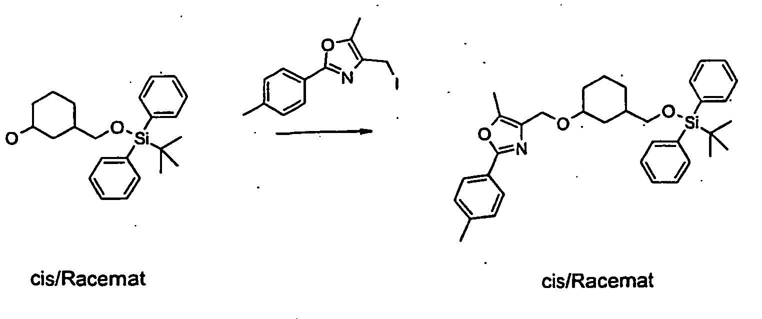

6.4 g cis-3-(tert-Butyl-diphenyl-silanyloxymethyl)-cyclohexanol werden mit 6.5 g 4-lodomethyl-5-methyl-2-p-tolyl-oxazol in 200 ml Dimethylformamid gelöst und mit 1 g Natriumhydrid (60%ige Suspension in Mineralöl) versetzt. Nach 1 Stunde Rühren bei Raumtemperatur werden nochmals 2 g Natriumhydrid und 5 g 4-Iodomethyl-5-methyl-2-p-tolyl-oxazol zugegeben. Nach 4 stündigem Rühren bei Raumtemperatur wird das Reaktionsgemisch durch Zugabe von 400 ml Essigsäureethylester verdünnt und fünfmal mit je 200 ml Wasser gewaschen. Die organische Phase Phase wird über Magnesiumsulfat-getrocknet und anschließend das Lösungsmittel im Vakuum entfernt. Der Rückstand wird an Kieselgel mit dem Eluens n-Heptan:Ethytacetat =10:1 gereinigt. Man erhält 6.8 g 4-cis-3-(tert-Butyl-diphenyl-silanyloxymethyl)-cyclohexyloxymethyl]-5-methyl-2-p-tolyl-oxazol als Öl. C₃₅H₄₃NO₃Si (553.28), Rf(n-Heptan:Ethylacetat = 2:1) = 0.50.

### [(cis-3-(5-Methyl-2-p-tolyl-oxazol-4-ylmethoxy)-cyclohexyl]-methanol

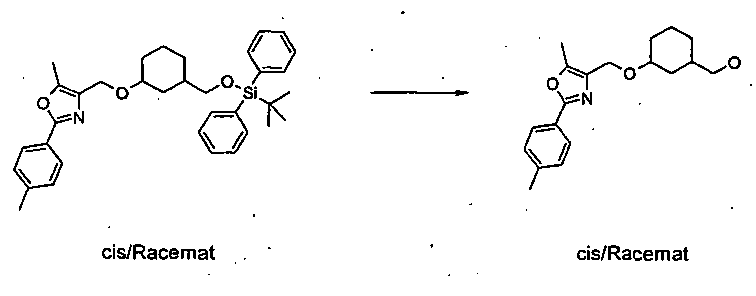

6.8 g 4-cis-3-(tert-Butyl-diphenyl-silanyloxymethyl)-cyclohexyloxymethyl]-5-methyl-2-p-tolyl-oxazol werden in 40 ml Tetrahydrofuran gelöst und mit 40 ml einer 1M Lösung, von Tetrabutylammoniumfluorid versetzt. Es wird 1 Stunde auf 50 °C erwärmt, anschließend das Lösungsmittel im Vakuum entfernt und der resultierende Rückstand an Kieselgel mit dem Eluens n-Heptan:Ethylacetat = 5:1=> 1:1 gereinigt. Man erhält 1.0 g cis-3-(5-Methyl-2-p-tolyl-oxazol-4-ylmethoxy)-cyclohexyl]-methanol als Öl. C19H25NO3 (315.42), Rf(n-Heptan:Ethylacetat = 1:1) = 0.13.

### cis-3-(5-Methyl-2-p-tolyl-oxazol-4-ylmethoxy)cyclohexancarbaldehyd

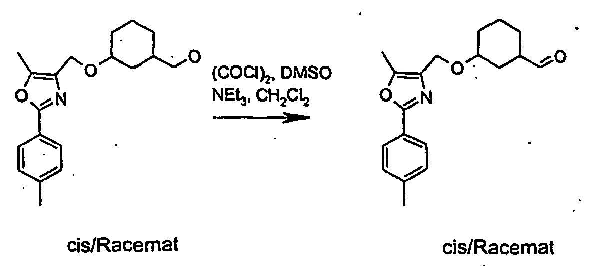

Zu 0.48 ml Oxalylchlorid in 15 ml Dichlormethan werden bei -78 °C 0.89 ml DMSO in 1 ml Dichlormethan so zugetropft, daß die Temperatur -70 °C nicht übersteigt. Nach beendeter Zugabe wird die Lösung 30 Minuten bei dieser Temperatur gerührt. Dann werden 1,5 g cis-3-(5-Methyl-2-p-tolyl-oxazol-4-ylmethoxy)-cyclohexyl]-methanol in 2 ml Dichlormethan so zugetropft, daß die Temperatur unter -78 °C bleibt. Die Lösung wird 30 Minuten bei dieser Temperatur gerührt. Dann werden 3,2 ml Triethylamin zugetropft, das Kühlbad wird entfernt und die Lösung auf 0 °C erwärmt. Bei dieser Temperatur werden 10 ml Wasser zugegeben, und die Mischung wird heftig bei Raumtemperatur gerührt. Die wäßrige Phase wird abgetrennt und mit Dichlormethan extrahiert. Die vereinigten organischen Phasen werden mit gesättigter Ammoniumchlorid-Lösung gewaschen, über Magnesiumsulfat getrocknet und eingeengt, wobei 1,50 g cis-3-(5-Methyl-2-p-tolyl-oxazol-4-ylmethoxy)-cyclohexancarbaldehyd erhalten werden. C₁₉H₂₃NO₃ (313.40); LCMS (ESI): 314 (MH⁺).

### 5-[1-[cis-3-(5-Methyl-2-tolyl-oxazol-4-ylmethoxy)-cyclohexyl]-methyliden]-thiazolidin-2,4-dion

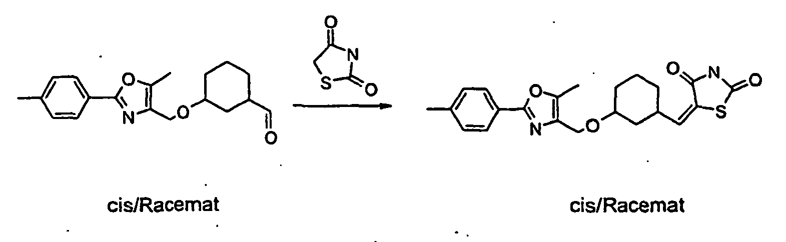

0.25 g Thizolidindion wird in einem ausgeheizten Dreihals-Kolben vorgelegt, in 15 ml Tetrahydrofuran gelöst, auf -78 °C gekühlt und 2.4 ml n-Buthyllithium (1.6 M Lösung in n-Hexan) langsam zugetropft, so dass die Innentemperatur nicht über -65 °C ansteigt. Anschließend wird auf Raumtemperatur erwärmt, wobei sich die Lösung gelb färbt. Nach erneutem Abkühlen auf -70 °C werden 0.4 g cis-3-(5-Methyl-2-p-tolyl-oxazol-4-ylmethoxy)-cyclohexanecarbaldehyd - gelöst in 5 ml Tetrahydrofuran - zugetropft und das Reaktionsgemisch auf Raumtemperatur erwärmt. Dabei bildet sich der tertiäre Alkohol als Additionsprodukt (Reaktionskontrolle (DC und LCMS) M = 430 g/mol). Durch saure Aufarbeitung (5 ml 1 N HCl) und Extraktion mit 2 x 10 ml Ethylacetat wird das gewünschte Eliminierungs-Produkt nach Entfernen des Lösungsmittels im Vakuum erhalten. Durch Aufnehmen des Rohproduktes in Acetonitril und Abfiltrieren von der Mutterlauge werden 0.5 g 5-[1-[cis-3-(5-Methyl-2-tolyl-oxazol-4-ylmethoxy)-cyclohexyl]-methyliden]-thiazolidin-2,4-dion als weißer Feststoff. C₂₂H₂₄N₂O₄S (412,54), MS(ESI): 413 (M+H⁺).

### Beispiel XXXV

### 5-[cis-3-(5-Methyl-2-tolyl-oxazol-4-ylmethoxy)-cyclohexylmethyl]-thiazolidin-2,4-dion

Aus der in Beispiel XXXIV genannten Verbindung 5-[1-[cis-3-(5-Methyl-2-tolyl-oxazol-4-ylmethoxy)-cyclohexyl]-methyliden]-thiazolidin-2,4-dion wird durch Hydrierung die Verbindung 5-[cis-3-(5-Methyl-2-tolyl-oxazol-4-ylmethoxy)-cyclohexylmethyl]-thiazolidin-2,4-dion erhalten.

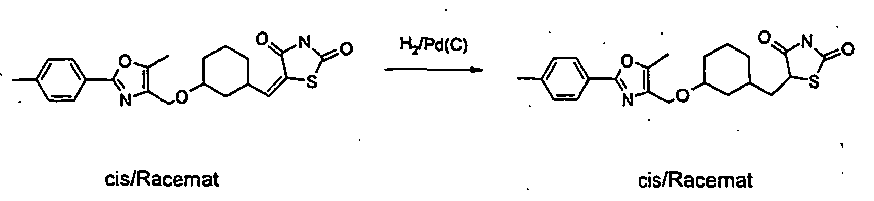

0.35 g 5-[1-[cis-3-(5-Methyl-2-tolyl-oxazol-4-ylmethoxy)-cyclohexyl]-methyliden]-thiazolidin-2,4-dion wird in 12 ml eines Lösungsmitteigemisch aus Ethylacetat und Methanol (3:1) gelöst und mit 20 mg Palladium auf Kohle versetzt. Anschließend wird 2 Stunden bei 3 bar Wasserstoffdruck bei Raumtemperatur hydriert. Nach Filtration vom Katalysator wird das Lösungsmittel im Vakuum entfernt und der Rückstand in Acetonitril aufgenommen. Das Produkt kann abfiltriert werden und man erhält 0.3 g 5-[cis-3-(5-Methyl-2-tolyl-oxazol-4-ylmethoxy)-cyclohexylmethyl]-thiazolidin-2,4-dion als weißen Feststoff. C₂₂H₂₆N₂O₄S (414,52), MS(ESI): 415 (M+H⁺).

### Beispiel XXXVI

### 5-(2-{(cis-3-[5-cyclohexyl-2-(3-methoxy-phenyl)-oxazol-4-ylmethoxy]-cyclohexyl}-ethyl)-thiazolidin- 2,4-dion

Analog zu Beispiel XXVIII und Beispiel XXXI wurde aus cis-3-Allyl-cyclohexanol, Thiazolidindion und 4-Iodomethyl-5-cyclohexyl-2-(3-methoxy -phenyl)-oxazol die Verbindung 5-(2-{(cis-3-[5-cyclohexyl-2-(3-methoxy -phenyl)-oxazol-4-ylmethoxy]-cyclohexyl}-ethyl)- thiazolidin- 2,4-dion erhalten. 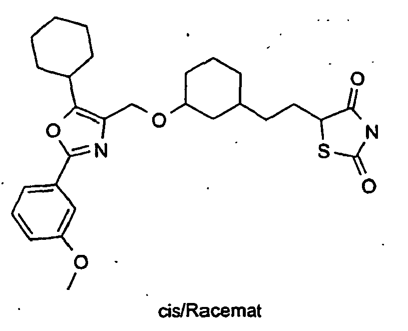 C28H36N2O5S (512.67), MS(ESI): 513 (M+H⁺).

### Beispiel XXXVII

### 5-(2-{(cis-3-[5-cyclohexyl-2-(4-methyl-phenyl)-oxazol-4-ylmethoxy]-cyclohexyl}-ethyl)-thiazolidin- 2,4-dion

Analog zu Beispiel **XXVIII** und Beispiel **XXXI** wurde aus cis-3-Allyl-cyclohexanol, Thiazolidindion und 4-Iodomethyl-5-cyclohexyl-2-(4-methyl -phenyl)-oxazol die Verbindung 5-(2-{(cis-3-[5-cyclohexyl-2-(4-methyl-phenyl)-oxazol-4-ylmethoxy]-cyclohexyl}-ethyl)- thiazolidin- 2,4-dion erhalten. 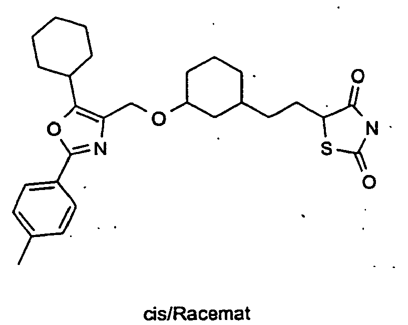 C28H36N2O4S (496.67), MS(ESI): 497 (M+H⁺).

### Beispiel XXXVIII

### 5-(2-{(cis-3-[5-ethyl-2-(3,5-bis-trifluormethyl-phenyl)-oxazol-4-ylmethoxy]-cyclohexyl}-ethyl)- thiazolidin- 2,4-dion

Analog zu Beispiel **XXVIII** und Beispiel **XXXI** wurde aus cis-3-Allyl-cyclohexanol, Thiazolidindion und 4-lodomethyl-5-ethyl-2-(3,5-bis-trifluormethyl -phenyl)-oxazol die Verbindung 5-(2-{(cis-3-[5-ethyl-2-(3,5-bis-trifluormethyl-phenyl)-oxazol-4-ylmethoxy]-cyclohexyl}-ethyl)- thiazolidin- 2,4-dion erhalten. 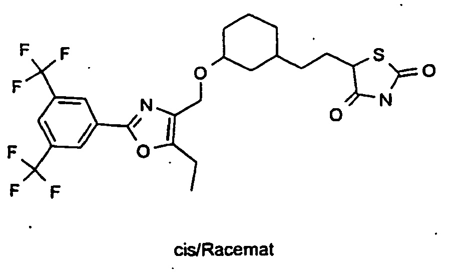 C25H26F6N2O4S (564.55), MS(ESI): 565 (M+H⁺). -

### Beispiel XXXIX

### 5-(2-{(cis-3-[5-ethyl-2-(2,6-dimethyl-phenyl)-oxazol-4-ylmethoxy]-cyclohexyl}-ethyl)-thiazolidin- 2,4-dion

Analog zu Beispiel **XXVIII** und Beispiel **XXXI** wurde aus cis-3-Allyl-cyclohexanol, Thiazolidindion und 4-lodomethyl-5-ethyl-2-(2,6-dimethyl -phenyl)-oxazol die Verbindung 5-(2-{(cis-3-[5-ethyl-2-(2,6-dimethyl -phenyl)-oxazol-4-ylmethoxy]-cyclohexyl}-ethyl)- thiazolidin- 2,4-dion erhalten. 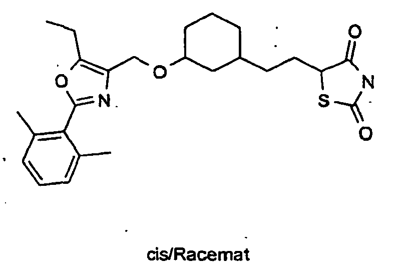 C25H32N2O4S (456.64), MS(ESI): 457 (M+H⁺).

### Beispiel XL

### 5-(2-{(cis-3-[5-methyl-2-(2-trifluormethyl-phenyl)-oxazol-4-ylmethoxy]-cyclohexyl}-ethyl)- thiazolidin- 2,4-dion

Analog zu Beispiel **XXVIII** und Beispiel **XXXI** wurde aus cis-3-Allyl-cyclohexanol, Thiazolidindion und 4-Iodomethyl-5-methyl-2-(2-trifluormethyl -phenyl)-oxazol die Verbindung 5-(2-{(cis-3-[5-methyl-2-(2-trifluormethyl-phenyl)-oxazol-4-ylmethoxy]-cyclohexyl}-ethyl)- thiazolidin- 2,4-dion erhalten. 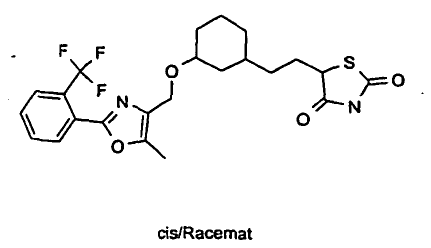 C23H25F3N2O4S (482.53), MS(ESI): 483 (M+H⁺).

### Beispiel XLI

### 5-(2-{(cis-3-[5-ethyl-2-(3-methoxy-phenyl)-oxazol-4-ylmethoxy]-cyclohexyl}-ethyl)-thiazolidin- 2,4-dion

Analog zu Beispiel **XXVIII** und Beispiel **XXXI** wurde aus cis-3-Allyl-cyclohexanol, Thiazolidindion und 4-Iodomethyl-5-ethyl-2-(3-methoxy-phenyl)-oxazol die Verbindung 5-(2-{(cis-3-[5-ethyl-2-(3-methoxy-phenyl)-oxazol-4-ylmethoxy]-cyclohexyl}-ethyl)-thiazolidin- 2,4-dion erhalten. 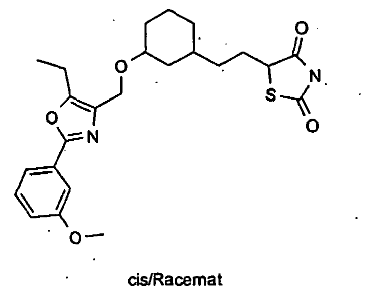 C24H30N2O5S (458.58), MS(ESI): 459 (M+H⁺).

### Beispiel XLII

### 5-(2-{(cis-3-[5-ethyl-2-(2-trifluormethyl-phenyl)-oxazol-4-ylmethoxy]-cyclohexyl}-ethyl)-thiazolidin- 2,4-dion

Analog zu Beispiel **XXVIII** und Beispiel **XXXI** wurde aus cis-3-Allyl-cyclohexanol, Thiazolidindion und 4-Iodomethyl-5-ethyl-2-(2-trifluormethyl-phenyl)-oxazol die Verbindung 5-(2-{(cis-3-[5-ethyl-2-(2-trifluormethyl-phenyl)-oxazol-4-ylmethoxy]-cyclohexyl}-ethyl)-thiazolidin- 2,4-dion erhalten. 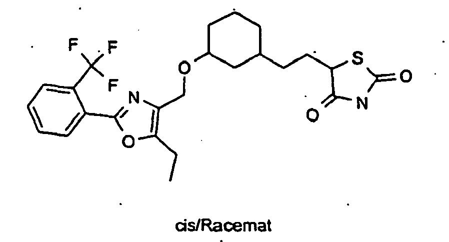 C24H27F3N2O4S (496.55), MS(ESI): 497 (M+H⁺).

### Beispiel XLIII

5-{2-[3-(5-Ethyl-2-p-tolyl-oxazol-4-ylmethoxy)-cyclohexyl]-ethyl}-thiazolidine-2,4- dione Analog zu Beispiel **XXVIII** und Beispiel **XXXI** wurde aus cis-3-Allyl-cyclohexanol, Thiazolidindion und 4-Iodomethyl-5-ethyl-2-(4-methyl-phenyl)-oxazol die Verbindung 5-{2-[cis-3-(5-Ethyl-2-p-tolyl-oxazol-4-ylmethoxy)-cyclohexyl]-ethyl}-thiazolidine-2,4- dion erhalten. 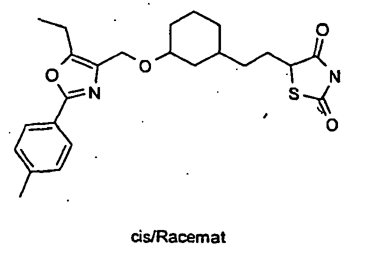 C24H30N2O4S (442.58), MS(ESI): 443 (M+H⁺).

### Beispiel XLIV

### 5-(2-{(cis-3-[5-Ethyl-2-(4-isopropyl-phenyl)-oxazol-4-ylmethoxy]-cyclohexyl}-ethyl)-thiazolidin- 2,4-dion

Analog zu Beispiel **XXVIII** und Beispiel **XXXI** wurde aus cis-3-Allyl-cyclohexanol, Thiazolidindion und 4-Iodomethyl-5-ethyl-2-(4-isopropyl-phenyl)-oxazol die Verbindung 5-(2-{cis-3-[5-Ethyl-2-(4-isopropyl-phenyl)-oxazol-4-ylmethoxy]-cyclohexyl}-ethyl)-thiazolidin- 2,4-dion erhalten. 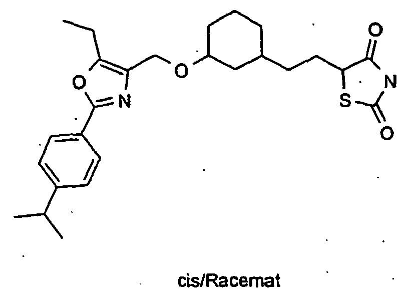 C26H34N2O4S (470.64), MS(ESI): 471 (M+H⁺).

### Beispiel XLV

### 5-(2-{(cis-3-[5-isopropyl-2-(4-methyl-phenyl)-oxazol-4-ylmethoxy]-cyclohexyl}-ethyl)-thiazolidin- 2,4-dion

Analog zu Beispiel **XXVIII** und Beispiel **XXXI** wurde aus cis-3-Allyl-cyclohexanol, Thiazolidindion und 4-Iodomethyl-5-isopropyl-2-(4-methyl-phenyl)-oxazol die Verbindung 5-(2-{cis-3-[5-tsopropy)-2-(4-methy)-phenyl)-oxazol-4-ylmethoxy]-cyclohexyl}-ethyl)- thiazolidin- 2,4-dion erhalten. 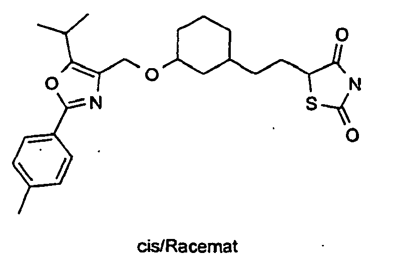 C25H32N2O4S (456.61), MS(ESI): 457 (M+H⁺).

### Beispiel XLVI

### 5-(2-{(cis-3-[5-methyl-2-(3-trifluormethyl-phenyl)-oxazol-4-ylmethoxy]-cyclohexyl}-ethyl)- thiazolidin- 2,4-dion

Analog zu Beispiel **XXVIII** und Beispiel **XXXI** wurde aus cis-3-Allyl-cyclohexanol, Thiazolidindion und 4-Iodomethyl-5-methyl-2-(3-trifluormethyl-phenyl)-oxazol die Verbindung 5-(2-{(cis-3-[5-methyl-2-(3-trifluormethyl-phenyl)-oxazol-4-ylmethoxy]-cyclohexyl}-ethyl)- thiazolidin- 2,4-dion erhalten. 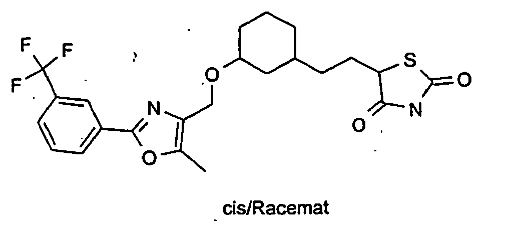 C23H25F3N2O4S (482.53), MS(ESI): 483 (M+H⁺).

### Beispiel XLVII

Die Verbindung 5-{2-[cis-3-(5-Methyl-2-m tolyl-oxazol-4-ylmethoxy)-cyclohexyl]-ethylidene}-thiazolidin-2,4-dion aus Beispiel **XXXI** wurde durch Chromatographie an chiraler Phase in die Verbindungen 5-{2-[(1S,3S)-3-(5-Methyl-2-m-tolyl-oxazol-4-ylmethoxy)-cyclohexyl]-ethylidene}-thiazolidin-2,4-dion und 5-{2-[(1R,3RF3-(5-Methyl-2-m-tolyl-oxazol-4-ylmethoxy)-cyclohexyl]-ethylidene}-thiazolidin-2,4-dion getrennt. 5-{2-[(1R,3R)-3-(5-Methyl-2-m-tolyl-oxazol-4-ylmethoxy)-cyclohexyl]-ethylidene}-thiazolidin-2,4-dion: 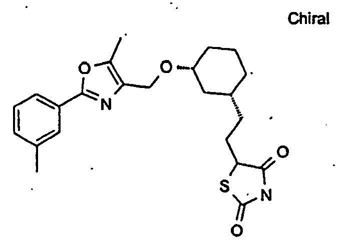 C₂₃H₂₈N₂O₄S (428.55), MS(ESI): 429 (M+H⁺)

### Verbindungen 5-{2-[(1S,3S)-3-(5-Methyl-2-m-tolyl-oxazol-4-ylmethoxy)-cyclohexyl]-ethylidene}-thiazolidin-2,4-dion:

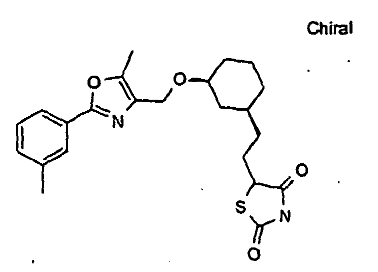

C₂₃H₂₈N₂O₄S (428.55), MS(ESI): 429 (M+H⁺).

### Beispiel XLVIII

Die Verbindung 5-(2-{cis-3-[5-Ethyl-2-(4-isopropyl-phenyl)-oxazol-4-ylmethoxy]-cyclohexyl}-ethyl)- thiazolidin- 2,4-dion aus Beispiel **XLIV** wurde durch Chromatographie an chiraler Phase in die Verbindungen 5-(2-{(1s,3S)-3-[5-Ethyl-2-(4-isopropyl-phenyl)-oxazol-4-ylmethoxy]-cyclohexyl}-ethyl)- thiazolidin- 2,4-dion und 5-(2-{(1R,3R)-3-[5-Ethyl-2-(4-isopropyl-phenyl)-oxazol-4-ylmethohy]-cyclohexyl}-ethyl)-thiazolidin- 2,4-dion getrennt.

### 5-(2-{(1s,3S)-3-[5-Ethyl-2-(4-isopropyl-phenyl)-oxazol-4-ylmethoxy]-cyclohexyl}-ethyl)-thiazolidin- 2,4-dion:

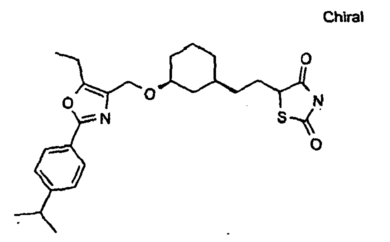

C26H34N2O4S (470.64), MS(ESI): 471 (M+H⁺).

### 5-(2-{(1R,3R)-3-[5-Ethyl-2-(4-isopropyl-phenyl)-oxazol-4-ylmethoxy]-cyclohexyl}-ethyl)-thiazolidin-2,4-dion:

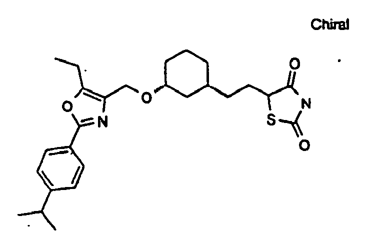

C26H34N2O4S (470.64), MS(ESI): 471 (M+H⁺).

### Beispiel XLIX

Die Verbindung 5-{2-[cis-3-(5-Methyl-2-p-tolyl-oxazol-4-ylmethoxy)-cyclohexyl]-ethyl}-thiazolidin-2,4-dion aus Beispiel **XXXIII** wurde durch Chromatographie an chiraler Phase in die 5-{2-[(1S,3S)-3-(5-Methyl-2-p-tolyl-oxazol-4-ylmethoxy)-cyclohexyl]-ethyl}-thiazolidin-2,4-dion und 5-{2-[(1 R,3R)-3-(5-Methyl-2-p-tolyl-oxazol-4-ylmethoxy)-cyclohexyl]-ethyl}-thiazolidin-2,4-dion getrennt.

### 5-{2-[(1S,3S)-3-(5-Methyl-2-p-tolyl-oxazol-4-ylmethoxy)-cyclohexyl]-ethyl}-thiazolidin-2,4-dion

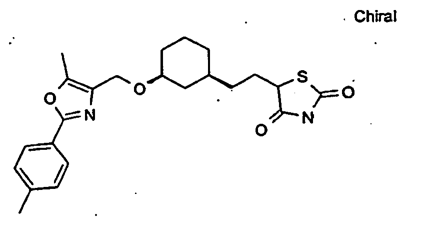

C₂₃H₂₈N₂O₄S (428.55), MS(ESI): 429 (M+H⁺).

### 5-{2-[(1R,3R)-3-(5-Methyl-2-p-tolyl-oxazol-4-ylmethoxy)-cyclohexyl]-ethyl}-thiazolidin-2,4-dion

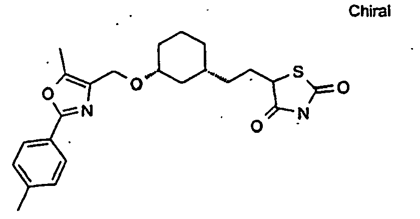

C₂₃H₂₈N₂O₄S (428.55), MS(ESI): 429 (M+H⁺).

### Beispiel L

### 5-(2-{(1S,3S)-3-[2-(3,4-Dimethyl-phenyl)-5-ethyl-oxazol-4-ylmethoxy]-cyclohexyl}-ethyl)- thiazolidin-2,4-dion

### ((1S,3S)-3-Allyl-cydohexanol

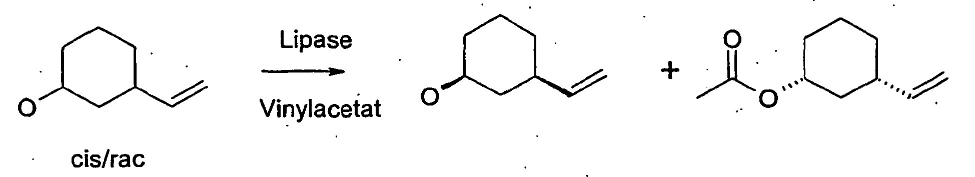

50 g cis-3-Allyl-cyclohexanol werden in 200 ml Vinylacetat gelöst und mit 3g Lipase. (Novozym) versetzt. Man rührt bei. RT nach bis ein Umsatz von 58 % erreicht ist (Umsatzkontrolle mittels GC). Das Enzym wird abfiltriert und das Vinylacetat im Vakuum entfernt. Der reslutierende Rückstand wird an Kieselgel chromatographisch gereinigt. Man erhält 17 g ((1S,3S)-3-Ally-cyclohexanol als farbloses Öl.
C8H140 (126.20), Rf(n-Heptan:Ethylacetat = 1:1) = 0.46.

### 5-(2-{(1S,3S)-3-[2-(3,4-Dimethyl-phenyl)-5-ethyl-oxazol-4-ylmethoxy]-cyclohexyl}-ethyl)- thiazolidin-2,4-dion

Analog zu Beispiel **XXVIII** und Beispiel **XXXI** wurde aus (1S,3S)-3-Allyl-cyclohexanol, Thiazolidindion und 4-Iodomethyl-5-ethyl-2-(3,4-dimethyl-phenyl)-oxazol die Verbindung 5-(2-{(1 S,3S)-3-[2-(3,4-Dimethyl-phenyl)-5-ethyl-oxazol-4-ylmethoxy]-cyclohexyl}-ethyl)- thiazolidin-2,4-dion erhalten. 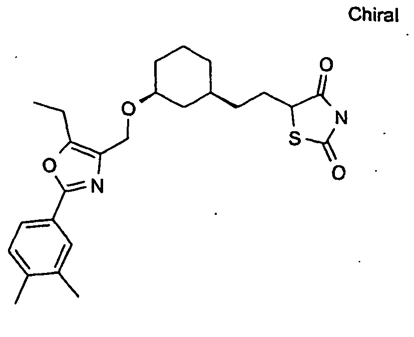 C₂₅H₃₂N₂O₄S (456.61), MS(ESI): 457 (M+H⁺).

### Beispiel LI

### 5-(2-{(1S,3S)-3-[5-Ethyl-2-(4-trifluoromethyl-phenyl)-oxazol-4-ylmethoxy]-cyclohexyl}-ethyl)- thiazolidin-2,4-dion

Analog zu Beispiel **XXVIII** und Beispiel **XXXI** wurde aus (1S,3S)-3-Allyl-cyclohexanol, Thiazolidindion und 4-Iodomethyl-5-ethyl-2-(4-trifluormethyl-phenyl)-oxazol die Verbindung 5-(2-{(1S,3S)-3-[5-Ethyl-2-(4-trifluoromethyl-phenyl)-oxazol-4-ylmethoxy]-cyclohexyl}-ethyl)- thiazolidin-2,4-dion erhalten. 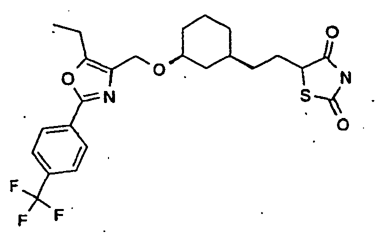 C24H27F3N2O4S (496.55), MS(ESI): 497 (M+H⁺).

### Beispiel LII

### 5-{2-[(1S,3S)-3-(5-Ethyl-2-naphthalen-2-yl-oxazol-4-ylmethoxy)-cyclohexyl]-ethyl}-thiazolidin- 2,4-dion

Analog zu Beispiel **XXVIII** und Beispiel **XXXI** wurde aus (1S,3S)-3-Allyl-cyclohexanol, Thiazolidindion und 5-Ethyl-4-iodomethyl-2-naphthalen-2-yl-oxazole die Verbindung 5-{2-[(1S,3S)-3-(5-Ethyl-2-naphthalen-2-yl-oxazol-4-ylmethoxy)-cyclohexyl]-ethyl}-thiazolidin- 2,4-dion erhalten. 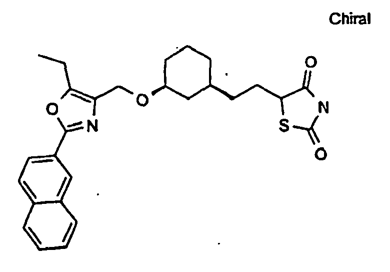 C27H30N2O4S (478.62), MS(ESI): 479 (M+H⁺).

### Beispiel LIII

### 5-(2-{(1S,3S)-3-[5-Ethyl-2-(3-trifluoromethyl-phenyl)-oxazol-4-ylmethoxy]-cyclohexyl}-ethyl)- thiazolidin- 2,4-dion

Analog zu Beispiel **XXVIII** und Beispiel **XXXI** wurde aus (1S,3S)-3-Allyl-cyclohexanol, Thiazolidindion und 4-Iodomethyl-5-ethyl-2-(3-trifluormethyl-phenyl)-oxazol die Verbindung 5-(2-{(1S,3S)-3-[5-Ethyl-2-(3-trifluoromethyl-phenyl)-oxazol-4-ylmethoxy]-cyclohexyl}-ethyl)- thiazolidin- 2,4-dion erhalten. 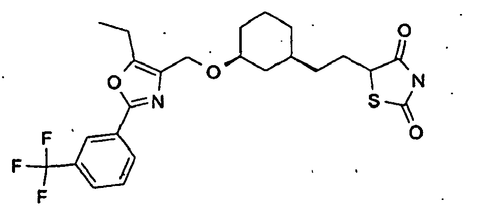 C24H27F3N2O4S (496.55), MS(ESI): 497 (M+H⁺).

### Beispiel LIV

### 5-(2-{(1S,3S)-3-[5-Ethyl-2-(4-tert.-butyl-phenyl)-oxazol-4-ylmethoxy]-cyclohexyl}-ethyl)-thiazolidin- 2,4-dion

Analog zu Beispiel **XXVIII** und Beispiel **XXXI** wurde aus (1S,3S)-3-Allyl-cyclohexanol, Thiazolidindion und 4-Iodomethyl-5-ethyl-2-(4-tert.-butyl-phenyl)-oxazol die Verbindung 5-(2-{(1S,3S)-3-[5-Ethyl-2-(4-tert.-butyl-phenyl)-oxazol-4-ylmethoxy]-cyclohexyl}-ethyl)-thiazolidin- 2,4-dion erhalten. 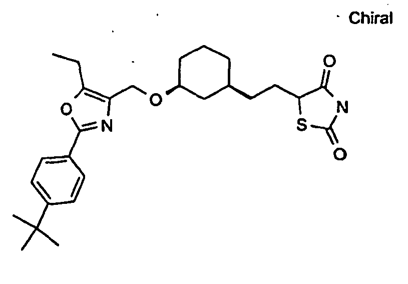 C27H36N2O4S (484.66), MS(ESI): 485 (M+H⁺).

### Beispiel LV

### 5-(2-{(1S,3S)-3-[5-Isopropyl-2-(3,4-dimethyl-phenyl)-oxazol-4-ylmethoxy]-cyclohexyl}-ethyl)- thiazolidin- 2,4-dion

Analog zu Beispiel **XXVIII** und Beispiel **XXXI** wurde aus (1S,3S)-3-Allyl-cyclohexanol, Thiazolidindion und 4-Iodomethyl-5-isopropyl-2-(3,4-dimethyl-phenyl)-oxazol die Verbindung 5-(2-{(1S,3S)-3-[5-Isopropyl-2-(3,4-dimethyl-phenyl)-oxazol-4-ylmethoxy]-cyclohexyl}-ethyl)- thiazolidin- 2,4-dion erhalten. 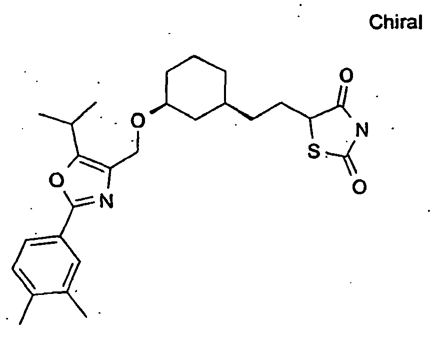 C26H34N2O4S (470.64), MS(ESI): 471 (M+H⁺).

### Beispiel LVI

### 5-(2-{(1S,3S)-3-[5-Ethyl-2-(4-isobutyl-phenyl)-oxazol-4-ylmethoxy]-cyclohexyl}-ethyl)-thiazolidin- 2,4-dion

Analog zu Beispiel **XXVIII** und Beispiel **XXXI** wurde aus (1S,3S)-3-Allyl-cyclohexanol, Thiazolidindion und 4-Iodomethyl-5-ethyl-2-(4-isobutyl-phenyl)-oxazol die Verbindung 5-(2-{(1S,3S)-3-[5-Ethyl-2-(4-isobutyl-phenyl)-oxazol-4-ylmethoxy]-cyclohexyl}-ethyl)-thiazolidin- 2,4-dion erhalten. 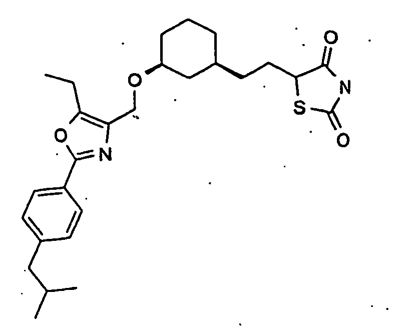 C27H36N2O4S (484.66), MS(ESI): 485 (M+H⁺).

### Beispiel LVII

### 5-(2-{(1S,3S)-3-[5-Isopropyl-2-(3-trifluormethyl-phenyl)-oxazol-4-ylmethoxy]-cyclohexyl}-ethyl)- thiazolidin- 2,4-dion

Analog zu Beispiel **XXVIII** und Beispiel **XXXI** wurde aus (1S,3S)-3-Allyl-cyclohexanol, Thiazolidindion und 4-Iodomethyl-5-isopropyl-2-(3-trifluormethyl -phenyl)-oxazol die Verbindung 5-(2-{(1S,3S)-3-[5-Isopropyl-2-(3-trifluormethyl-phenyl)-oxazol-4-ylmethoxy]-cyclohexyl}-ethyl)- thiazolidin- 2,4-dion erhalten. 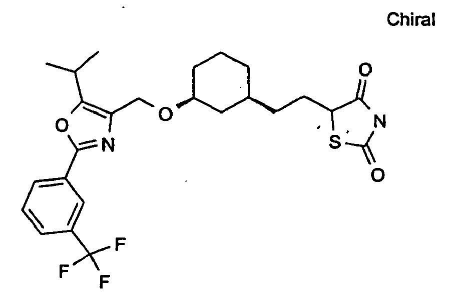 C25H29F3N2O4S (510.58), MS(ESI): 511 (M+H⁺).

### Beispiel LVIII

### 5-(2-{(1S,3S)-3-[5-Isopropyl-2-(4-tert.-butyl-phenyl)-oxazol-4-ylmethoxy]-cyclohexyl}-ethyl)- thiazolidin- 2,4-dion

Analog zu Beispiel **XXVIII** und Beispiel **XXXI** wurde aus (1S,3S)-3-Allyl-cyclohexanol, Thiazolidindion und 4-Iodomethyl-5-isopropyl-2-(4-tert.-butyl -phenyl)-oxazol die Verbindung 5-(2-{(1S,3S)-3-[5-Isopropyl-2-(4-tert.-butyl -phenyl)-oxazol-4-ylmethoxy]-cyclohexyl}-ethyl)- thiazolidin- 2,4-dion erhalten. 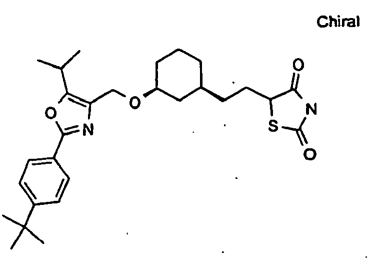 C28H38N2O4S (498.69), MS(ESI): 499 (M+H⁺).

### Beispiel LIX

### Beispiel LIX

### 5-(2-{(1S,3S)-3-[5-Isopropyl-2-(4-isobutyl-phenyl)-oxazol-4-ylmethoxy]-cyclohexyl}-ethyl)- thiazolidin- 2,4-dion

Analog zu **Beispiel XXVIII** und Beispiel **XXXI** wurde aus (1S,3S)-3-Allyl-cyclohexanol, Thiazolidindion und 4-lodomethyl-5-isopropyl-2-(4-isobutyl -phenyl)-oxazol die Verbindung 5-(2-{(1S,3S)-3-[5-Isopropyl-2-(4-isobutyl -phenyl)-oxazol-4-ylmethoxy]-cyclohexyl}-ethyl)- thiazolidin- 2,4-dion erhalten. 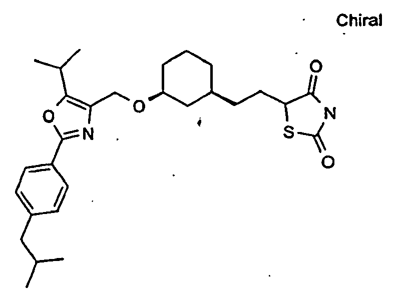 C28H38N2O4S (498.69), MS(ESI): 499 (M+H⁺).

### Beispiel LX

### 5-(2-{(1S,3S)-3-[5-Isopropyl-2-(4-trifluormethyl-phenyl)-oxazol-4-ylmethoxy]-cyclohexyl}-ethyl)-thiazolidin- 2,4-dion

Analog zu Beispiel **XXVIII** und Beispiel **XXXI** wurde aus (1S,3S)-3-Allyl-cyclohexanol, Thiazolidindion und 4-lodomethyl-5-isopropyl-2-(4- trifluormethyl -phenyl)-oxazol die Verbindung 5-(2-{(1S,3S)-3-[5-Isopropyl-2-(4-trifluormethyl -phenyl)-oxazol-4-ylmethoxy]-cyclohexyl}-ethyl)-thiazolidin- 2,4-dion erhalten. 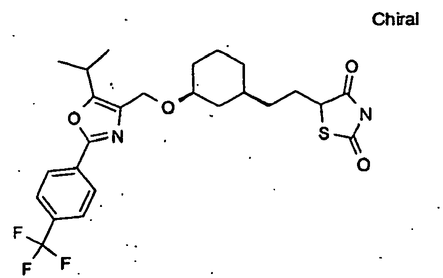 C25H29F3N2O4S (510.58), MS(ESI): 511 (M+H⁺).

### Beispiel LXI

### 5-{2-[(1S,3S)-3-(5-Isopropyl-2-naphthalen-2-yl-oxazol-4-ylmethoxy)-cyclohexyl]-ethyl}-thiazolidin-2,4-dion

Analog zu Beispiel **XXVIII** und Beispiel **XXXI** wurde aus (1S,3S)-3-Allyl-cyclohexanol, Thiazolidindion und 5-Isopropyl-4-iodomethyl-2-naphthalen-2-yl-oxazole die Verbindung 5-{2-[(1S,3S)-3-(5-Isopropyl-2-naphthalen-2-yl-oxazol-4-ylmethoxy)-cyclohexyl]-ethyl}- thiazolidin-2,4-dion erhalten. 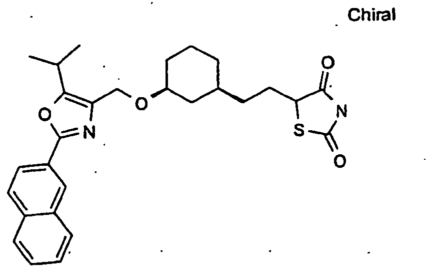 C28H32N2O4S (492.64), MS(ESI): 493 (M+H⁺).

### Beispiel LXII

### 5-(2-{cis-3-[2-(3-Methoxy-phenyl)-5-methyl-oxazol-4-ylmethoxy]-cyclohexyl}-ethyl)-3-methyl- thiazolidin-2,4-dion

Analog zu Beispiel **XXVIII** und Beispiel **XXXI** wurde aus cis-3-Allyl-cyclohexanol, 3-Methyltiazolidindion und 4-Iodomethyl-5-methyl-2-(3-methoxy-phenyl)-oxazol die Verbindung 5-(2-{cis-3-[2-(3-Methoxy-phenyl)-5-methyl-oxazol-4-ylmethoxy]-cyclohexyl}-ethyl)-3-methyl- thiazolidin-2,4-dion erhalten. 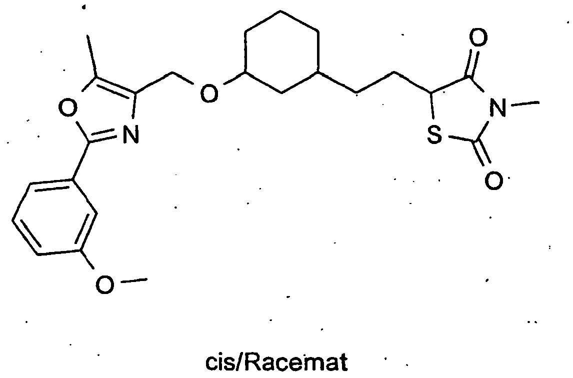 C24H30N2O5S (458.58), MS(ESI): 459 (M+H⁺).

### Beispiel LXIII

### 5-(2-{cis-3-[2-(3-Methoxy-phenyl)-5-methyl-oxazol-4-ylmethoxy]-cyclohexyl}-ethyl)-3-phenyl- thiazolidin-2,4-dion

Analog zu Beispiel **XXVIII** und Beispiel **XXXI** wurde aus cis-3-Allyl-cyclohexanol, 3-Phenyltiazolidindion und 4-lodomethyl-5-methyl-2-(3-methoxy-phenyl)-oxazol die Verbindung 5-(2-{cis-3-[2-(3-Methoxy-phenyl)-5-methyl-oxazol-4-ylmethoxy]-cyclohexyl}-ethyl)-3-phenyl- thiazolidin-2,4-dion erhalten. 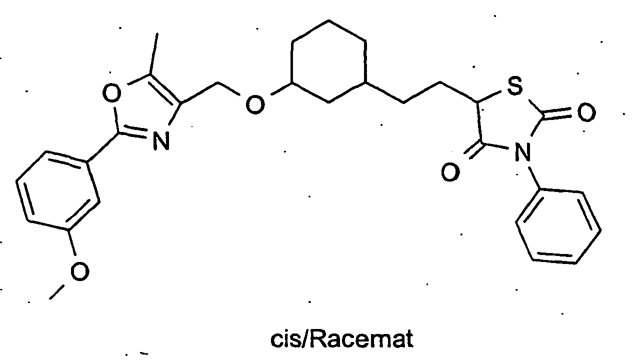 C29H32N2O5S (520.65), MS(ESI): 521 (M+H⁺).

### Beispiel LXIV

### 5-(2-{cis-3-[2-(4-Methyl-phenyl)-5-methyl-oxazol-4-ylmethoxy]-cyclohexyl}-ethyl)-3-methyl- thiazolidin-2,4-dion

Analog zu Beispiel **XXVIII** und Beispiel **XXXI** wurde aus cis-3-Allyl-cyclohexanol, 3-Methyltiazolidindion und 4-lodomethyl-5-methyl-2-(4-methyl-phenyl)-oxazol die Verbindung 5-(2-{cis-3-[2-(4-Methyl-phenyl)-5-methyl-oxazol-4-ylmethoxy]-cyclohexyl}-ethyl)-3-methyl- thiazolidin-2,4-dion erhalten. 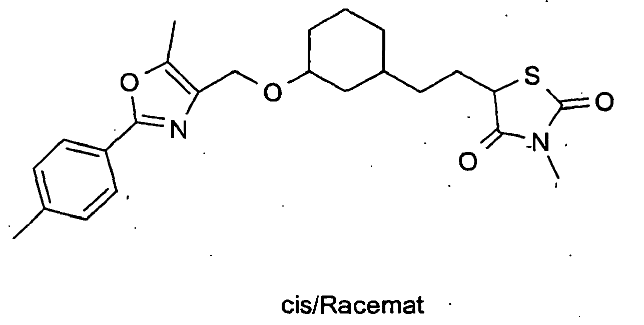 C24H30N2O4S (442.58), MS(ESI): 443 (M+H⁺).

### Beispiel LXV

### 5-(2-{cis-3-[2-(4-Methyl-phenyl)-5-methyl-oxazol-4-ylmethoxy]-cyclohexyl}-ethyl)-3-benzyl- thiazolidin-2,4-dion

Analog zu Beispiel **XXVIII** und Beispiel **XXXI** wurde aus cis-3-Allyl-cyclohexanol, 3-Benzyltiazolidindion und 4-Iodomethyl-5-methyl-2-(4-methyl-phenyl)-oxazol die Verbindung 5-(2-{cis-3-[2-(4-Methyl-phenyl)-5-methyl-oxazol-4-ylmethoxy]-cyclohexyl}-ethyl)-3-benzyl-thiazolidin-2,4-dion erhalten. 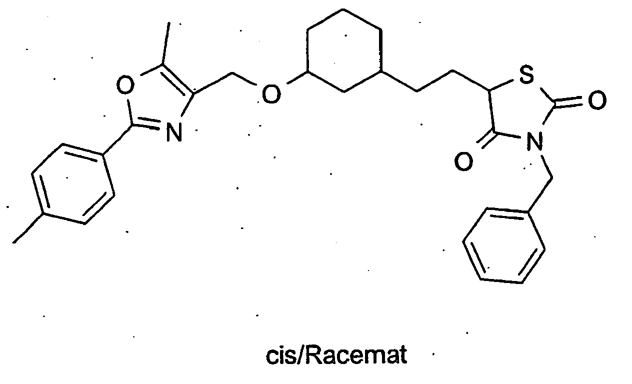 C30H34N2O4S (518.58), MS(ESI): 519 (M+H⁺).

### Beispiel LXVI

### 5-(2-{cis-3-[2-(3-Methoxy-phenyl)-5-isopropyl-oxazol-4-ylmethoxy]-cyclohexyl}-ethyl)-3-methyl- thiazolidin-2,4-dion

Analog zu Beispiel **XXVIII** und Beispiel **XXXI** wurde aus cis-3-Allyl-cyclohexanol, 3-Methyltiazolidindion und 4-Iodomethyl-5-isopropyl-2-(3-methoxy-phenyl)-oxazol die Verbindung 5-(2-{cis-3-[2-(3-Methoxy-phenyl)-5-isopropyl-oxazol-4-ylmethoxy]-cyclohexyl}-ethyl)-3-methyl- thiazolidin-2,4-dion erhalten. 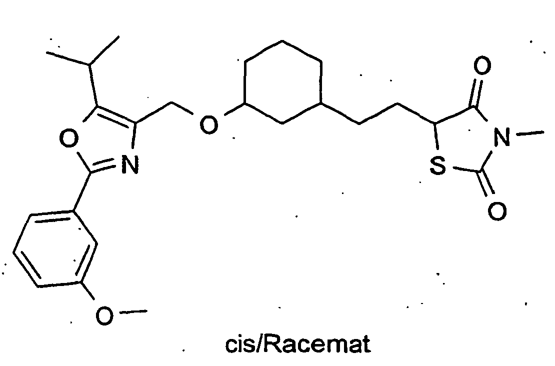 C26H34N2O5S (486.64), MS(ESI): 487 (M+H⁺).

### Beispiel LXVII

### 5-(2-{cis-3-[2-(3-Methoxy-phenyl)-5-phenyl-oxazol-4-ylmethoxy]-cyclohexyl}-ethyl)-3-methyl- thiazolidin-2,4-dion

Analog zu Beispiel **XXVIII** und Beispiel **XXXI** wurde aus cis-3-Allyl-cyclohexanol, 3-Methyltiazolidindion und 4-Iodomethyl-5-phenyl-2-(3-methoxy-phenyl)-oxazol die Verbindung 5-(2-{cis-3-[2-(3-Methoxy-phenyl)-5-phenyl-oxazol-4-ylmethoxy]-cyclohexyl}-ethyl)-3-methyl-thiazolidin-2,4-dion erhalten. 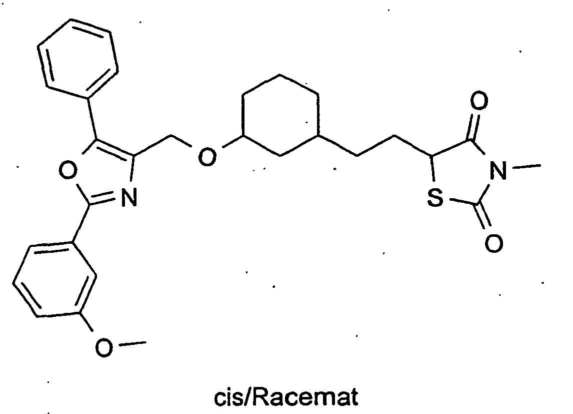 C29H32N2O5S (520.65), MS(ESI): 521 (M+H⁺).

### Beispiel LXVIII

### 5-(2-{cis-3-[2-(3-Methoxy-phenyl)-5-phenyl-oxazol-4-ylmethoxy]-cyclohexyl}ethyl)-3-phenyl- thiazolidin-2,4-dion

Analog zu Beispiel **XXVIII** und Beispiel **XXXI** wurde aus cis-3-Allyl-cyclohexanol, 3-Phenyltiazolidindion und 4-Iodomethyl-5-phenyl-2-(3-methoxy-phenyl)-oxazol die Verbindung 5-(2-{cis-3-[2-(3-Methoxy-phenyl)-5-phenyl-oxazol-4-ylmethoxy]-cyclohexyl}-ethyl)-3-phenyl- thiazolidin-2,4-dion erhalten. 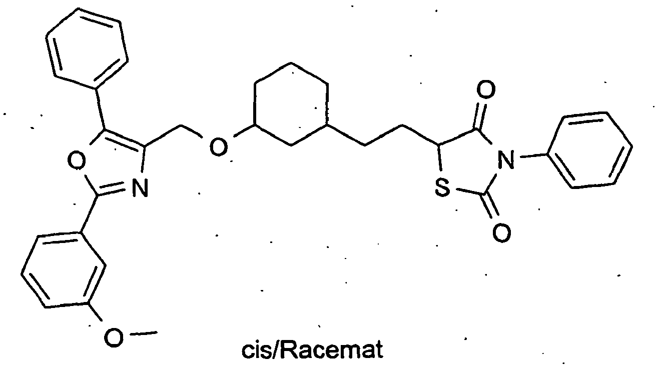 C34H34N2O5S (582.72), MS(ESI): 583 (M+H⁺).

## Patentansprüche

1. Verbindungen der Formel I 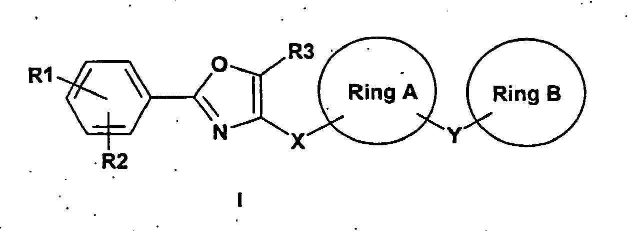 worin bedeuten
Ring A (C₃-C₈)-Cycloalkandiyl, (C₃-C₈)-Cycloalkendiyl, wobei in den Cycloalkandiyl- oder Cycloalkendiylringen ein oder mehrere Kohlenstoffatome durch Sauerstoffatome ersetzt sein können;
R1, R2 unabhängig voneinander H, F, Br, CF₃, OCF₃, (C₁-C₆)-Alkyl, O-(C₁-C₆)- Alkyl, SCF₃, SF₅, OCF₂-CHF₂, O-Phenyl, OH, NO₂; oder
R1 und R2 in Nachbarstellung zusammen mit dem Phenylring für ein kondensiertes, ganz, teilweise oder nicht gesättigtes bicyclisches (C₉-C₁₂)-Aryl- oder (C₉- C₁₁)-Heteroaryl-Ring-System;
R3 H, CF₃, (C₁-C₆)-Alkyl, (C₁-C₆)-Alkoxy, (C₃-C₈)-Cycloalkyl, Phenyl;
X (C₁-C₆)-Alkandiyl, wobei in der Alkandiylgruppe ein oder mehrere Kohlenstoffatome durch Sauerstoffatome ersetzt sein können;
Y (C₁-C₆)-Alkandiyl oder (C₁-C₆)-Alkendiyl, wobei in der Alkandiyl- oder Alkendiylgruppe ein oder mehrere CH₂-Gruppen durch O CO, S, SO oder SO₂ ersetzt sein können und Alkandiyl oder Alkendiyl gegebenenfalls durch OH substituiert sein können;
Ring B Phenyl, das ein- oder zweifach substituiert sein kann mit NO₂, Cl, CN, (C₁- C₆)-Alkyl, (C₁-C₆)-Alkoxy oder Tetrazol; oder Pyrrolidin-2-on, das zusätzlich ein N- oder S-Atom im Ring enthält und durch Oxo oder Thioxo substituiert ist und durch R4 am N-Atom substituiert sein kann;
R4 (C₁-C₆)-Alkyl, Phenyl, Benzyl;
sowie deren physiologisch verträgliche Salze.

2. Verbindungen der Formel gemäß Anspruch 1, worin bedeuten
Ring A (C₃-C₈)-Cycloalkandiyl, (C₃-C₈)-Cycloalkendiyl, wobei in den Cycloalkandiyl- oder Cycloalkendiylringen ein Kohlenstoffatom durch ein Sauerstoffatom ersetzt sein kann;
R1, R2 unabhängig voneinander H, F, Br, CF₃, OCF₃, (C₁-C₆)-Alkyl, O-(C₁-C₆)- Alkyl, SCF₃, SF₅, OCF₂CHF₂, O-Phenyl, OH, NO₂; oder
R1 und R2 in Nachbarstellung zusammen mit dem Phenylring für ein kondensiertes, ganz, teilweise oder nicht gesättigtes bicyclisches (C₉-C₁₂)-Aryl- oder (C₉- C₁₁)-Heteroaryl-Ring-System;
R3 H, CF₃, (C₁-C₆)-Alkyl, (C₃-C₈)-Cycloalkyl, Phenyl;
X (C₁-C₆)-Alkandiyl, wobei in der Alkandiylgruppe ein Kohlenstoffatom durch ein Sauerstoffatom ersetzt sein kann;
Y (C₁-C₆)-Alkandiyl oder (C₁-C₆)Alkendiyl, wobei in der Alkandiyl- oder Alkendiylgruppe ein oder zwei CH₂-Gruppen durch O, CO, S, SO oder SO₂ ersetzt sein können und Alkandiyl oder Alkendiyl gegebenenfalls durch OH substituiert sein können;
Ring B Phenyl, das ein- oder zweifach substituiert sein kann mit NO₂, Cl, CN, (C₁- C₆)-Alkyl , (C₁-C₆)-Alkoxy oder Tetrazol; oder Pyrrolidin-2-on, das zusätzlich ein N- oder S-Atom in 4-Position enthält und durch Oxo oder Thioxo in 5-Position substituiert ist und durch R4 am N1-Atom substituiert sein kann;
R4 (C₁-C₆)-Alkyl, Phenyl, Benzyl;
sowie deren physiologisch verträgliche Salze.

3. Verbindungen der Formel I gemäß Anspruch 1 oder 2, worin bedeuten
Ring A (C₃-C₈)-Cycloalkandiyl, worin ein Kohlenstoffatom durch ein Sauerstoffatom ersetzt ist;
R1, R2 unabhängig voneinander H, F, Br, CF₃,OCF₃, (C₁-C₆)-Alkyl, O-(C₁-C₆)- Alkyl, SCF₃, SF₅, OCF₂-CHF₂, O-Phenyl, OH, NO₂; der
R1 und R2 in Nachbarstellung zusammen mit dem Phenylring für ein kondensiertes, nicht gesättigtes bicyclisches (C₉-C₁₂)-Aryl- oder (C₉-C₁₁)-Heteroaryl-Ring- System;
R3 H, CF₃, (C₁-C₆)-Alkyl, (C₃-C₈)-Cycloalkyl, Phenyl;
X (C₁-C₆)-Akandiyl, worin das C1-Kohlenstoffatom durch ein Sauerstoffatom ersetzt ist;
Y (C₁-C₆)-Alkandiyl oder (C₁-C₆)-Alkendiyl, wobei in der Alkandiyl- oder Alkendiylgruppe ein oder zwei CH₂-Gruppen durch O, CO oder SO₂ ersetzt sein können und Alkandiyl oder Alkendiyl gegebenenfalls durch OH substituiert sein können;
Ring B Phenyl, das ein- oder zweifach substituiert sein kann mit NO₂, Cl, CN, (C₁- C₆)-Alkyl, (C₁-C₆)-Alkoxy oder Tetrazol; oder Pyrrolidin-2-on, das zusätzlich ein N- oder S-Atom in 4-Position enthält und durch Oxo oder Thioxo in 5-Position substituiert ist und durch R4 am N1-Atom substituiert sein kann;
R4 (C₁-C₆)-Alkyl, Phenyl, Benzyl;
sowie deren physiologisch verträgliche Salze.

4. Verbindungen der Formel I gemäß den Ansprüchen 1 bis 3, worin bedeuten
Ring A Cyclohexan-1,3-diyl;
R1, R2 unabhängig voneinander H, F, Br, CF₃, OCF₃, (C₁-C₆)-Alkyl, O-(C₁-C₆)- Alkyl, SCF₃, SF₅, OCF₂-CHF₂, O-Phenyl, OH, NO₂; oder
R1 und R2 in Nachbarstellung zusammen mit dem Phenylring für ein kondensiertes, nicht gesättigtes bicyclisches (C₉-C₁₀)-Aryl- oder (C₉-C₁₀)-Heteroaryl-Ring- System steht;
R3 H, CF₃, (C₁-C₆)-Alkyl, (C₃-C₆)-Cycloalkyl, Phenyl;
X CH₂-O;
Y (C₁-C₄)-Alkandiyl,O-(C₁-C₄)-Alkandiyl, (C₁-C₄)-Alkendiyl, O-(C₁-C₄) Alkendiyl, O-SO₂, O-CO, wobei Alkandiyl durch OH substituiert sein kann;
Ring B Phenyl, das ein- oder zweifach substituiert sein kann mit NO₂, Cl, CN, (C₁- C₆)-Alkyl , (C₁-C₆)-Alkoxy oder Tetrazol; oder Thiazolidin-1,4-dion, das durch R4 am N3-Atom substituiert sein kann;
R4 (C₁-C₆)-Alkyl, Phenyl, Benzyl;
sowie deren physiologisch verträgliche Salze

5. Verbindungen der Formel I gemäß den Ansprüchen 1 bis 4, worin bedeuten
Ring A Cyclohexan-1,3-diyl;
R1, R2 unabhängig voneinander H, F, Br, CF₃, (C₁-C₆)-Alkyl, O-(C₁-C₆)-Alkyl; oder
R1 und R2 zusammen mit dem Phenylring für Naphthyl steht;
R3 (C₁-C₆)-Alkyl, (C₅-C₆)-Cycloalkyl, Phenyl;
X CH₂-O;
Y (C₁-C₄)-Alkandiyl, O-(C₁-C₄)-Alkandiyl, (C₁-C₄)-Alkendiyl, O-(C₁-C₄)- Alkendiyl, O-SO₂, O-CO, wobei Alkandiyl durch OH substituiert sein kann;
Ring B Phenyl, das ein- oder zweifach substituiert sein kann mit NO₂, Cl, CN, (C₁- C₆)-Alkyl , (C₁-C₆)-Alkoxy oder Tetrazol oder
Ring B Thiazolidin-2,4-dion, das durch R4 am N3-Atom substituiert sein kann;
R4 (C₁-C₆)-Alkyl, Phenyl, Benzyl;
sowie deren physiologisch verträgliche Salze.

6. Verbindungen der Formel I gemäß den Ansprüchen 1 bis 5, worin bedeuten
R2 Wasserstoff und
R1 in meta oder para-Stellung verknüpft ist.

7. Verbindungen der Formel I gemäß den Ansprüchen 1 bis 6, worin bedeutet
Y -CH₂-CH₂-.

8. Arzneimittel enthaltend eine oder mehrere der Verbindungen der Formel I gemäß einem oder mehreren der Ansprüche 1 bis 7.

9. Arzneimittel enthaltend eine oder mehrere der Verbindungen der Formel I gemäß einem oder mehreren der Ansprüche 1 bis 7 und einen oder mehrere Wirkstoffe, die günstige Wirkungen auf Stoffwechselstörungen oder damit assozierte Erkrankungen haben.

10. Arzneimittel enthaltend eine oder mehrere der Verbindungen der Formel I gemäß einem oder mehreren der Ansprüche 1 bis 7 und einen oder mehrere Antidiabetika.

11. Arzneimittel enthaltend eine oder mehrere der Verbindungen der Formel I gemäß einem oder mehreren der Ansprüche 1 bis 7 und einen oder mehrere Lipidmodulatoren.

12. Verbindungen der Formel I gemäß einem oder mehreren der Ansprüche 1 bis 7 zur Verwendung bei der Behandlung und/oder Prävention von Störungen des Fettsäurestoffwechsels und Glucoseverwertungsstörungen.

13. Verbindungen der Formel I gemäß einem oder mehreren der Ansprüche 1 bis 7 zur Verwendung bei der zur Behandlung und/oder Prävention von Störungen, bei denen Insulin Resistenz eine Rolle spielt.

14. Verbindungen der Formel I gemäß einem oder mehreren der Ansprüche 1 bis 7 zur Verwendung bei der Behandlung und/oder Prävention von Diabetes mellitus und der damit verbundenen Folgeerkrankungen.

15. Verbindungen der Formel I gemäß einem oder mehreren der Ansprüche 1 bis 7 zur Verwendung bei der zur Behandlung und/oder Prävention von Dyslipidämien und deren Folgen.

16. Verbindungen der Formel I gemäß einem oder mehreren der Ansprüche 1 bis 7 zur Verwendung bei der zur Behandlung und/oder Prävention von Zuständen, die mit dem Metabolischen Syndrom assoziert sind.

17. Verbindungen gemäß einem oder mehreren der Ansprüche 1 bis 7 in Kombination mit mindestens einem weiteren Wirkstoff zur Verwendung bei der Behandlung und/oder Prävention von Störungen des Fettsäurestoffwechsels und Glucoseverwertungsstörungen.

18. Verbindungen gemäß einem oder mehreren der Ansprüche 1 bis 7 in Kombination mit mindestens einem weiteren Wirkstoff zur Verwendung bei der zur Behandlung und/oder Prävention von Störungen, bei denen Insulin Resistenz eine Rolle spielt.

19. Verfahren zur Herstellung eines Arzneimittels enthaltend eine oder mehrere der Verbindungen gemäß einem oder mehreren der Ansprüche 1 bis 7, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 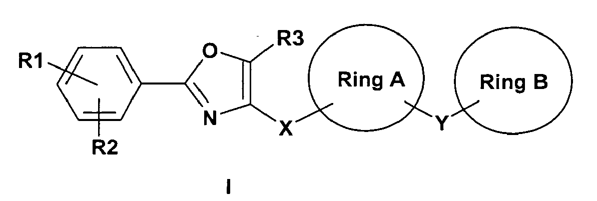 in which
Ring A is (C₃-C₈)-cycloalkanediyl or (C₃-C₈)-cyclo- alkenediyl, where in the cycloalkanediyl or cycloalkenediyl rings one or more carbon atoms may be replaced by oxygen atoms;
R1, R2 independently of one another are H, F, Br, CF₃, OCF₃, (C₁-C₆)-alkyl, O-(C₁-C₆)-alkyl, SCF₃, SF₅, OCF₂-CHF₂, O-phenyl, OH, or NO₂; or
R1 and R2 in juxtaposition together with the phenyl ring are a fused, unsaturated or completely or partially saturated bicyclic (C₉-C₁₂)-aryl or (C₉-C₁₁)-heteroaryl ring system;
R3 is H, CF₃, (C₁-C₆)-alkyl, (C₁-C₆)-alkoxy, (C₃-C₈)-cycloalkyl or phenyl;
X is (C₁-C₆)-alkanediyl, where one or more carbon atoms in the alkanediyl group may be replaced by oxygen atoms;
Y is (C₁-C₆)-alkanediyl or (C₁-C₆)-alkenediyl, where one or more CH₂ groups in the alkanediyl or alkenediyl group may be replaced by O, CO, S, SO or SO₂ and alkanediyl or alkenediyl may be unsubstituted or substituted by OH;
Ring B is phenyl which may be mono- or disubstituted by NO₂, Cl, CN, (C₁-C₆)-alkyl, (C₁-C₆)-alkoxy or tetrazole; or pyrrolidin-2-one which additionally contains a nitrogen or sulfur atom in the ring and is substituted by oxo or thioxo and may be substituted on the nitrogen atom by R4;
R4 is (C₁-C₆)-alkyl, phenyl or benzyl;
and its physiologically acceptable salts.

2. A compound of the formula I as claimed in claim 1, in which
Ring A is (C₃-C₈)-cycloalkanediyl or (C₃-C₈)-cyclo- alkenediyl, where in the cycloalkanediyl or cycloalkenediyl rings one carbon atom may be replaced by an oxygen atom;
R1, R2 independently of one another are H, F, Br, CF₃, OCF₃, (C₁-C₆)-alkyl, O-(C₁-C₆)-alkyl, SCF₃, SF₅, OCF₂-CHF₂, O-phenyl, OH or NO₂; or
R1 and R2 in juxtaposition together with the phenyl ring are a fused, unsaturated or completely or partially saturated bicyclic (C₉-C₁₂)-aryl or (C₉-C₁₁)-heteroaryl ring system;
R3 is H, CF₃, (C₁-C₆)-alkyl, (C₃-C₆)-cycloalkyl or phenyl;
X is (C₁-C₆)-alkanediyl, where one carbon atom in the alkanediyl group may be replaced by an oxygen atom;
Y is (C₁-C₆)-alkanediyl or (C₁-C₆)-alkenediyl, where one or two CH₂ groups in the alkanediyl or alkenediyl group may be replaced by O, CO, S, SO or SO₂ and alkanediyl or alkenediyl may be unsubstituted or substituted by OH;
Ring B is phenyl which may be mono- or disubstituted by NO₂, Cl, CN, (C₁-C₆)-alkyl, (C₁-C₆)-alkoxy or tetrazole; or pyrrolidin-2-one which additionally contains a nitrogen or sulfur atom in the 4-position and is substituted by oxo or thioxo in the 5-position and may be substituted on the N1-atom by R4;
R4 is (C₁-C₆)-alkyl, phenyl or benzyl;
and its physiologically acceptable salts.

3. A compound of the formula I as claimed in claim 1 or 2, in which
Ring A is (C₃-C₈)-cycloalkanediyl in which one carbon atom is replaced by an oxygen atom;
R1, R2 independently of one another are H, F, Br, CF₃, OCF₃, (C₁-C₆)-alkyl, O-(C₁-C₆)-alkyl, SCF₃, SF₅, OCF₂-CHF₂, O-phenyl, OH or NO₂; or
R1 and R2 in juxtaposition together with the phenyl ring are a fused, unsaturated bicyclic (C₉-C₁₂)-aryl or (C₉-C₁₁)-heteroaryl ring system;
R3 is H, CF₃, (C₁-C₆)-alkyl, (C₃-C₈)-cycloalkyl or phenyl;
X is (C₁-C₆)-alkanediyl, where the C1 carbon atom is replaced by an oxygen atom;
Y is (C₁-C₆)-alkanediyl or (C₁-C₆)-alkenediyl, where one or two CH₂ groups in the alkanediyl or alkenediyl group may be replaced by O, CO or SO₂ and alkanediyl or alkenediyl may be substituted by OH;
Ring B is phenyl which may be mono- or disubstituted by NO₂, Cl, CN, (C₁-C₆)-alkyl, (C₁-C₆)-alkoxy or tetrazole; or pyrrolidin-2-one which additionally contains a nitrogen or sulfur atom in the 4-position and is substituted by oxo or thioxo in the 5-position and may be substituted on the N1-atom by R4;
R4 is (C₁-C₆)-alkyl, phenyl or benzyl;
and its physiologically acceptable salts.

4. A compound of the formula I as claimed in any of claims 1 to 3, in which
Ring A is cyclohexane-1,3-diyl;
R1, R2 are independently of one another H, F, Br, CF₃, OCF₃, (C₁-C₆)-alkyl, O-(C₁-C₆)-alkyl, SCF₃, SF₅, OCF₂-CHF₂, O-phenyl, OH or NO₂; or
R1 and R2 in juxtaposition together with the phenyl ring are a fused unsaturated bicyclic (C₉-C₁₀)-aryl or (C₉-C₁₀)-heteroaryl ring system;
R3 is H, CF₃, (C₁-C₆)-alkyl, (C₃-C₆)-cycloalkyl or phenyl;
X CH₂-O;
Y is (C₁-C₄)-alkanediyl, O-(C₁-C₄)-alkanediyl, (C₁-C₄)-alkenediyl, O-(C₁-C₄)-alkenediyl, O-SO₂ or O-CO, where alkanediyl may be substituted by OH;
Ring B is phenyl which may be mono- or disubstituted by NO₂, Cl, CN, (C₁-C₆)-alkyl, (C₁-C₆)-alkoxy or tetrazole; or thiazolidine-1,4-dione which may be substituted on the N3-atom by R4;
R4 is (C₁-C₆)-alkyl, phenyl or benzyl;
and its physiologically acceptable salts.

5. A compound of the formula I as claimed in any of claims 1 to 4, in which
Ring A is cyclohexane-1,3-diyl;
R1, R2 independently of one another are H, F, Br, CF₃, (C₁-C₆)-alkyl or O- (C₁-C₆)-alkyl; or
R1 and R2 together with the phenyl ring are naphthyl;
R3 is (C₁-C₆)-alkyl, (C₅-C₆)-cycloalkyl or phenyl;
X is CH₂-O;
Y is (C₁-C₄)-alkanediyl, O-(C₁-C₄)-alkanediyl, (C₁-C₄)-alkenediyl, O-(C₁-C₄)-alkenediyl, O-SO₂ or O-CO, where alkanediyl may be substituted by OH;
Ring B is phenyl which may be mono- or disubstituted by NO₂, Cl, CN, (C₁-C₆)-alkyl, (C₁-C₆)-alkoxy or tetrazole; or
Ring B is thiazolidine-2,4-dione which may be substituted on the N3-atom by R4;
R4 is (C₁-C₆)-alkyl, phenyl or benzyl;
and its physiologically acceptable salts.

6. A compound of the formula I as claimed in any of claims 1 to 5, in which
R2 is hydrogen and
R1 is attached in the meta- or para-position.

7. A compound of the formula I as claimed in any of claims 1 to 6, in which
Y is -CH₂-CH₂-.

8. A pharmaceutical comprising one or more compounds of the formula I as claimed in one or more of claims 1 to 7.

9. A pharmaceutical comprising one or more compounds of the formula I as claimed in one or more of claims 1 to 7 and one or more active compounds which act favorably on metabolic disorders or diseases associated with them.

10. A pharmaceutical comprising one or more compounds of the formula I as claimed in one or more of claims 1 to 7 and one or more antidiabetics.

11. A pharmaceutical comprising one or more compounds of the formula I as claimed in one or more of claims 1 to 7 and one or more lipid modulators.

12. A compound of the formula I as claimed in one or more of claims 1 to 7 for use in the treatment and/or prevention of disorders of fatty acid metabolism and disorders of glucose utilization.

13. A compound of the formula I as claimed in one or more of claims 1 to 7 for use in the treatment and/or prevention of disorders in which insulin resistance plays a role.

14. A compound of the formula I as claimed in one or more of claims 1 to 7 for use in the treatment and/or prevention of diabetes mellitus and sequelae associated therewith.

15. A compound of the formula I as claimed in one or more of claims 1 to 7 for use in the treatment and/or prevention of dyslipidemias and their sequelae.

16. A compound of the formula I as claimed in one or more of claims 1 to 7 for use in the treatment and/or prevention of conditions associated with metabolic syndrome.

17. A compound as claimed in one or more of claims 1 to 7 in combination with at least one further active compound for use in the treatment and/or prevention of disorders of the fatty acid metabolism and disorders of glucose utilization.

18. A compound as claimed in one or more of claims 1 to 7 in combination with at least one further active compound for use in the treatment and/or prevention of disorders in which insulin resistance plays a role.

19. A process for preparing a pharmaceutical comprising one or more of the compounds as claimed in one or more of claims 1 to 7, which comprises mixing the active compound with a pharmaceutically suitable carrier and bringing this mixture into a form suitable for administration.

## Revendications

1. Composés de formule I 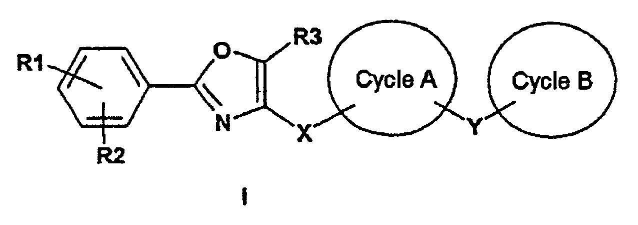 où
cycle A signifie (C₃-C₈)-cycloalcanediyle, (C₃-C₈)- cycloalcènediyle, où, dans les cycles cycloalcanediyle ou cycloalcènediyle, un ou plusieurs atomes de carbone peuvent être remplacés par des atomes d'oxygène ;
R1, R2 signifient, indépendamment l'un de l'autre, H, F, Br, CF₃, OCF₃, (C₁-C₆)-alkyle, O-(C₁-C₆)-alkyle, SCF₃, SF₅, OCF₂-CHF₂, O-phényle, OH, NO₂ ; ou
R1 et R2 dans une position adjacente signifient ensemble avec le cycle phényle un système bicyclique (C₉-C₁₂)-aryle ou (C₉-C₁₁)-hétéroaryle condensé, totalement, partiellement ou non saturé ;
R3 signifie H, CF₃, (C₁-C₆)-alkyle, (C₁-C₆)-alcoxy, (C₃-C₈)-cycloalkyle, phényle ;
X signifie (C₁-C₆)-alcanediyle, où dans le groupe alcanediyle, un ou plusieurs atomes de carbone peuvent être remplacés par des atomes d'oxygène ;
Y signifie (C₁-C₆) -alcanediyle ou (C₁-C₆)-alcènediyle, où, dans le groupe alcanediyle ou alcènediyle, un ou plusieurs groupes CH₂ peuvent être remplacés par O, CO, S, SO ou SO₂ et alcanediyle ou alcènediyle peuvent le cas échéant être substitués par OH ;
cycle B signifie phényle, qui peut être monosubstitué ou disubstitué par NO₂, Cl, CN, (C₁-C₆)-alkyle, (C₁- C₆)-alcoxy ou tétrazole ; ou pyrrolidin-2-one, qui contient en outre un atome de N ou S dans le cycle et qui est substituée par oxo ou thioxo et qui peut être substituée par R4 sur l'atome de N ;
R4 signifie (C₁-C₆)-alkyle, phényle, benzyle ;
ainsi que leurs sels physiologiquement acceptables.

2. Composés de formule I selon la revendication 1, où
cycle A signifie (C₃-C₈)-cycloalcanediyle, (C₃-C₈)- cycloalcènediyle, où, dans les cycles cycloalcanediyle ou cycloalcènediyle, un atome de carbone peut être remplacé par un atome d'oxygène ;
R1, R2 signifient, indépendamment l'un de l'autre, H, F, Br, CF₃, OCF₃, (C₁-C₆)-alkyle, O-(C₁-C₆)-alkyle, SCF₃, SF₅, OCF₂-CHF₂, O-phényle, OH, NO₂ ; ou
R1 et R2 dans une position adjacente signifient ensemble avec le cycle phényle un système bicyclique (C₉-C₁₂)-aryle ou (C₉-C₁₁)-hétéroaryle condensé, totalement, partiellement ou non saturé ;
R3 signifie H, CF₃, (C₁-C₆)-alkyle, (C₃-C₈)- cycloalkyle, phényle ;
X signifie (C₁-C₆)-alcanediyle, où, dans le groupe alcanediyle, un atome de carbone peut être remplacé par un atome d'oxygène ;
Y signifie (C₁-C₆)-alcanediyle ou (C₁-C₆)-alcènediyle, où, dans le groupe alcanediyle ou alcènediyle, un ou deux groupes CH₂ peuvent être remplacés par O, CO, S, SO ou SO₂ et alcanediyle ou alcènediyle peuvent le cas échéant être substitués par OH ;
cycle B signifie phényle, qui peut être monosubstitué ou disubstitué par NO₂, Cl, CN, (C₁-C₆)-alkyle, (C₁- C₆)-alcoxy ou tétrazole ; ou pyrrolidin-2-one, qui contient en outre un atome de N ou S en 4ème position et qui est substituée par oxo ou thioxo en 5ème position et qui peut être substituée par R4 sur l'atome N1 ;
R4 signifie (C₁-C₆)-alkyle, phényle, benzyle ;
ainsi que leurs sels physiologiquement acceptables.

3. Composés de formule I selon la revendication 1 ou 2, où
cycle A signifie (C₃-C₈)-cycloalcanediyle, où un atome de carbone est remplacé par un atome d'oxygène ;
R1, R2 signifient, indépendamment l'un de l'autre, H, F, Br, CF₃, OCF₃, (C₁-C₆)-alkyle, O-(C₁-C₆)-alkyle, SCF₃, SF₅, OCF₂-CHF₂, O-phényle, OH, NO₂ ; ou
R1 et R2 dans une position adjacente signifient ensemble avec le cycle phényle un système bicyclique (C₉-C₁₂)-aryle ou (C₉-C₁₁)-hétéroaryle condensé, non saturé ;
R3 signifie H, CF₃, (C₁-C₆)-alkyle, (C₃-C₈)-cycloalkyle, phényle ;
X signifie (C₁-C₆)-alcanediyle, où l'atome de carbone C1 est remplacé par un atome d'oxygène ;
Y signifie (C₁-C₆)-alcanediyle ou (C₁-C₆)-alcènediyle, où, dans le groupe alcanediyle ou alcènediyle, un ou deux groupes CH₂ peuvent être remplacés par O, CO ou SO₂ et alcanediyle ou alcènediyle peuvent le cas échéant être substitués par OH ;
cycle B signifie phényle, qui peut être monosubstitué ou disubstitué par NO₂, Cl, CN, (C₁-C₆)-alkyle, (C₁- C₆)-alcoxy ou tétrazole ; ou pyrrolidin-2-one, qui contient en outre un atome de N ou S en 4ème position et qui est substituée par oxo ou thioxo en 5ème position et qui peut être substituée par R4 sur l'atome N1 ;
R4 signifie (C₁-C₆)-alkyle, phényle, benzyle ;
ainsi que leurs sels physiologiquement acceptables.

4. Composés de formule I selon les revendications 1 à 3, où
cycle A signifie cyclohexane-1,3-diyle ;
R1, R2 signifient, indépendamment l'un de l'autre, H, F, Br, CF₃, OCF₃, (C₁-C₆)-alkyle, O-(C₁-C₆)-alkyle, SCF₃, SF₅, OCF₂-CHF₂, O-phényle, OH, NO₂ ; ou
R1 et R2 dans une position adjacente signifient ensemble avec le cycle phényle un système bicyclique (C₉-C₁₀)-aryle ou (C₉-C₁₀)-hétéroaryle condensé, non saturé ;
R3 signifie H, CF₃, (C₁-C₆)-alkyle, (C₃-C₆) - cycloalkyle, phényle ;
X signifie CH₂-O ;
Y signifie (C₁-C₄)-alcanediyle, O-(C₁-C₄)-alcanediyle, (C₁-C₄)-alcènediyle, O-(C₁-C₄)-alcènediyle, O-SO₂, O-CO ; où alcanediyle peut être substitué par OH ;
cycle B signifie phényle, qui peut être monosubstitué ou disubstitué par NO₂, Cl, CN, (C₁-C₆)-alkyle, (C₁- C₆)-alcoxy ou tétrazole ; ou thiazolidine-1,4-dione, qui peut être substituée par R4 sur l'atome N3 ;
R4 signifie (C₁-C₆)-alkyle, phényle, benzyle ;
ainsi que leurs sels physiologiquement acceptables.

5. Composés de formule I selon les revendications 1 à 4, où
cycle A signifie cyclohexane-1,3-diyle ;
R1, R2 signifient, indépendamment l'un de l'autre H, F, Br, CF₃, (C₁-C₆)-alkyle, O-(C₁-C₆)-alkyle ; ou
R1 et R2 ensemble avec le cycle phényle représentent naphtyle ;
R3 signifie (C₁-C₆)-alkyle, (C₅-C₆)-cycloalkyle, phényle ;
X signifie CH₂-O ;
Y signifie (C₁-C₄)-alcanediyle, O-(C₁-C₄)-alcanediyle, (C₁-C₄)-alcènediyle, O-(C₁-C₄)-alcènediyle, O-SO₂, O-CO, où alcanediyle peut être substitué par OH ;
cycle B signifie phényle, qui peut être monosubstitué ou disubstitué par NO₂, Cl, CN, (C₁-C₆)-alkyle, (C₁- C₆)-alcoxy ou tétrazole ; ou
cycle B signifie thiazolidine-2,4-dione, qui peut être substituée par R4 sur l'atome N3 ;
R4 signifie (C₁-C₆)-alkyle, phényle, benzyle ;
ainsi que leurs sels physiologiquement acceptables.

6. Composés de formule I selon les revendications 1 à 5, où
R2 signifie hydrogène et
R1 est lié en position méta ou para.

7. Composés de formule I selon les revendications 1 à 6, où
Y signifie -CH₂CH₂-.

8. Médicament contenant un ou plusieurs des composés de formule I selon l'une ou plusieurs des revendications 1 à 7.

9. Médicament contenant un ou plusieurs des composés de formule I selon l'une ou plusieurs des revendications 1 à 7 et une ou plusieurs substances actives, qui ont des effets favorables sur les troubles du métabolisme ou les maladies qui y sont associées.

10. Médicament contenant un ou plusieurs des composés de formule I selon l'une ou plusieurs des revendications 1 à 7 et un ou plusieurs antidiabétiques.

11. Médicament contenant un ou plusieurs des composés de formule I selon l'une ou plusieurs des revendications 1 à 7 et un ou plusieurs modulateurs de lipides.

12. Composés de formule I selon l'une ou plusieurs des revendications 1 à 7 destinés à une utilisation lors du traitement et/ou de la prévention de troubles du métabolisme des acides gras et de troubles de l'utilisation du glucose.

13. Composés de formule I selon l'une ou plusieurs des revendications 1 à 7 destinés à une utilisation lors du traitement et/ou de la prévention de troubles dans lesquels la résistance à l'insuline joue un rôle.

14. Composés de formule I selon l'une ou plusieurs des revendications 1 à 7 destinés à une utilisation lors du traitement et/ou de la prévention du diabète sucré et des maladies secondaires qui y sont liées.

15. Composés de formule I selon l'une ou plusieurs des revendications 1 à 7 destinés à une utilisation lors du traitement et/ou de la prévention de dyslipidémies et de leurs conséquences.

16. Composés de formule I selon l'une ou plusieurs des revendications 1 à 7 destinés à une utilisation lors du traitement et/ou de la prévention d'états qui sont associés au syndrome métabolique.

17. Composés selon l'une ou plusieurs des revendications 1 à 7 en combinaison avec au moins une autre substance active destinés à une utilisation lors du traitement et/ou de la prévention de troubles du métabolisme des acides gras et de troubles de l'utilisation du glucose.

18. Composés selon l'une ou plusieurs des revendications 1 à 7 en combinaison avec au moins une autre substance active destinés à une utilisation lors du traitement et/ou de la prévention de troubles dans lesquels la résistance à l'insuline joue un rôle.

19. Procédé pour la préparation d'un médicament contenant un ou plusieurs des composés selon l'une ou plusieurs des revendications 1 à 7, **caractérisé en ce que** la substance active est mélangée avec un support pharmaceutiquement approprié et ce mélange est amené dans une forme appropriée pour l'administration.
